# EUROPEAN PATENT APPLICATION

(11) **EP 4 653 430 A1**
(43) Date of publication of application: **26.11.2025**
(21) Application number: 24177883.6
(22) Date of filing: 24.05.2024
(51) Int. Cl.: C07D 401/14, A61P 25/02, A61P 25/08, A61P 25/14, A61P 25/28, A61P 29/00, A61P 35/00, A61P 37/00, C07D 413/14, C07D 417/14, A61K 31/426

(54) **AUTOPHAGY INDUCING COMPOUNDS AND USES THEREOF, IN PARTICULAR FOR THE PREVENTION OR TREATMENT OF DISEASES OF THE CNS**

(71) Applicant: Samsara Therapeutics Inc., Lewes, DE 19958 (US)
(72) Inventor: HAMLEY, Peter, Littlemore, Oxford OX4 4GA (GB); GALLOWAY, Warren, 61476 Kronberg im Taunus (DE); ATKINSON, Ben, Littlemore, Oxford OX4 4GA (GB)
(74) Representative: Krauss, Jan

(57) **Abstract**

The invention relates to pharmaceutical compositions and methods of treating autophagy related diseases and disorders, and in particular neurological and/or CNS-related diseases. The present invention relates to compounds according to Formula (I) or salts, solvates and/or hydrates thereof, wherein said compounds induce and/or stimulate the process of autophagy, as well as uses of the compounds in the treatment and prevention of autophagy related diseases and disorders. Examples are cancer, age-related diseases, and viral infection, in particular of the CNS.

## Description

The invention relates to pharmaceutical compositions and methods of treating autophagy related diseases and disorders, and in particular neurological and/or CNS-related diseases. The present invention relates to compounds according to Formula (I) or salts, solvates and/or hydrates thereof, wherein said compounds induce and/or stimulate the process of autophagy, as well as uses of the compounds in the treatment and prevention of autophagy related diseases and disorders. Examples are cancer, age-related diseases, and viral infection, in particular of the CNS.

### Background of the invention

Autophagy is the process of removing unnecessary organelles and proteins from cells that are lost or lost function, it helps maintain cell homeostasis and is a cell survival mechanism.

The cell-autonomous antimicrobial defense functions of autophagy, demonstrated initially in the case of streptococci and Mycobacterium tuberculosis have been extended to a wide variety of microbes with a caveat that most highly adapted pathogens have evolved specific protective mechanisms against autophagic elimination of microbes. Other studies have uncovered orderly intersections between autophagy and innate and adaptive immunity, T cell development, differentiation and homeostasis, and inflammatory responses. Thus, autophagy plays a large role in various diseases such as cancer, inflammatory disease, degenerative neurological disease, and immune disease. Autophagy is a cell survival mechanism that is induced in stressed cells.

Towers and Thorburn (in: Therapeutic Targeting of Autophagy. EBioMedicine. 2016;14:15-23. doi:10.1016/j.ebiom.2016.10.034) disclose that autophagy is widely accepted as cytoprotective against neurodegenerative diseases and a variety of clinical interventions are moving forward to increase autophagy as a therapeutic intervention.

Autophagy has both positive and negative roles in cancer and this has led to controversy over whether or how autophagy manipulation should be attempted in cancer therapy. Nevertheless, cancer is the disease where most current activity in trying to manipulate autophagy for therapy is taking place and dozens of clinical trials are using autophagy inhibition with Chloroquine or Hydroxychloroquine in combination with other drugs for the treatment of multiple neoplasms. They review recent literature implicating autophagy in neurodegenerative diseases and cancer and highlight some of the opportunities, controversies and potential pitfalls of therapeutically targeting autophagy.

Mulcahy Levy and Thorburn (in: Autophagy in cancer: moving from understanding mechanism to improving therapy responses in patients. Cell Death Differ 27, 843-857 (2020).https://doi.org/10.1038/s41418-019-0474-7) disclose that autophagy allows for cellular material to be delivered to lysosomes for degradation resulting in basal or stress-induced turnover of cell components that provide energy and macromolecular precursors. These activities are thought to be particularly important in cancer where both tumor-promoting and tumor-inhibiting functions of autophagy have been described. Autophagy has also been intricately linked to apoptosis and programmed cell death, and understanding these interactions is becoming increasingly important in improving cancer therapy and patient outcomes. In the review, they consider how recent discoveries about how autophagy manipulation elicits its effects on cancer cell behavior can be leveraged to improve therapeutic responses.

Grainger et al. (1995, Nature Medicine 1: 1067-1073) and Reckless et al. (1997, Circulation 95: 1542-1548) have demonstrated that tamoxifen, a potent inducer of autophagy, inhibited atherosclerosis in mice models by suppressing the diet-induced formation of lipid lesions in the aorta by lowering of low-density lipoprotein (LDL) cholesterol.

CN 102516239A discloses aromatic thiazole micro-molecular organic compounds with the structural formula represented by Formula (I), or hydrates thereof or pharmaceutically acceptable salts thereof. The compounds of the invention or compositions containing the compounds can be used for sun protection, anti-ultraviolet and skin damage protection as a cosmetic additive and for inhibition of the cell apoptosis caused by excess ultraviolet irradiation and the expression of cyclooxygenase COX2.

US 2004-0116425A1 discloses related compounds useful in the treatment of diseases associated with prenylation of proteins and pharmaceutically acceptable salts thereof, to pharmaceutical compositions comprising same, and to methods for inhibiting protein prenylation in an organism using the same.

WO 2009-103432A2 extremely broadly relates to molecular probes of the formula (I) L1-R1-L-A-X as defined herein that allow for the observation of the catalytic activity of a selected caspase, cathepsin, MMP and carboxypeptidase in in vitro assays, in cells or in multicellular organisms.

In WO 2010-147653A1 compounds are extremely broadly disclosed for treating ophthalmic conditions related to mislocalization of opsin proteins, the misfolding of mutant opsin proteins and the production of toxic visual cycle products that accumulate in the eye.

WO 2017-216579A1 relates to a heterocyclic compound 1,1'-(((propane-2,2-diylbis(4,1-phenylene))bis(oxy))bis(ethane-2,1-diyl))dipyrrolidine and its medical uses, for example as an autophagy inducer.

WO 2023-089074A1 relates to pharmaceutical compositions and methods of treating autophagy related diseases and disorders, in particular for a systemic treatment thereof. The present invention relates to compounds according to Formula (I) or salts, solvates and/or hydrates thereof, wherein said compounds induce and/or stimulate the process of autophagy, as well as uses of the compounds in the treatment and prevention of autophagy related diseases and disorders. Examples are cancer, age-related diseases, and viral infection that can be effectively treated with compounds that are effective when provided systemically.

The development of more selective autophagy inducers is needed if they are to become medicinally useful in the treatment and/or prevention of diseases where autophagy plays a role, in particular in the central nervous system (CNS). It is an object of the present invention to provide effective agents that can be used for the prevention and treatment of conditions and diseases that can be treated/prevented by inducing and/or stimulating of autophagy, in particular cancer, age-related diseases, and infections. Other objects and advantages will become apparent to the person of skill when studying the present description of the present invention.

In a first aspect of the present invention, the above object is solved by a compound according to the general Formula (I) 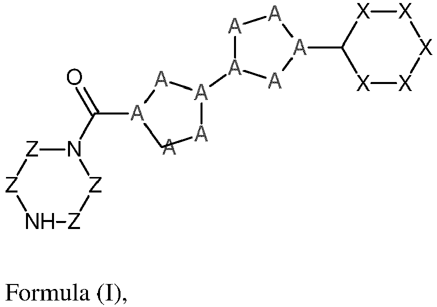 wherein
A is independently selected from chemically possible combinations of C, CH, CH₂, N, NH, O, and S, optionally substituted with cyano,
X is independently selected from chemically possible combinations of C, CH, CH₂, N and NH, optionally substituted with straight or branched C₂ to C₆ alkyl, straight or branched C₁ to C₄ hydroxyalkyl, such as, for example, -CH₂-CH₂-OH, straight or branched C₁ to C₄ hydroxyalkoxy, such as, for example, -O-CH₂-CH₂-OH), halo, trifluoromethyl, cyclopropyl, and cyano,
Z is independently selected from chemically possible combinations of CH, CH₂, N and NH, optionally substituted with straight or branched C₂ to C₆ alkyl,
and a physiologically acceptable salt, a solvate, a hydrate, an enantiomer or a polymorph thereof.

The compounds described herein were shown surprisingly to be highly effective autophagy inducers/stimulators. The present inventors thus invented compounds for the induction and/or stimulation of autophagy in disease- and undesired conditions-related cells of a patient or subject. In the case of a compound represented by the general Formula according to the present invention, the autophagy is effectively induced and/or stimulated.

It was surprisingly found that the compounds according to Formula I, as an autophagy inducer, were effective and exhibit many advantages compared to other autophagy-related treatment options. In comparison with prior art compounds as tested (including data not shown), an improvement of the present invention lies in the unexpected observation that the compounds described herein are highly effective autophagy inducers (see Examples below). Furthermore, the compounds exhibit higher concentrations inside compared to outside of the CNS or brain (peripheral), preferably a minimum of a 2:1 brain/CNS to peripheral ratio, after an administration thereof, which makes them ideal candidates for the induction and/or stimulation of autophagy in disease- and undesired conditions-related cells of a patient or subject involving the CNS and brain.

Based on these results this is evidence that these compounds are active in autophagy-related diseases as disclosed herein. Furthermore, examples for this activity are shown in the Examples below which also relate to specific activities in models of viral infections and diseases involving protein misfolding. By way of an exemplar medical application, the compounds according to Formula F to I, as well as A and D 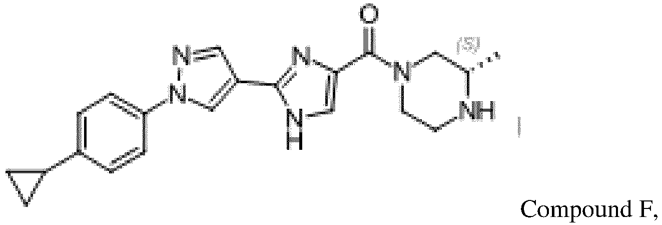 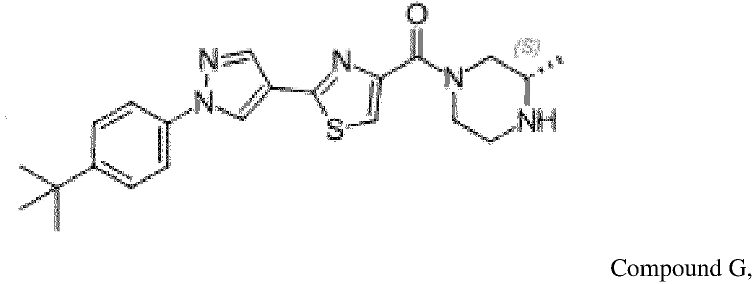 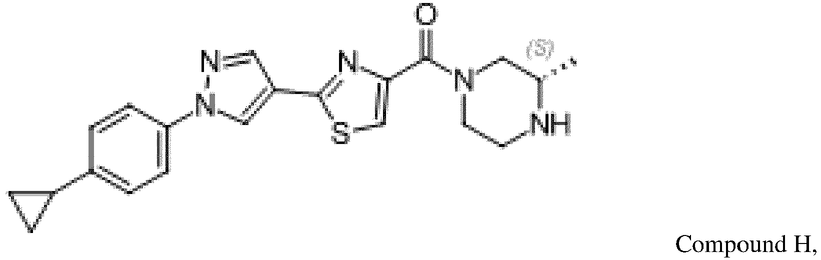 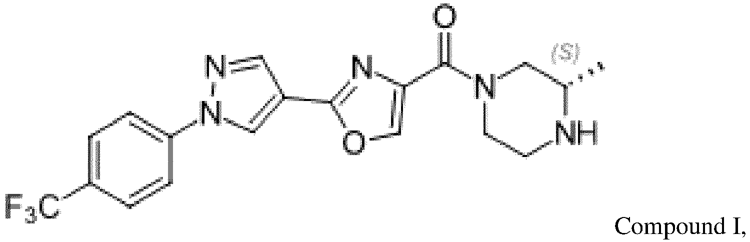 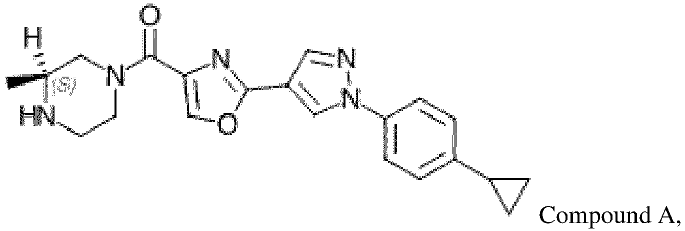 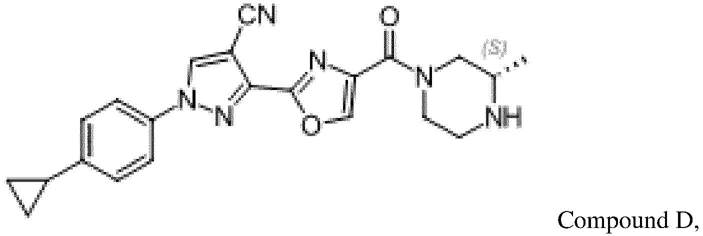 were surprisingly efficacious (see Examples below).

Preferred is a compound according to the present invention according to the following formula II, 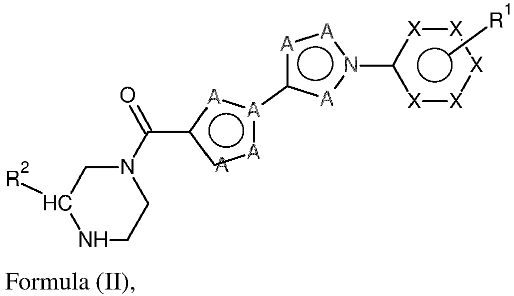 wherein
A is as above,
X is independently selected from chemically possible combinations of C, CH, CH₂, N and NH, and is optionally substituted with R¹,
R¹ is independently selected from straight or branched C₂ to C₆ alkyl, straight or branched C₁ to C₄ hydroxyalkyl (for example, -CH₂-CH₂-OH), straight or branched C₁ to C₄ hydroxyalkoxy (for example, -O-CH₂-CH₂-OH), halo, trifluoromethyl, cyclopropyl, or cyano,
R² is independently selected from H, or straight or branched C₂ to C₆ alkyl, and
a physiologically acceptable salt, a solvate, a hydrate, an enantiomer or a polymorph thereof.

Preferred is a compound according to the present invention according to the following formula III, 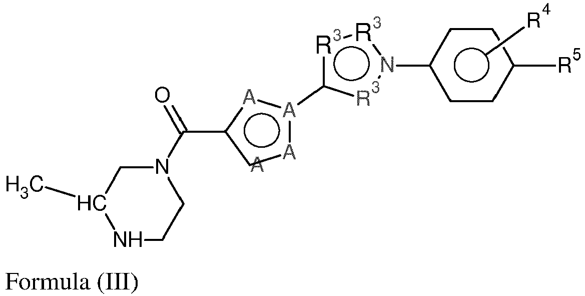 wherein
A is as above,
R⁵ is independently selected from H, straight or branched C₂ to C₆ alkyl, straight or branched C₁ to C₄ hydroxyalkyl, such as, for example, -CH₂-CH₂-OH, straight or branched C₁ to C₄ hydroxyalkoxy, such as, for example, -O-CH₂-CH₂-OH, halo, trifluoromethyl, cyclopropyl, or cyano,
R³ is independently selected from chemically possible combinations of C, CH, N and NH, optionally substituted with cyano,
R⁴ is independently selected from H or cyano,
   and
a physiologically acceptable salt, a solvate, a hydrate, an enantiomer or a polymorph thereof.

Preferred is a compound according to the present invention selected from 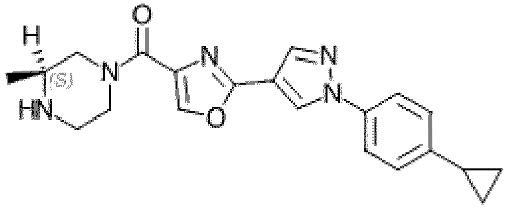
[(S)-3-methyl-1-piperazinyl]{2-[1-(p-cyclopropylphenyl)-4-pyrazolyl]-1,3-oxazol-4-yl}methanone, 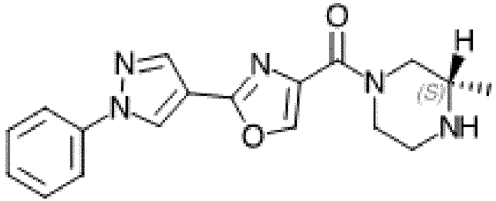
[(S)-3-methyl-1-piperazinyl] [2-(1-phenyl-4-pyrazolyl)-1,3-oxazol-4-yl]methanone, 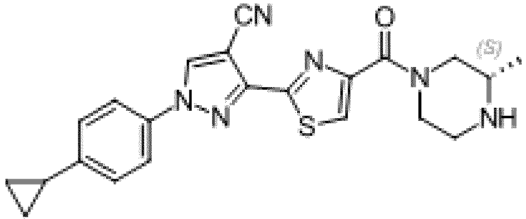
3-(4-{ [(S)-3-methyl-1-piperazinyl]carbonyl}-1,3-thiazol-2-yl)-1-(p-cyclopropylphenyl)-4-pyrazolecarbonitrile, 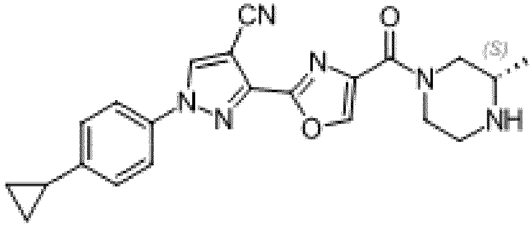
3-(4-{ [(S)-3-methyl-1-piperazinyl]carbonyl}-1,3-oxazol-2-yl)-1-(p-cyclopropylphenyl)-4-pyrazolecarbonitrile, 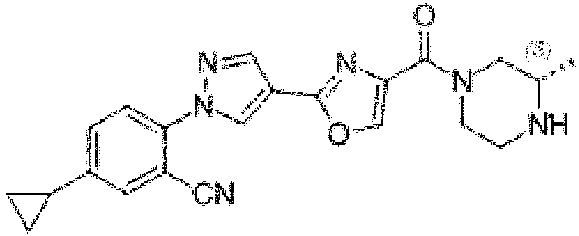
2-[4-(4-{[(S)-3-methyl-1-piperazinyl]carbonyl}-1,3-oxazol-2-yl)-1-pyrazolyl]-5-cyclopropylbenzonitrile, 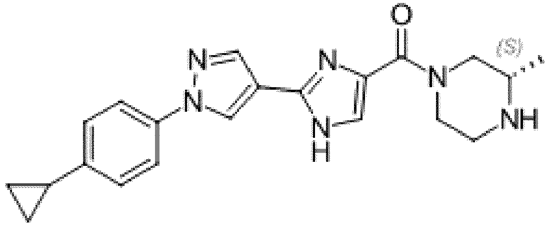
[(S)-3-methyl-1-piperazinyl] {2-[1-(p-cyclopropylphenyl)-4-pyrazolyl]-4-imidazolyl} methanone, 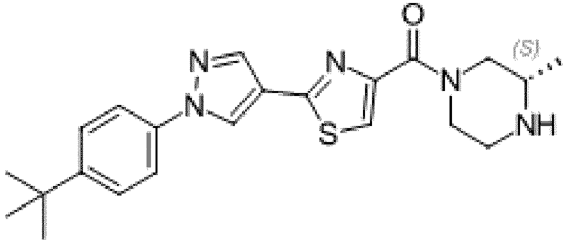
[(S)-3-methyl-1-piperazinyl](2-{1-[p-(tert-butyl)phenyl]-4-pyrazolyl}-1,3-thiazol-4-yl)methanone, 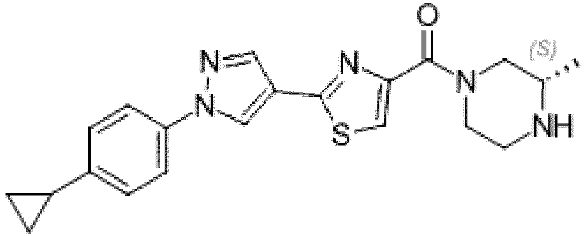
[(S)-3-methyl-1-piperazinyl]{2-[1-(p-cyclopropylphenyl)-4-pyrazolyl]-1,3-thiazol-4-yl}methanone, 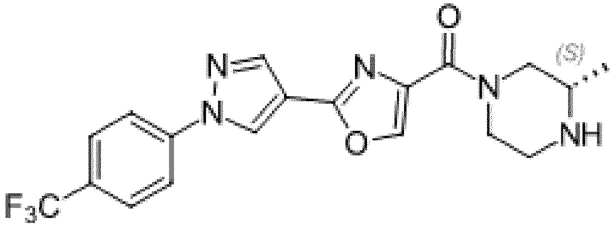
[(S)-3-methyl-1-piperazinyl](2-{1-[p-(trifluoromethyl)phenyl]-4-pyrazolyl}-1,3-oxazol-4-yl)methanone, 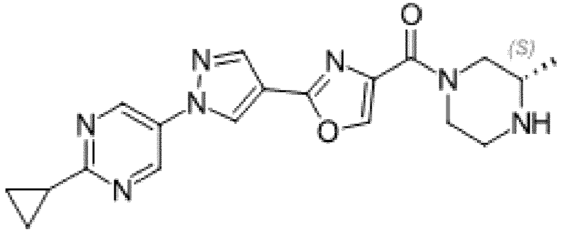
[(S)-3-methyl-1-piperazinyl]{2-[1-(2-cyclopropyl-5-pyrimidinyl)-4-pyrazolyl]-1,3-oxazol-4-yl}methanone, 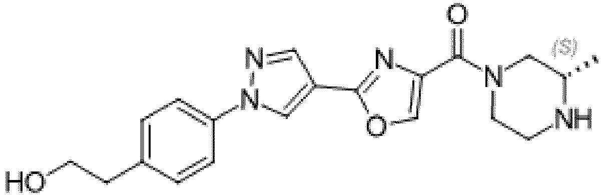
[(S)-3-methyl-1-piperazinyl](2-{1-[p-(2-hydroxyethyl)phenyl]-4-pyrazolyl}-1,3-oxazol-4-yl)methanone, 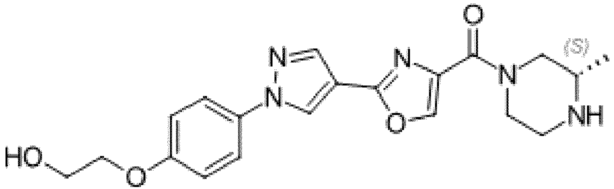
[(S)-3-methyl-1 -piperazinyl] (2- {1 -[p-(2-hydroxyethoxy)phenyl]-4-pyrazolyl} -1,3-oxazol-4-yl)methanone, 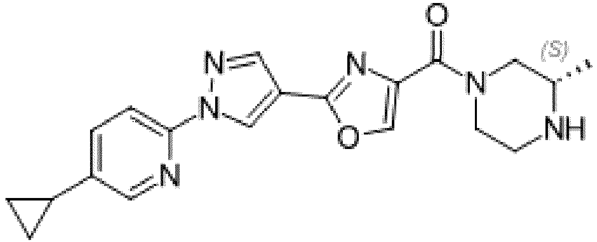
[(S)-3-methyl-1-piperazinyl] {2-[1-(5-cyclopropyl-2-pyridyl)-4-pyrazolyl]-1,3-oxazol-4-yl}methanone, 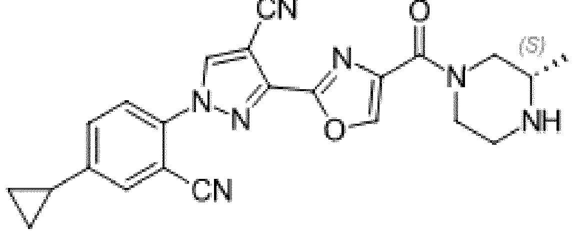
3-(4-{[(S)-3-methyl-1-piperazinyl]carbonyl}-1,3-oxazol-2-yl)-1-(2-cyano-4-cyclopropylphenyl)-4-pyrazolecarbonitrile, 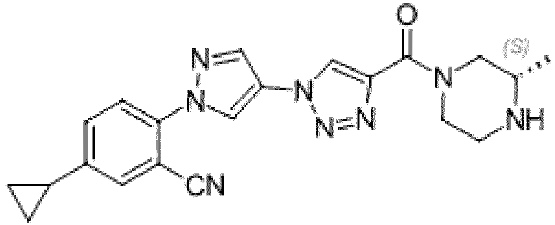
2-[4-(4-{[(S)-3-methyl-1-piperazinyl]carbonyl}-1H-1,2,3-triazol-1-yl)-1-pyrazolyl]-5-cyclopropylbenzonitrile, and 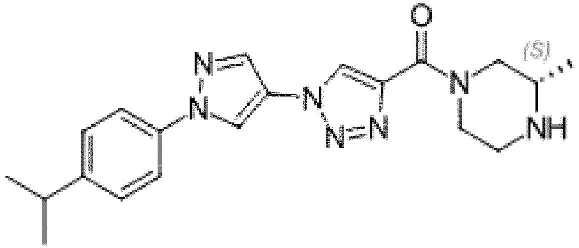
[(S)-3-methyl-1-piperazinyl] { 1-[1-(p-cumenyl)-4-pyrazolyl]-1H-1,2,3-triazol-4-yl} methanone.

The term "pharmaceutically acceptable salt" refers to a pharmaceutically acceptable organic or inorganic salt of the compound of the invention. This may include addition salts of inorganic acids such as hydrochloride, hydrobromide, hydroiodide, sulphate, phosphate, diphosphate and nitrate or of organic acids such as acetate, maleate, fumarate, tartrate, succinate, citrate, lactate, methanesulphonate, p-toluenesulphonate, palmoate and stearate. Exemplary salts also include oxalate, chloride, bromide, iodide, bisulphate, acid phosphate, isonicotinate, salicylate, acid citrate, oleate, tannate, pantothenate, bitartrate, ascorbate, gentisinate, gluconate, glucuronate, saccharate, formate, benzoate, glutamate, ethanesulfonate, and benzenesulfonate salts. For other examples of pharmaceutically acceptable salts, reference can be made to Gould (1986, Int J Pharm 33: 201-217).

According to a further aspect of the invention, there is a provided a method for producing a compound according to the present invention, comprising the steps according to any one of general experimental procedures, as disclosed herein and below.

According to a further aspect of the invention, there is a provided a pharmaceutical composition comprising a pharmaceutically effective amount of the compound according to the present invention, and a pharmaceutically or therapeutically acceptable excipient or carrier.

The term "pharmaceutically or therapeutically acceptable excipient or carrier" refers to a solid or liquid filler, diluent or encapsulating substance which does not interfere with the effectiveness or the biological activity of the active ingredients and which is not toxic to the host, which may be either humans or animals, to which it is administered. Depending upon the particular route of administration, a variety of pharmaceutically-acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water. Pharmaceutically acceptable carriers or excipients also include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal Si0₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for the compounds according to the present invention, and will readily be able to choose suitable pharmaceutically acceptable carriers or excipients, depending, e.g., on the formulation and administration route of the pharmaceutical composition.

All suitable modes of administration are contemplated according to the invention. For example, administration of the medicament may be via oral, subcutaneous, direct intravenous, slow intravenous infusion, continuous intravenous infusion, intravenous or epidural patient controlled analgesia (PCA and PCEA), intramuscular, intrathecal, epidural, intracistemal, intraperitoneal, transdermal, topical, buccal, sublingual, transmucosal, inhalation, intra-atricular, intranasal, rectal or ocular routes, abuse deterrent and abuse resistant formulations, sterile solutions suspensions and depots for parenteral use, and the like, administered as immediate release, sustained release, delayed release, controlled release, extended release and the like. The medicament may be formulated in discrete dosage units and can be prepared by any of the methods well known in the art of pharmacy. The pharmaceutical composition of the present invention can be formulated using methods known in the art to provide rapid, sustained or delayed release of the active ingredient after administration to a mammal. In general, at least one compound according to the present invention is admixed with at least one pharmaceutically acceptable carrier and/or excipient.

In addition to the aforementioned compounds of the invention, the pharmaceutical composition can contain two or more compounds according to the present invention and also other therapeutically active substances.

The dosage of the pharmaceutical composition according to the present invention can be appropriately selected according to the route of administration, the subject to be administered, the target disease and its severity, age, sex weight, individual differences and disease state. Dosage may be repeated several times a day.

According to the present invention, a mammalian subject can be preferably selected from a mouse, rat, cat, dog, rabbit, goat, sheep, horse, camel, lama, cow, monkey, a farm animal, a sport animal, and a pet, and a human.

Further provided is a compound of the invention as defined herein for use in the prevention and/or treatment of conditions and/or diseases in a mammalian subject, such as a human. A condition and/or disease suitable for treatment according to the relevant aspects of the invention is one which is characterized by defective or insufficient autophagy or which would benefit from modulation such as induction of autophagy.

The invention further encompasses the use of a compound of the invention as an autophagy inducer. The use may be a cosmetic use and/or in vitro, for example in an in vitro assay.

US 9,138,400, for example, relates to a cosmetic process for detoxifying the skin and/or for combating cutaneous aging, comprising the topical application on the skin of a composition that comprises at least one activator of the autophagy of cells of the skin. Eckhart L, Tschachler E, and Gruber F (in: Autophagic Control of Skin Aging. Front Cell Dev Biol. 2019;7:143. Published 2019 Jul 30. doi:10.3389/fcell.2019.00143) review the evidence for cell type-specific roles of autophagy in the skin and their differential contributions to aging.

Modified or altered autophagy has been shown to be relevant in neurodegenerative disease, as demonstrated by the accumulation of protein aggregates, for example in Alzheimer disease, Parkinson's disease, polyglutamine diseases, muscle diseases, and amyotrophic lateral sclerosis. Modified autophagy has also been implicated in other neurological diseases including epilepsies, neurometabolic and neurodevelopmental disorders such as schizophrenia.

A crucial role for therapy-induced autophagy in cancer cells has recently emerged, in modulating the interface of cancer cells and the immune system; primarily, by affecting the nature of danger signaling (i.e., the signaling cascade that facilitates the exposure and/or release of danger signals) associated with immunogenic cell death (ICD).

Therefore, compounds according to the present invention are for use in the prevention and/or treatment of an autophagy-related disease or condition in a mammalian subject, such as a human.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require complete recovery or complete prevention of a stress.

Preferred is a compound for use according to the present invention, wherein said autophagy-related disease or condition is selected from the group consisting of neurodegenerative diseases, Huntington's disease, Alzheimer's disease, Parkinson's disease, epilepsy, brain or other neuronal cancer, Wilson Disease, viral infection and related neuronal diseases, Niemann-Pick type C (NPC) disease, autoimmune diseases, multiple sclerosis, stroke, Wegener's granulomatosis, neuropathic pain, post-operative phantom limb pain or postherpetic neuralgia, ALS, spinal cord injury, and diseases and conditions of the CNS and/or neurons involving misfolded and/or nonfolded proteins as well as age-related forms of the diseases and conditions (such as neurodegenerative disease, cancer, and metabolic syndrome). Particularly preferred are ALS, Alzheimer's disease, and Parkinson's disease.

Cheon SY, Kim H, Rubinsztein DC, and Lee JE. (in: Autophagy, Cellular Aging and Age-related Human Diseases. Exp Neurobiol 2019;28:643-657. https://doi.org/10.5607/en.2019.28.6.643) disclose that during aging, cellular factors suggested as the cause of aging have been reported to be associated with progressively compromised autophagy. Dysfunctional autophagy may contribute to age-related diseases, such as neurodegenerative disease, cancer, and metabolic syndrome, in the elderly. Therefore, restoration of impaired autophagy to normal may help to prevent age-related disease and extend lifespan and longevity. They provide an overview of the mechanisms of autophagy underlying cellular aging and the consequent disease.

Similarly, Leidal, A.M., Levine, B. & Debnath, J. (in: Autophagy and the cell biology of age-related disease. Nat Cell Biol 20, 1338-1348 (2018).https://doi.org/10.1038/s41556-018-0235-8) review that autophagy declines with age and that impaired autophagy predisposes individuals to age-related diseases, whereas interventions that stimulate autophagy often promote longevity.

Also, Vaccaro Maria Ines, De Tata Vincenzo, and Gonzalez Claudio Daniel (in: Editorial: Autophagy in Endocrine-Metabolic Diseases Associated With Aging; Frontiers in Endocrinology, 11, 2020, pp 572, doi=10.3389/fendo.2020.00572) present a special issue containing a collection of 12 articles covering a broad range of key topics on the interplay of the different types of autophagy alterations with aging, endocrine-metabolic, and degenerative diseases.

Further preferred is the compound for use according to the present invention, wherein said prevention and/or treatment comprises a combination of at least two compounds for use according to the present invention, and/or a combination with at least one additional pharmaceutically active substance for said autophagy-related disease or condition.

It is to be understood that the present compound and/or a pharmaceutical composition comprising the present compound is for use to be administered to a human patient. The term "administering" means administration of a sole therapeutic agent or in combination with another therapeutic agent. It is thus envisaged that the pharmaceutical composition of the present invention are employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the compound for use, if required, as long as they act in combination (i.e. directly and/or indirectly, preferably synergistically) with the present compound(s) (for use).

In another aspect of the compound for use according to the present invention the prevention and/or treatment further comprises detecting and/or monitoring in said subject a response of at least one autophagy-biomarker. The biomarker is preferably selected from the group consisting of BECN1, ATG8/LC3 family, including LC3A, LC3B, LC3C), LC3-II, ULK1, p62, NBR1, ATG5 and ATG7.

This monitoring usually is performed on a biologically sample taken from the mammalian subject, and comprises commonly known tests, for example antibody based, PCR based, and the like. The tests are repeated over time, and can be compared to control samples and/or samples taken earlier from the mammalian subject. The results help the attending physician to maintain or modify the course of a treatment, usually based on the severity of the clinical symptoms of the autophagy-related disease and/or condition as treated.

In another aspect thereof, the present invention provides methods for preventing and/or treating an autophagy-related disease and/or condition in a mammalian subject, such as a human, comprising administering to said mammal an effective amount of a compound or a pharmaceutical composition according to the present invention.

Preferred is the method according to the present invention, wherein said autophagy-related disease or condition is selected from the group consisting of diseases and conditions of the CNS, neuronal diseases involving misfolded and/or non-folded proteins, neurodegenerative diseases, Huntington's disease, Alzheimer's disease, Parkinson's disease, epilepsy, brain or other neuronal cancer, Wilson Disease, viral infection and related neuronal diseases, Niemann-Pick type C (NPC) disease, autoimmune diseases, multiple sclerosis, stroke, Wegener's granulomatosis, neuropathic pain, post-operative phantom limb pain or postherpetic neuralgia, ALS, and spinal cord injury. Particularly preferred are ALS, Alzheimer's disease, and Parkinson's disease.

The dosage of the pharmaceutical composition to be administered according to the present invention can be appropriately selected according to the route of administration, the subject to be administered, the target disease and its severity, age, sex weight, individual differences and disease state. Dosage may be repeated several times a day.

According to the present invention, a mammalian subject can be preferably selected from a mouse, rat, cat, dog, rabbit, goat, sheep, horse, camel, lama, cow, monkey, a farm animal, a sport animal, and a pet, and a human.

In addition to the aforementioned compounds of the invention, the pharmaceutical composition as administered can contain two or more compounds according to the present invention and also other therapeutically active substances. In the method, the compound for use can be provided and/or is administered as a suitable pharmaceutical composition as discussed above. The compounds can be administered alone or in combination with other active compounds - for example with medicaments already known for the treatment of the aforementioned conditions and/or diseases, whereby in the latter case a favorable additive, amplifying or preferably synergistically effect is noticed.

In another aspect of the method according to the present invention the prevention and/or treatment further comprises detecting and/or monitoring in said subject a response of at least one autophagy-biomarker. The biomarker is preferably selected from the group of BECN1, and the ATG8/LC3 family, including LC3A, LC3B, LC3C), LC3-II, ULK1, p62, NBR1, ATG5 and ATG7.

This monitoring usually is performed on a biologically sample taken from the mammalian subject, and comprises commonly known tests, for example antibody based, PCR based, and the like. The tests are repeated over time, and can be compared to control samples and/or samples taken earlier from the mammalian subject. The results help the attending physician to maintain or modify the course of a treatment, usually based on the severity of the clinical symptoms of the autophagy-related disease and/or condition as treated.

It was surprisingly found that the compounds according to Formula I, as an autophagy inducer, were effective and exhibits many advantages compared to other autophagy-related treatment options, particularly as a combination treatment. In comparison with prior art compounds as tested (including data not shown), an improvement of the present invention lies in the unexpected observation that the compounds described herein are highly effective autophagy inducers (see Examples below).

The present invention relates to the following items:
Item 1. A compound according to the general Formula (I) 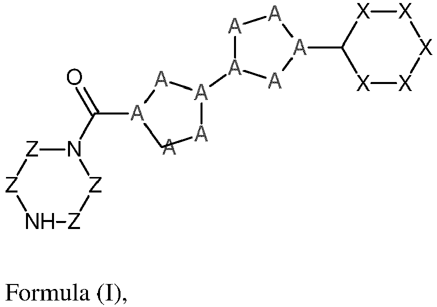 wherein
   A is independently selected from chemically possible combinations of C, CH, CH₂, N, NH, O, and S, optionally substituted with cyano,
   X is independently selected from chemically possible combinations of C, CH, CH₂, N and NH, optionally substituted with straight or branched C₂ to C₆ alkyl, straight or branched C₁ to C₄ hydroxyalkyl, such as, for example, -CH₂-CH₂-OH, straight or branched C₁ to C₄ hydroxyalkoxy, such as, for example, -O-CH₂-CH₂-OH), halo, trifluoromethyl, cyclopropyl, and cyano,
   Z is independently selected from chemically possible combinations of CH, CH₂, N and NH, optionally substituted with straight or branched C₂ to C₆ alkyl,
   and a physiologically acceptable salt, a solvate, a hydrate, an enantiomer or a polymorph thereof.
Item 2. A compound according to Item 1 according to the following formula II, 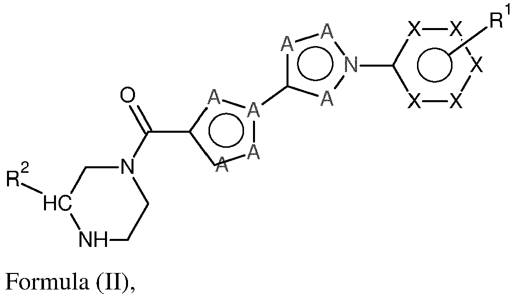 wherein
   A is as above,
   X is independently selected from chemically possible combinations of C, CH, CH₂, N and NH, and is optionally substituted with R¹,
   R¹ is independently selected from straight or branched C₂ to C₆ alkyl, straight or branched C₁ to C₄ hydroxyalkyl (for example, -CH₂-CH₂-OH), straight or branched C₁ to C₄ hydroxyalkoxy (for example, -O-CH₂-CH₂-OH), halo, trifluoromethyl, cyclopropyl, or cyano,
   R² is independently selected from H, or straight or branched C₂ to C₆ alkyl,
      and
   a physiologically acceptable salt, a solvate, a hydrate, an enantiomer or a polymorph thereof.
Item 3. A compound according to Item 1 or 2 according to the following formula III, 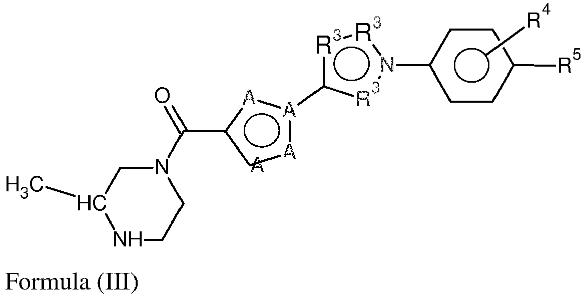 wherein
   A is as above,
   R⁵ is independently selected from H, straight or branched C₂ to C₆ alkyl, straight or branched C1 to C₄ hydroxyalkyl, such as, for example, -CH₂-CH₂-OH, straight or branched C₁ to C₄ hydroxyalkoxy, such as, for example, -O-CH₂-CH₂-OH, halo, trifluoromethyl, cyclopropyl, or cyano,
   R³ is independently selected from chemically possible combinations of C, CH, N and NH, optionally substituted with cyano
   R⁴ is independently selected from H or cyano
      and
   a physiologically acceptable salt, a solvate, a hydrate, an enantiomer or a polymorph thereof.
Item 4. A compound selected from the following group 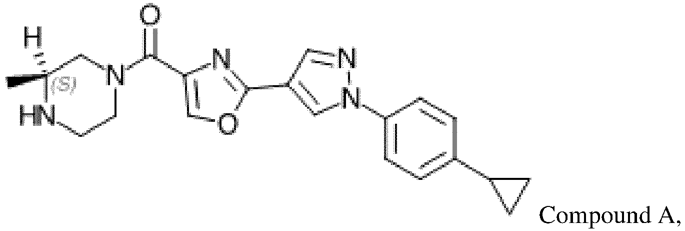 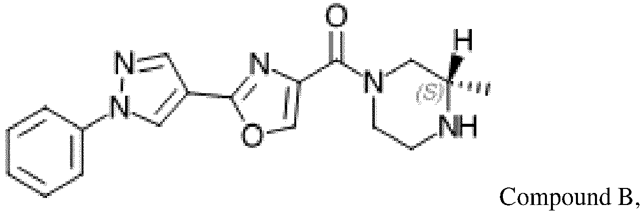 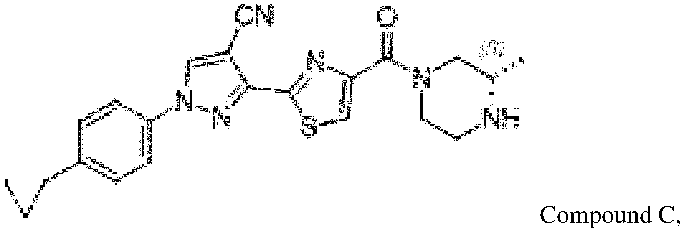 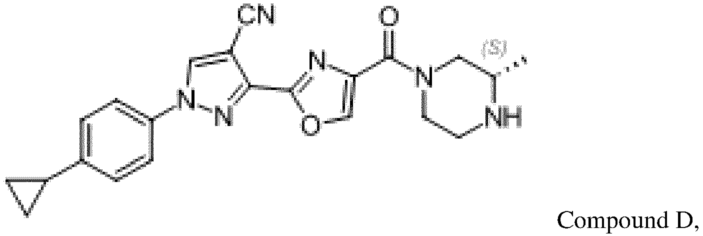 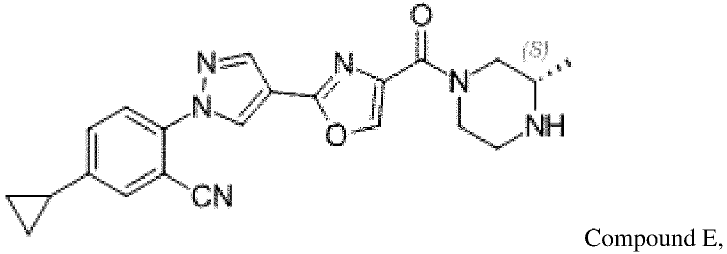 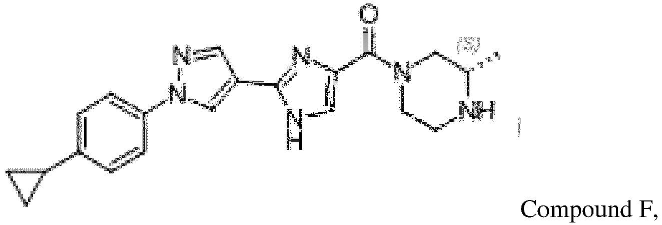 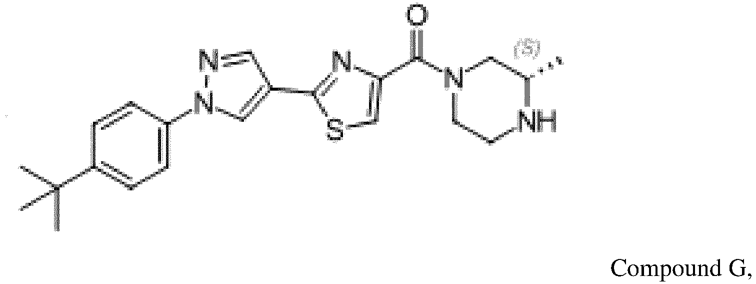 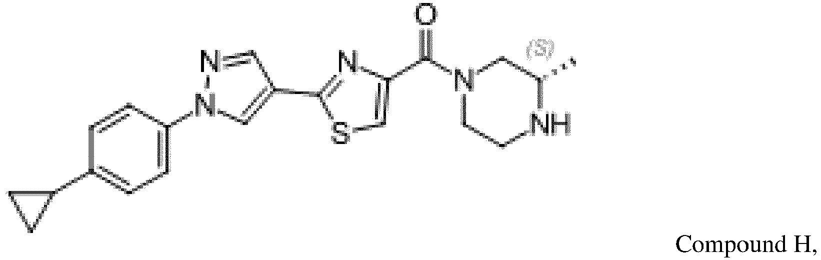 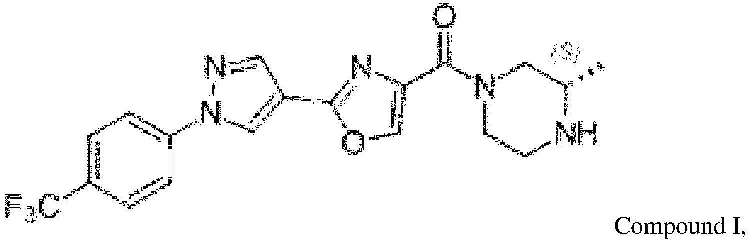 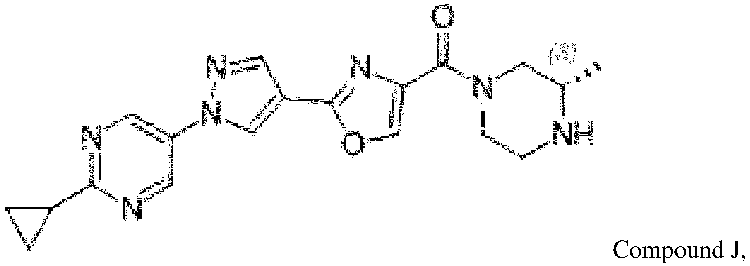 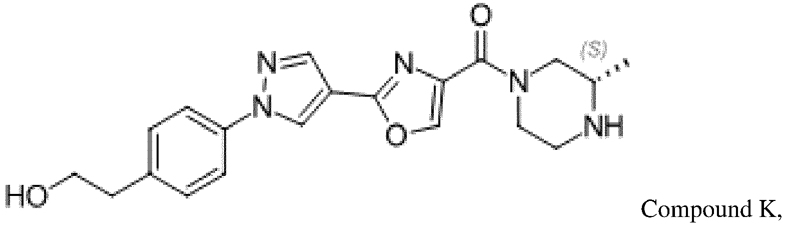 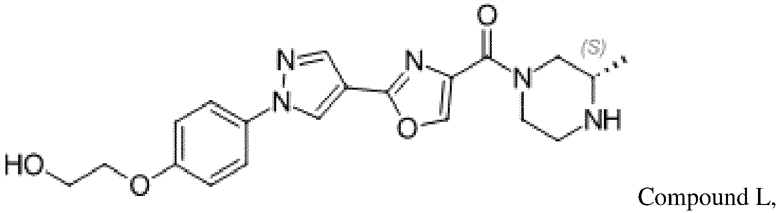 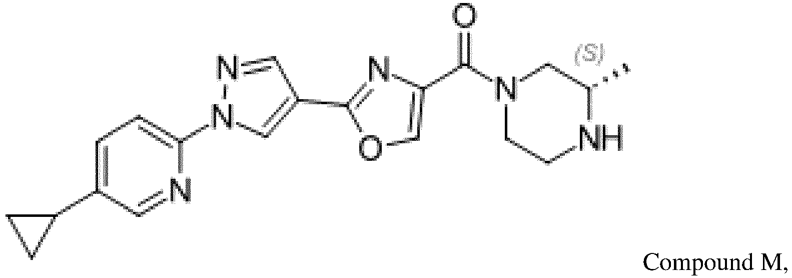 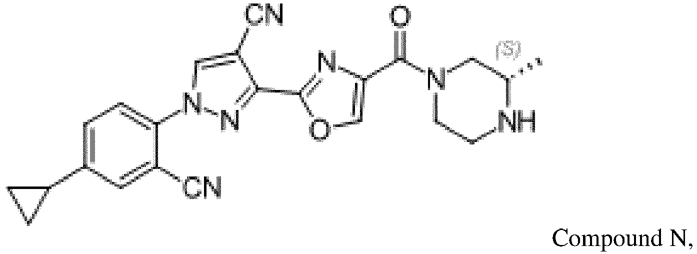 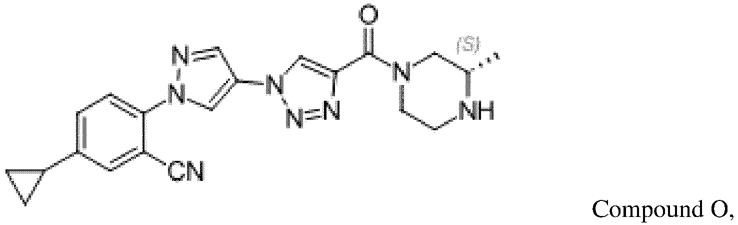 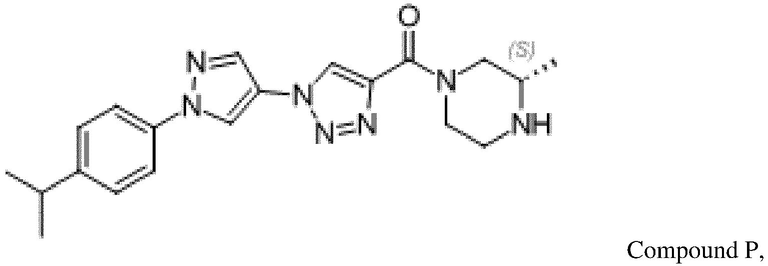 and
   a physiologically acceptable salt, a solvate, a hydrate, an enantiomer or a polymorph thereof.
Item 5. A pharmaceutical composition, comprising a pharmaceutically effective amount of the compound according to any one of Items 1 to 4, and a pharmaceutically acceptable carrier.
Item 6. The compound according to any one of Items 1 to 4 or the pharmaceutical composition according to Item 5 for use in the prevention and/or treatment of diseases in a mammalian subject, such as a human.
Item 7. The compound according to any one of Items 1 to 5 or the pharmaceutical composition according to Item 6 for use as an autophagy inducer, wherein preferably said use is cosmetic and/or in vitro.
Item The compound according to any one of Items 1 to 4 or the pharmaceutical composition according to Item 5 for use in the prevention and/or treatment of an autophagy-related disease or condition in a mammalian subject, such as a human.
Item 9. The compound for use according to Item 8, wherein said autophagy-related disease or condition is selected from the group consisting of diseases and conditions of the CNS, neuronal diseases involving misfolded and/or non-folded proteins, neurodegenerative diseases, Huntington's disease, Alzheimer's disease, Parkinson's disease, epilepsy, brain or other neuronal cancer, Wilson Disease, viral infection and related neuronal diseases, Niemann-Pick type C (NPC) disease, autoimmune diseases, multiple sclerosis, stroke, Wegener's granulomatosis, neuropathic pain, post-operative phantom limb pain or postherpetic neuralgia, ALS, and spinal cord injury.
Item 10. The compound for use according to Item 8 or 9, wherein said prevention and/or treatment comprises a combination of at least two compounds and/or a combination with at least one additional pharmaceutically active substance for said autophagy-related disease or condition.
Item 11. The compound for use according to any one of Items 8 to 10, wherein said prevention and/or treatment further comprises detecting and/or monitoring in said subject a response of at least one autophagy -biomarker, preferably selected from the group of BECN1, and the ATG8/LC3 family, including LC3A, LC3B, LC3C), LC3-II, ULK1, p62, NBR1, ATG5 and ATG7.
Item 12. The compound according to any one of Items 1 to 4 or the pharmaceutical composition according to Item 5 for use according to any one of Items 8 to 11, wherein said compound after administration thereof exhibits an increased ratio of concentration levels in the CNS and brain to levels outside the CNS and brain and/or when compared to a control.
Item 13. A method for preventing and/or treating an autophagy-related disease or condition in a mammalian subject, such as a human, comprising administering to said mammal an effective amount of at least one compound according to any one of Items 1 to 4 or of a pharmaceutical composition according to Item 5, the method preferably further comprising detecting and/or monitoring in said subject a response of at least one autophagy-biomarker, preferably selected from the group of BECN1, and the ATG8/LC3 family, including LC3A, LC3B, LC3C), LC3-II, ULK1, p62, NBR1, ATG5 and ATG7.
Item 14. The method according to Item 13, wherein said autophagy-related disease or condition is selected from the group consisting of diseases and conditions of the CNS, neuronal diseases involving misfolded and/or non-folded proteins, neurodegenerative diseases, Huntington's disease, Alzheimer's disease, Parkinson's disease, epilepsy, brain or other neuronal cancer, Wilson Disease, viral infection and related neuronal diseases, Niemann-Pick type C (NPC) disease, autoimmune diseases, multiple sclerosis, stroke, Wegener's granulomatosis, neuropathic pain, post-operative phantom limb pain or postherpetic neuralgia, ALS, and spinal cord injury.

The present invention will now be described further in the following examples, nevertheless, without being limited thereto. For the purposes of the present invention, all references as cited herein are incorporated by reference in their entireties.

### EXAMPLES

The following examples have been performed in part using preferred compounds as disclosed. Nevertheless, it should be understood that the present invention is nor in any way limited thereto, and the person of skill is readily able to adjust the conditions as described to other compounds according to the present invention.

The compounds of the present invention were synthesized from commercially available starting materials, preferably the compounds of the present invention by following the general synthetic procedures and Examples and are further exemplified by the following specific examples. Unless otherwise indicated in the following, the starting materials are obtained from commercial suppliers, which is indicated for example by the CAS numbers, and used without further purification. However, it is to be understood that in case the CAS number is not indicated below a starting material, this does not mean that the starting material was not obtained from commercial suppliers. Further, it is to be understood that the starting materials for the examples are either commercially available or are readily prepared by standard methods from known materials.

### Abbreviations used:

- Boc: tertiary-butoxycarbonyl
- tBu: tertiary butyl
- DCM: dichloromethane
- DIPEA: N,N-Diisopropylethylamine
- DMF: dimethylformamide
- DMSO: dimethylsulfoxide
- dppf: 1,1'-bis(diphenylphosphino)ferrocene
- eq: equivalents
- ESI: electrospray ionisation
- Hz: hertz
- H: hours
- HATU: 1-[Bis(dimethylamino)methylene]-1H-1,2,3-triazolo[4,5-b]pyridinium 3-oxide hexafluorophosphate
- HPLC: high performance liquid chromatography
- *J*: coupling constant in Hz
- LCMS: liquid chromatography-mass spectrometry
- min: minutes
- NMR: nuclear magnetic resonance
- N: normal (equivalents per litre)
- Ph: phenyl
- RT: retention time
- rt: room temperature
- SFC: supercritical fluid chromatography
- THF: tetrahydrofuran
- TLC: thin-layer chromatography
- vol: volumes
- X-Phos: 2-Dicyclohexylphosphin-2 ',4',6 '-triisopropylbiphenyl
- X-Phos Pd G2: Second generation XPhos Precatalyst (X-Phos aminobiphenyl palladium chloride precatalyst); Chloro(2-dicyclohexylphosphino-2',4',6'-triisopropyl-1,1'-biphenyl)[2-(2'-amino-1,1'-biphenyl)]palladium(II)

### Liquid chromatography mass spectrometry (LC-MS)

LC-MS spectra were recorded on a Waters Acquity Ultra performance LC system equipped with a Photodiode Array (PDA) detector with an attached Quadrupole Dalton (QDa) detector.

### Standard set-up:

***Column ID:*** X-BRIDGE BEH C18 2.1 × 50mm, 2.5µm
***Machine details*** Column temperature: 35°C, Auto sampler temperature: 5°C, Mobile Phase A: 0.1 % Formic acid in Milli Q water (pH= 2.70), Mobile Phase B : 0.1% Formic acid in Milli Q water : Acetonitrile (10:90).
***Mobile phase gradient details*** T = 0 min (97% A, 3% B) flow: 0.8 mL/min; T = 0.75 min (97% A, 3% B) flow: 0.8 mL/min; gradient to T = 2.7 min (2% A, 98% B) flow : 0.8 mL/min; gradient to T = 3 min (0% A, 100% B) flow : 1 mL/min; T = 3.5 min (0% A, 100% B) flow : 1 mL/min; gradient to T= 3.51 min (97% A, 3% B) flow: 0.8 mL/min; end of run at T = 4 min (97% A, 3% B), Flow rate: 0.8 mL/min, Run Time: 4 min, UV Detection Method: PDA.
***Mass parameter*** Probe:-ESI, Mode of Ionisation: Positive and Negative, Cone voltage : 30 V and 10 V, capillary voltage: 0.8 KV, Extractor Voltage: 1 V, Rf Lens: 0.1 V, Temperature of source: 120°C,Temperature of Probe: 600°C Cone Gas Flow: 50 L/Hr, Desolvation Gas flow: 850 L/Hr.

### High-performance liquid chromatography (HPLC)

For most compounds, HPLC was carried out using a SHIMADZU i-Series LC-2050C 3D system with a Photodiode Array (PDA) detector with one of the following columns: Sunfire C18 (150 x 4.6mm), 3.5µm; Atlantis C18 (150 x 4.6mm), 3.5µm (compound H)

### Standard set-up:

***Column temperature*** 25°C
***Auto sampler temperature*** 25°C
***Mobile Phase A*** 0.05% Trifluoroacetic acid in HPLC water
***Mobile Phase B*** 100% Acetonitrile
***Mobile phase gradient details*** T = 0 min (90% A, 10% B) flow : 1 mL/min; T = 7 min (10%A, 90% B) flow : 1 mL/min; gradient to T = 9 min (0% A, 100% B) flow : 1 mL/min; gradient to T = 14 min (0% A, 100% B) flow : 1 mL/min; T = 14.01 min (90% A, 10% B) flow : 1 mL/min; gradient to T= 17 min (90% A, 10% B) flow : 1 mL/min; end of run at T = 17 min (90% A, 10% B), Flow rate: 1 mL/min, Run Time: 17 min, UV Detection Method: PDA.

A different mobile phase gradient was used for **compound G:**
Mobile phase gradient details: T = 0 min (90% A, 10% B) flow : 1 mL/min; T = 7 min (10%A, 90% B) flow : 1 mL/min; gradient to T = 9 min (0% A, 100% B) flow : 1 mL/min; gradient to T = 14 min (0% A, 100% B) flow : 1 mL/min; T = 14.01 min (90% A, 10% B) flow : 1 mL/min; gradient to T= 17 min (90% A, 10% B) flow : 1 mL/min; end of run at T = 17 min (90% A, 10% B), Flow rate: 1 mL/min, Column Oven temperature: Ambient., Runtime: 17min, Diluent: Water:IPA:Methanol (20:40:40), Sample Preparation in concentration: 800ppm.

For compound L, HPLC was carried out using a Waters Alliance e2695 equipped with a 2998 Photodiode Array (PDA) detector and a Sunfire C18 (150 x 4.6mm), 5µm column.

### Set-up used:

***Column temperature*** 25°C
***Auto sampler temperature*** 25°C
***Mobile Phase A*** 0.1% Formic acid in HPLC water
***Mobile Phase B*** 100% ACETONITRILE
***Mobile phase gradient details*** T = 0 min (10% A, 90% B) flow : 1 mL/min; T = 7 min (90%A, 10% B) flow : 1 mL/min; gradient to T = 9 min (100% A, 0% B) flow : 1 mL/min; gradient to T = 14 min (100% A, 0% B) flow : 1 mL/min; T = 14.01 min (10% A, 90% B) flow : 1 mL/min; gradient to T= 17 min (10% A, 90% B) flow : 1 mL/min; end of run at T = 17 min (10% A, 90% B), Flow rate: 1 mL/min, Run Time: 17 min, UV Detection Method: PDA.

For compound J, HPLC was carried out using a Agilent Infinity II G6125C system with a Photodiode Array (PDA) detector with an X-bridge C18 (150 x 4.6mm), 3.5µm; column.

### Set-up used:

***Column temperature*** 25°C
***Auto sampler temperature*** 25°C
***Mobile Phase A*** 0.1% Formic acid in HPLC water
***Mobile Phase B*** 100% Acetonitrile
***Mobile phase gradient details*** T = 0 min (90% A, 10% B) T = 7 min (10%A, 90% B) T = 9 min (0% A, 100% B) T = 14 min (0% A, 100% B) T = 14.01 min (90% A, 10% B) gradient to T= 17 min (90% A, 10% B). Flowrate: 1.0 ml/min, Runtime: 17min, UV Detection Method: PDA.

### Chiral high-performance liquid chromatography (chiral HPLC)

For most compounds Chiral HPLC was carried out on was conducted with Waters Acquity UPC2 system with PDA detector.

### Standard set-up used:

***Column:*** Chiralpak IG (250mm x 4.6mm, 5µm)
***Column temperature*** 40°C
***Auto sampler temperature*** 25°C
***Mobile Phase A*** Liq. CO2
***Mobile Phase B*** 0.1% Methanolic Ammonia in Methanol:Acetonitrile(50:50)
***Mobile phase gradient details*** T = 0 min (95% A, 5% B) T = 5 min (50%A, 50% B) gradient to T= 12 min (50% A, 50% B); Flow rate: 2 mL/min; Runtime: 12 min; Back Pressure: 100 bar

For compound A, the flow rate was 4mL/min and the runtime was 15 min.

For compounds D, E, I, and N, an alternative column was used ((CHIRALPAK IG-3 (100 x 3mm), 3 mm)), with a flow rate of 2 mL/min and a runtime of 5 min.

For compound F, an alternative column was used ((CHIRALPAK IC-3 (100 x 3mm), 3mm)) with a runtime of 6 min.

For compound M and alternative column was used ((CHIRALPAK IH (250x 4.6mm), 5mm)) with a runtime of 10 min.

For compound J, an alternative column was used ((CHIRALPAK IB-N (250 x 4.6mm), 5mm) with a runtime 10 min.

For compound P, an alternative column was used ((Chiral ICT (100 x 3mm), 3mm)) with a runtime of 5 min.

For compound G, Chiral HPLC was carried out using a Waters SFC Investigator system with 2998 PDA detector with CHIRALPAK IB-N (250 x 4.6mm), 5 µm. Set-up used:
***Column temperature*** 40°C
***Auto sampler temperature*** 25°C
***Mobile Phase A*** Liq. CO2
***Mobile Phase B*** 0.1% Di-ethyl amine in Methanol-Acetonitrile (50-50)
***Mobile phase gradient details*** 0 min> 5% Co-solvent 5 min> 50% Co-solvent 10 min> 50% Co-solvent; Flow rate: 4 mL/min; Analysis time:10 min.

For compound K, Chiral HPLC was carried out using a Shimadzu LC-20 AD system with DAD detector with a CHIRALPAK AD-H (250 x 4.6mm) 5mm column.

### Set-up used:

***Column temperature*** 25°C
***Auto sampler temperature*** 25°C
***Mobile Phase A*** 0.1% Methanolic Ammonia in n-Heptane
***Mobile Phase B*** 0.1% Methanolic Ammonia in 2-Propanol:Acetonitrile(70-30)
***Mobile phase gradient details*** T = 0 min (80% A, 20% B), T = 5 min (80%A, 20% B), T= 10 min (50% A, 50% B), T = 15 min (30% A, 70%B), T = 25 min (30% A, 70%B), T = 25.01 min (80% A, 20%B), T = 30 min (80% A, 20%B),

### General experimental procedures

### General experimental procedure 1A (GP1A)

### Schematic overview of synthesis. X = unspecified heteroatom; Y = S or O; Z = C or N (chemically allowed combinations); R = various.

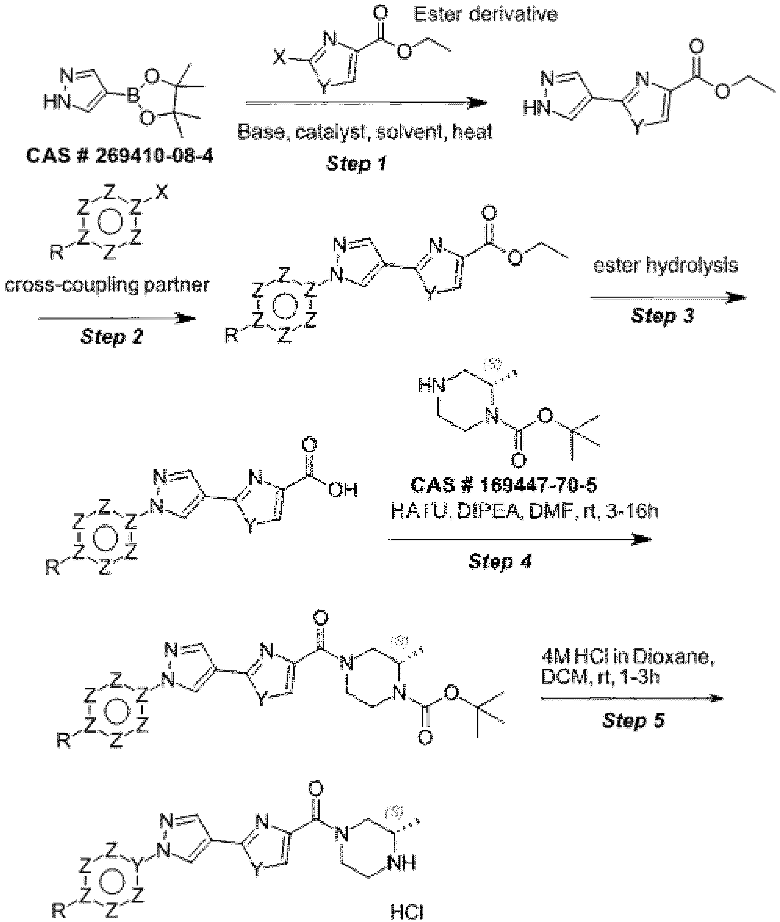

### Typical general conditions

Step 1: To a stirred solution of the ester derivative (1.0 eq) in 1,4-dioxane (1.8 mL per mmol ester derivative, 8 vol) at room temperature, 4-(4,4,5,5-tetramethyl-1,3,2-dioxaborolan-2-yl)-1H-pyrazole (1.2 eq) and potassium carbonate (3.9 eq) in water (0.5 mL per mmol of ester derivative) were added. The reaction mixture was purged with nitrogen gas for 25 min and then [1,1'-bis(diphenylphosphino)ferrocene]dichloropalladium(II) (0.1 eq) were added. The reaction mixture was heated at 110°C for 1h using microwave irradiation. The progress of the reaction was monitored by TLC and LCMS analysis. After completion of the reaction, the reaction mixture was diluted with water (180 mL per mmol ester derivative) and extracted with CH₂Cl₂ (2 x 180 mL per mmol of ester derivative). The combined organic fractions were dried over sodium sulphate and concentrated under reduced pressure. The crude material was purified by flash column chromatography to provide Intermediate 1.

### Step 2: Step 2A: Metal-catalysed cross coupling

To a stirred solution of the cross-coupling partner (1.0 eq) in 1,4-dioxane (4 mL per mmol of cross-coupling partner, 20 vol) at room temperature, potassium carbonate (1.5 eq) was added. The reaction was stirred for 5 minutes then copper iodide (0.5 eq) and trans-n,n'-dimethyl cyclohexane-1,2-diamine (0.6 eq) were added at 0°C. The reaction was stirred for 10 minutes then Intermediate 1 (1.0 eq) was added. The reaction mixture was stirred at 120°C for 2h. The progress of the reaction was monitored by TLC and LCMS analysis. After completion of the reaction, the reaction mixture was poured into cold water (80 mL per mmol of cross coupling partner) and extracted with EtOAc (3 x 80 mL per mmol of cross coupling partner). The combined organic fractions were dried over sodium sulphate and concentrated under reduced pressure. The crude material was purified by flash column chromatography to provide Intermediate 2.

### Step 2B: Cross coupling without a metal catalyst

To a stirred solution of Intermediate 1 (1 eq) in DMF (2mL per mmol of Intermediate 1, 10 vol) at 0 °C, Sodium hydride (60% in mineral oil) (2 eq) was added. The reaction was stirred at 0 °C for 30 min. The coupling partner (1.5 eq) was added at 0 °C and stirred for 10 minutes at room temperature. Then the reaction mixture was stirred at 85 °C for 16 h. The progress of the reaction was monitored by TLC and LCMS analysis. After completion of the reaction, the reaction mixture was poured in ice cold water (50 vol) and extracted with EtOAc (3 x 10 mL per mmol of Intermediate 1). The combined organic fractions were dried was dried over sodium sulphate and concentrated under reduced pressure. The crude material was purified by column chromatography to provide Intermediate 2.

Step 3: To a stirred solution of Intermediate 2 (1.0 eq) in THF:ethanol:water (ratio 1: 1: 1, total volume of 3 mL per mmol of Intermediate 2) at room temperature, LiOH (3.0 eq) was added. The reaction mixture was stirred at room temperature for 3h. The progress of the reaction was monitored by TLC and LCMS analysis. After completion of the reaction, the reaction mixture was concentrated under reduced pressure. The residue was dissolved in water (0.3 mL per mmol of Intermediate 2) and was acidified slowly with a saturated solution of citric acid to pH 4-5. A solid precipitate was formed which was isolated by filtration and dried under reduced pressure to provide Intermediate 3.

Step 4: To a stirred solution of Intermediate 3 (1.0 eq) in DMF (3 mL per mmol of Intermediate 3) at room temperature, HATU (1.5 eq) was added. The reaction was stirred at room temperature for 1h. Then, tert-butyl (S)-2-methylpiperazine-1-carboxylate (1 eq) and DIPEA (3 eq) were added to the reaction mixture at room temperature. The reaction mixture was stirred at room temperature for 3 h. The progress of the reaction was monitored by TLC and LCMS analysis. After completion of the reaction, the reaction mixture was poured into ice cold water (25 mL per mmol of Intermediate 3). A precipitate formed which was isolated by filtration, washed with ice cold water (42 mL per mmol of Intermediate 3) and dried under reduced pressure to provide Intermediate 4.

Step 5: To a stirred solution of Intermediate 4 (1.0 eq) in CH₂Cl₂ (5 mL per mmol of Intermediate 4, 10 vol) at 0°C, 4M HCl in dioxane (1.2 mL per mmol of Intermediate 4, 3.0 vol) was added. The reaction mixture was stirred at room temperature for 2h. The progress of the reaction was monitored by TLC and LCMS analysis. After completion of the reaction, the reaction mixture was concentrated under reduced pressure and the crude material purified by trituration using diethyl ether (3 x 20 mL per mmol of Intermediate 4) to provide the target compound.

### General experimental procedure 1B (GP1B)

### Schematic overview of synthesis. X = unspecified heteroatom; Y = S or O; Z = C or N (chemically allowed combinations); R = various.

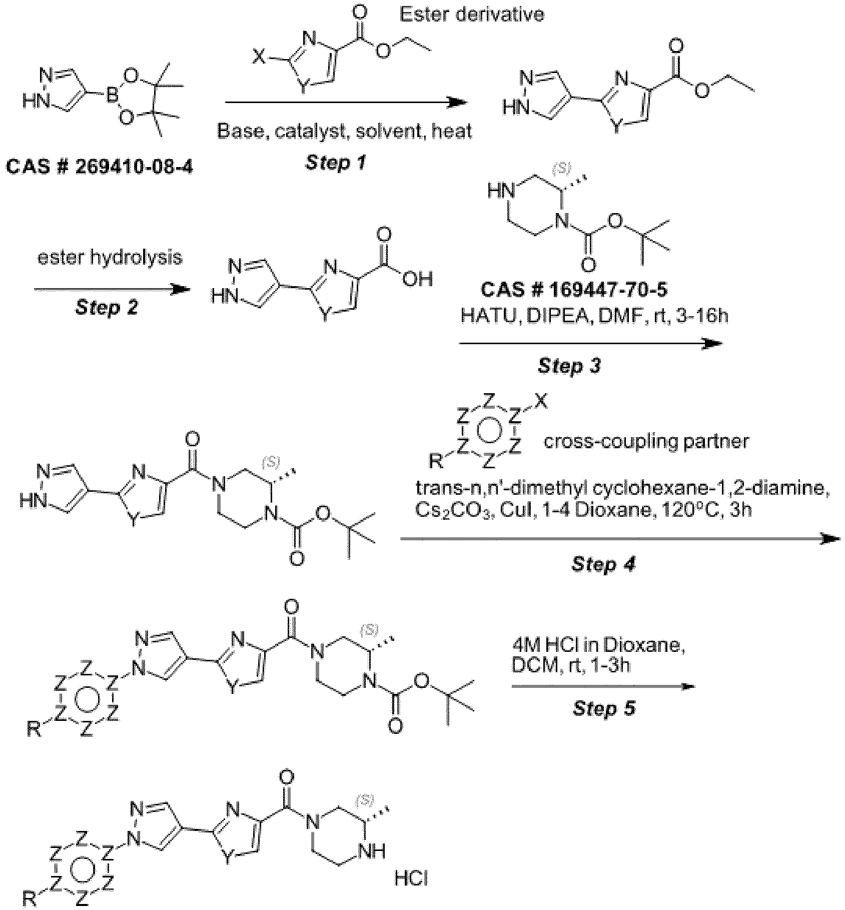

### Typical general conditions

Step 1 - See General experimental procedure 1A, step 1;

Step 2 - See General experimental procedure 1A, step 3;

Step 3 - See General experimental procedure 1A, step 4;

Step 4: To a stirred solution of the product isolated in Step 3 (1 eq) in 1-4 Dioxane (5 mL per mmol of starting material, 20 vol) at room temperature, caesium carbonate (1.9 eq) followed by copper iodide (0.6 eq) and trans-n n'-dimethyl cyclohexane-1,2-diamine (1 eq) were added. The reaction mixture was stirred for 10 minutes at room temperature. The cross coupling partner (1.3 eq) was then added and the reaction mixture was stirred at 120°C for 3h. The progress of reaction was monitored by TLC and LCMS analysis. After completion of the reaction, the reaction mixture was poured into cold water (60 mL per mmol of starting material) and extracted with EtOAc (3 x 60 mL per mmol of starting material). The combined organic fractions were and dried over sodium sulphate and concentrated under reduced pressure. The crude material was purified by column chromatography to provide the desired product.

Step 5 - See General experimental procedure 1A, step 5.

### Preparation of 5-cyclopropyl-2-fluorobenzonitrile

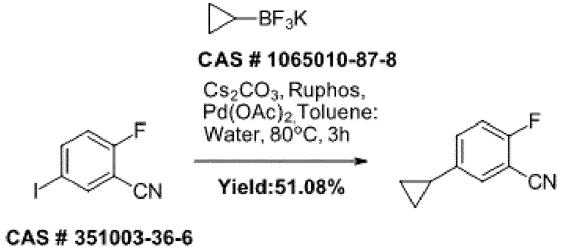

To a stirred solution of 2-fluoro-5-iodobenzonitrile (0.8g, 3.23mmol) in toluene (7.2mL, 9V): water (0.8mL, 1V) at room temperature, Potassium cyclopropyltrifluoroborate (1.42g, 9.71mmol) and Cs₂CO₃ (3.14g, 9.69mmol) were added. The reaction mixture was purged with nitrogen gas for 15min and Pd(OAc)₂ (0.07g, 0.32mmol) and Ruphos (0.30g, 0.64mmol) were added at room temperature. The reaction mixture was heated at 80°C for 3h. The progress of the reaction was monitored by TLC analysis. After completion of the reaction, the reaction mixture was poured into Ethyl acetate (200mL) and filtered through Celite^{®}. The filtrate was diluted with water (150mL) and extracted with ethyl acetate (2 x 100mL). The combined organic fractions were dried over Na₂SO₄ and concentrated under reduced pressure to provide 5-cyclopropyl-2-fluorobenzonitrile (0.8g, 51.08% yield) as a light brown liquid, which was used without further purification. Note that three batches of this reaction were carried out in parallel (on the same scale as described here) and the product yield quoted here is based on the combination of all these three batches.

### Examples of compounds prepared by the general experimental procedures

### [(S)-3-methyl-1-piperazinyl]{2-[1-(p-cyclopropylphenyl)-4-pyrazolyl]-1,3-oxazol-4-yl}methanone hydrochloride (compound A)

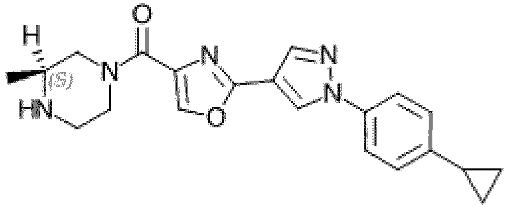

Prepared by GP1A using ethyl 2-bromooxazole-4-carboxylate (0.5g, 2.27mmol) is step-1 and 1-bromo-4-cyclopropylbenzene in step-2A. The title compound was isolate as an off-white solid.
**LCMS [ESI, M+1]**: 378.01 (RT: 1.27 min, Purity: 100%),
**HPLC:** RT: 3.96 min, Purity: 97.79%.
**Chiral HPLC:** RT: 10.66 min, Purity: 98.55%.
**¹H NMR (400 MHz, CD₃OD**) δ 8.85 (s, 1H), 8.45 (s, 1H), 8.22 (s, 1H), 7.72 (d, J = 8.6 Hz, 2H), 7.25 (d, J = 8.6 Hz, 2H), 5.20 (bs, 1H), 4.68 (bs, 1H), 3.61 (bs, 1H), 3.54 (dd, J = 10.0, 2.8 Hz, 3H), 3.31 - 3.32 (s, 1H), 2.03 - 1.98 (m, 1H), 1.43 (d, J = 6.4 Hz, 3H), 1.07 - 1.02 (m, 2H), 0.77 - 0.75 (m, 2H).

### [(S)-3-methyl-1-piperazinyl]{2-[1-(5-cyclopropyl-2-pyridyl)-4-pyrazolyl]-1,3-oxazol-4-yl}methanone hydrochloride (compound M)

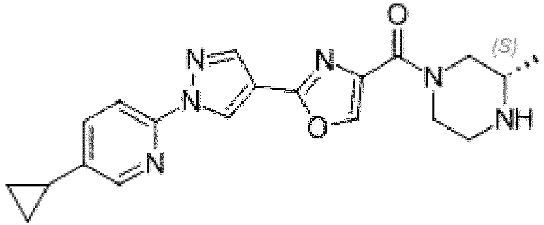

Prepared by general procedure 1A using ethyl 2-bromooxazole-4-carboxylate (10.0 g, 45.45 mmol) in Step-1 and 5-cyclopropyl-2-fluoropyridine in Step-2B. The title compound was isolated as an off-white solid (0.13 g).
**LCMS [ESI, M+1]**: 379.1 (RT: 1.232 min, Purity: 100 %),
**HPLC Purity:** RT: 5.93 min, Purity: 99.84 %.
**Chiral HPLC Purity:** RT: 4.85 min, Purity: 99.47 %.
**¹H NMR (400 MHz, CD₃OD**) δ 9.13 (s, 1H), 8.46 (s, 1H), 8.32 (s, 1H), 8.25 (s, 1H), 7.92 (d, J = 8.6 Hz, 1H), 7.64 (dd, J = 8.5, 2.1 Hz, 1H), 5.18 (bs, 1H), 4.71 (bs, 1H), 3.58 (bs, 1H), 3.54 - 3.51 (m, 3H), 3.15 (bs, 1H), 2.06 - 2.02 (m, 1H), 1.42 (d, J = 6.4 Hz, 3H), 1.12 - 1.09 (m, 2H), 0.82 - 0.80 (m, 2H).

### [(S)-3-methyl-1-piperazinyl]{2-[1-(2-cyclopropyl-5-pyrimidinyl)-4-pyrazolyl]-1,3-oxazol-4-yl}methanone hydrochloride (compound J)

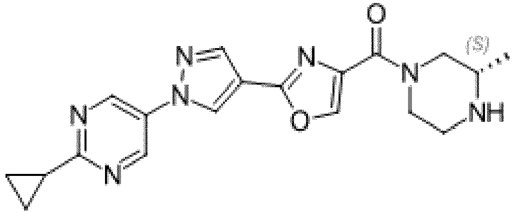

Prepared by GP 1B using ethyl 2-bromooxazole-4-carboxylate (60.0 g, 271.24 mmol) in step-1 and 5-bromo-2-cyclopropylpyrimidine in step-4. The title compound was isolated as a light yellow solid (0.13 g).
**LCMS [ESI, M+1]**: 380.06 (RT: 1.134 min, Purity: 98.28%),
**HPLC Purity:** RT: 5.39 min, Purity: 97.65%.
**Chiral HPLC Purity:** RT: 6.09 min, Purity: 100%.
**¹H NMR (400 MHz, CD₃OD**) δ 9.17 (s, 2H), 9.05 (s, 1H), 8.48 (s, 1H), 8.33 (s, 1H), 5.16 (bs, 1H), 4.66 (bs, 1H), 3.54 (bs, 1H), 3.53 - 3.51 (m, 3H), 3.33 - 3.32 (m, 1H), 2.38 - 2.31 (m, 1H), 1.43 (d, J = 6.4 Hz, 3H), 1.20 - 1.19 (m, 2H), 1.19 (dd, J = 5.1, 3.5 Hz, 2H).

### [(S)-3-methyl-1-piperazinyl](2-{1-[p-(2-hydroxyethyl)phenyl]-4-pyrazolyl}-1,3-oxazol-4-yl)methanone hydrochloride (compound K)

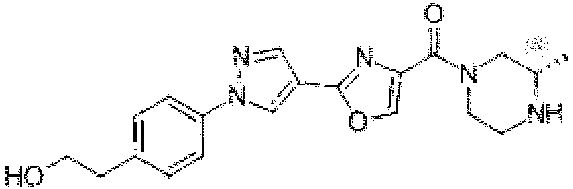

Prepared by general procedure 1B using ethyl 2-bromooxazole-4-carboxylate (60.0 g, 271.24 mmol) in step-1 and 2-(4-bromophenyl)ethan-1-ol in step-4. The title compound was isolated as a light brown solid (0.13 g).
**LCMS [ESI, M+1]**: 382.01 (RT: 1.023 min, Purity: 98.89 %),
**HPLC Purity:** RT: 4.84 min, Purity: 96.65 %.
**Chiral HPLC Purity:** RT: 12.73 min, Purity: 96.18 %.
**¹H NMR (400 MHz, CD₃OD**) δ 8.87 (s, 1H), 8.45 (s, 1H), 8.22 (s, 1H), 7.77 (d, J = 8.5 Hz, 2H), 7.42 (d, J = 8.5 Hz, 2H), 5.17 (bs, 1H), 4.66 (bs, 1H), 3.81 (t, J = 6.8 Hz, 2H), 3.53 - 3.50 (m, 3H), 3.31 - 2.99 (m, 2H), 2.90 (t, J = 6.8 Hz, 2H), 1.42 (d, J = 6.4 Hz, 3H).

### [(S)-3-methyl-1-piperazinyl](2-{1-[p-(2-hydroxyethoxy)phenyl]-4-pyrazolyl}-1,3-oxazol-4-yl)methanone hydrochloride (compound L)

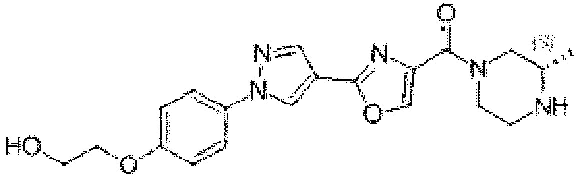

Prepared by general procedure 1B using ethyl 2-bromooxazole-4-carboxylate (60.0g, 271.24 mmol) in step-1 and 2-(4-bromophenoxy)ethan-1-ol in step-4. The title compound was isolated as an off-white sticky solid.
**LCMS [ESI, M+1]**: 398.06 (RT: 1.029 min, Purity: 100 %),
**HPLC Purity:** RT: 4.73 min, Purity: 98.63 %.
**Chiral HPLC Purity:** RT: 5.80 min, Purity: 100%.
**¹H NMR (400 MHz, CD₃OD**) δ 8.79 (s, 1H), 8.44 (s, 1H), 8.20 (s, 1H), 7.74 (d, J = 7.2 Hz, 2H), 7.12 (d, J = 7.2 Hz, 2H), 5.14 (bs, 1H), 4.89 (bs, 1H), 4.12 (t, J = 4.8 Hz, 2H), 3.91 (t, J = 4.4 Hz, 2H), 3.63 (bs, 1H), 3.53 - 3.50 (m, 3H), 3.31 - 3.25 (m, 1H), 1.42 (d, J = 6.4 Hz, 3H).

### [(S)-3-methyl-1-piperazinyl](2-{1-[p-(trifluoromethyl)phenyl]-4-pyrazolyl}-1,3-oxazol-4-yl)methanone (compound I)

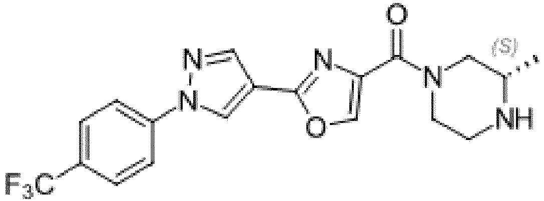

Prepared by general procedure 1B using ethyl 2-bromooxazole-4-carboxylate (60.0 g, 271.24 mmol) in step-1 and 1-bromo-4-(trifluoromethyl)benzene in step-4. The title compound was isolated as an off-white solid.
**LCMS [ESI, M+1]**: 406.01 (RT: 1.377 min, Purity: 99.59%),
**HPLC Purity:** RT: 6.38 min, Purity: 99.16%.
**Chiral HPLC Purity:** RT: 2.12 min, Purity: 100%.
**¹H NMR (400 MHz, MeOD)** δ 9.08 (s, 1H), 8.48 (s, 1H), 8.31 (s, 1H), 8.13 (d, J = 8.5 Hz, 2H), 7.88 (d, J = 8.6 Hz, 2H), 5.17 (Bs, 1H), 4.68 (bs, 1H), 3.68 (bs, 1H), 3.53 (dd, J = 9.9, 2.9 Hz, 2H), 3.33 (s, 1H) 3.32 - 3.07 (m, 1H), 1.43 (d, J = 6.4 Hz, 3H).

### Full synthetic details for a representative set of compounds

### Synthesis of [(S)-3-methyl-1-piperazinyl][2-(1-phenyl-4-pyrazolyl)-1,3-oxazol-4-yl]methanone hydrochloride (compound B)

### Overall synthetic scheme

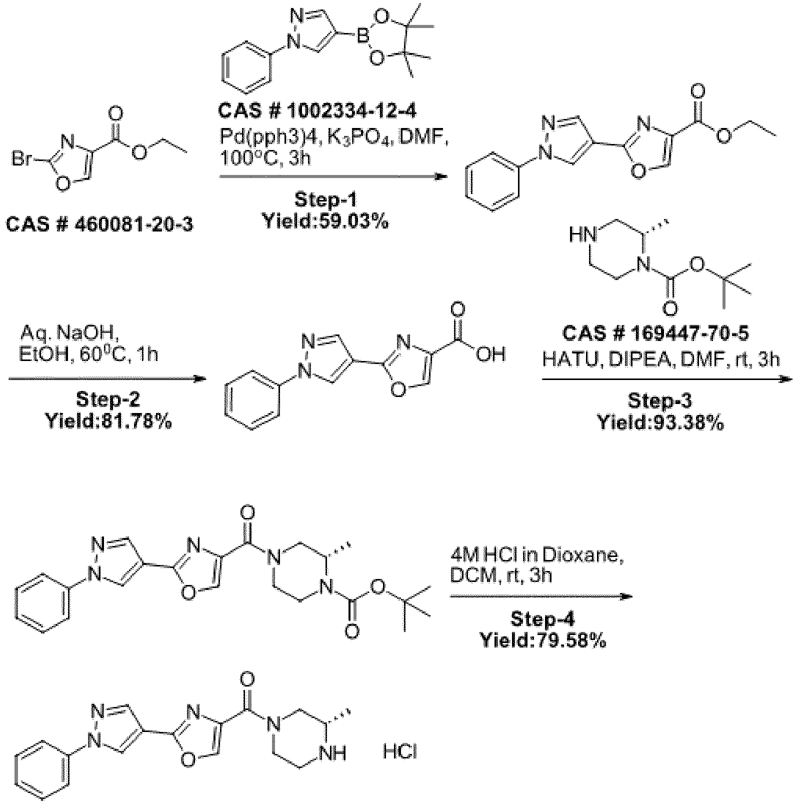

### Synthetic procedures

### Step-1: Synthesis of ethyl 2-(1-phenyl-1H-pyrazol-4-yl)oxazole-4-carboxylate

To a stirred solution of ethyl 2-bromooxazole-4-carboxylate (5.0 g, 22.72 mmol) and 1-phenyl-4-(4,4,5,5-tetramethyl-1,3,2-dioxaborolan-2-yl)-1H-pyrazole (7.3 g, 27.27 mmol) in DMF (50 mL) at room temperature, potassium phosphate (14.4 g, 68.16 mmol) was added. The reaction mixture was purged with nitrogen gas for 30 min. Tetrakis (triphenylphosphine)palladium (2.6 g, 2.27 mmol) was added and the reaction mixture was heated at 100°C for 3h. The progress of the reaction was monitored by TLC. After completion of the reaction, the reaction mixture was diluted with cold water (700 mL) and extracted with ethyl acetate (2 x 300 mL). The combined organic fractions were dried over Na₂SO₄ and concentrated under reduced pressure. The crude material was purified by column chromatography (24% ethyl acetate in hexane) to provide ethyl 2-(1-phenyl-1H-pyrazol-4-yl) oxazole-4-carboxylate (3.8g, 59.03% yield) as a light brown sticky solid.

**LCMS [ESI, M+1]**: 284.0 (RT: 1.688min, Purity: 75.39%),

### Step-2: Synthesis of 2-(1-phenyl-1H-pyrazol-4-yl)oxazole-4-carboxylic acid

To a stirred solution of ethyl 2-(1-phenyl-1H-pyrazol-4-yl) oxazole-4-carboxylate (3.8 g, 13.42 mmol) in EtOH (38 mL, 10V) at room temperature, NaOH solution (1.6 g, 40.28 mmol) in water (40 mL) was added. The reaction mixture was stirred at 60°C for 1h. The progress of the reaction was monitored by TLC and LCMS analysis. After completion of the reaction, the reaction mixture was distilled out, then diluted with water (30 mL). The solution was acidified to pH 2 by the addition of a saturated citric acid solution. A precipitate was formed which was isolated by filtration, washed with Diethyl ether (30 mL) and dried under reduced pressure to provide 2-(1-phenyl-1H-pyrazol-4-yl) oxazole-4-carboxylic acid (2.8g, 81.78% yield) as a white solid.

**LCMS [ESI, M+1]**: 255.9 (RT: 1.334 min, Purity: 92.14%)

### Step-3: Synthesis of tert-butyl (S)-2-methyl-4-(2-(1-phenyl-1H-pyrazol-4-yl)oxazole-4-carbonyl)piperazine-1-carboxylate

To a stirred solution of 2-(1-phenyl-1H-pyrazol-4-yl) oxazole-4-carboxylic acid (2.8 g, 11.02 mmol) in DMF (28 mL) at room temperature, HATU (6.2 g, 16.53 mmol) and DIPEA (4.2 g, 33.06 mmol) were added. The reaction mixture was stirred at room temperature for 30 min, then tert-butyl (S)-2-methylpiperazine-1-carboxylate (2.4g, 12.12mmol) was added to reaction mixture at room temperature. The reaction mixture was stirred at room temperature for 3 h. The progress of the reaction was monitored by TLC and LCMS analysis. After completion of the reaction, the reaction mixture was poured into cold water (1000 mL) and extracted with Ethyl Acetate (300 mL x 2). The combined organic fractions were concentrated under reduced pressure and the crude material purified by column chromatography (20% Ethyl acetate in Hexane) to provide tert-butyl 3-(2-(1-phenyl-1H-pyrazol-4-yl)-N-propyloxazole-4-carboxamido) pyrrolidine-1-carboxylate (4.4g, 93.38% yield) as off white solid.

**LCMS [ESI]:** 382.0 (M-56), 338.0 (M-Boc) (RT: 1.86 min, Purity: 100%).

### Step-4: Synthesis of (S)-(3-methylpiperazin-1-yl)(2-(1-phenyl-1H-pyrazol-4-yl)oxazol-4-yl)methanone hydrochloride (compound B)

To a stirred solution of tert-butyl 3-(2-(1-phenyl-1H-pyrazol-4-yl)-N-propyloxazole-4-carboxamido) pyrrolidine-1-carboxylate (2.5 g, 5.72 mmol) in CH₂Cl₂ (25 mL) at 0 °C, 4M HCl in Dioxane (12.5 mL, 5V) was added. The reaction mixture was stirred at room temperature for 3h. The progress of the reaction was monitored by TLC. After completion of the reaction, the reaction mixture was concentrated under reduced pressure. The crude material was diluted with HPLC-grade water (20 mL) and extracted with EtOAc (10 mL x 2). The aqueous layer was lyophilized to provide (S)-(3-methylpiperazin-1-yl)(2-(1-phenyl-1H-pyrazol-4-yl)oxazol-4-yl)methanone hydrochloride (compound B): 1.7 g, 79.58% yield) as an off-white solid.
**LCMS [ESI, M+1]**: 338.0 (RT: 1.173 min, Purity: 100%),
**HPLC Purity:** RT: 5.34 min, Purity:100%,
**Chiral HPLC:** RT: 7.54 min, Purity:100%,
**¹H NMR (400 MHz, CD₃OD**) **δ** 8.92 (s, 1H), 8.46 (s, 1H), 8.25 (s, 1H), 7.87 (d, J = 8.0 Hz, 2H), 7.56 (t, J = 7.8 Hz, 2H), 7.42 (t, J = 7.4 Hz, 1H), 5.12 (bs, 1H), 4.68 (bs, 1H), 3.54-3.50 (m, 1H), 3.51-3.40 (m, 3H), 3.25 - 3.31 (m, 1H), 1.42 (d, J = 6.4 Hz, 3H).

### Synthesis of 3-(4-{[(S)-3-methyl-1-piperazinyl]carbonyl}-1,3-thiazol-2-yl)-1-(p-cyclopropylphenyl)-4-pyrazolecarbonitrile hydrochoride (compound C)

### Overall synthetic scheme

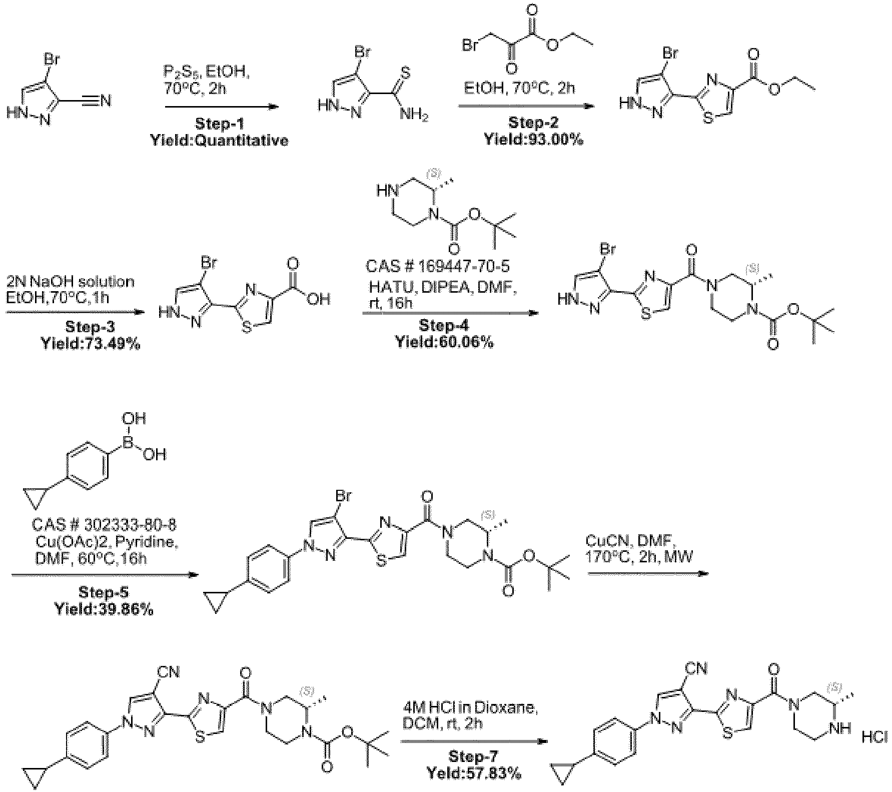

### Synthetic procedures

### Step-1: Synthesis of 4-bromo-1H-pyrazole-3-carbothioamide

To a stirred solution of 4-bromo-1H-pyrazole-3-carbonitrile (20.0 g, 116.2 mmol) in EtOH (200 mL, 10V) at 0 °C, P₂S₅ (52.85 g, 232.5 mmol) was added. The reaction mixture was stirred at 70 °C for 1h. The progress of the reaction was monitored by TLC and LCMS analysis. After completion of the reaction, the reaction mixture was concentrated under reduced pressure. The crude material was poured into a sat. NaHCOs solution (800mL) and extracted with EtOAc (2 x 500 mL). The combined organic fractions were dried over Na₂SO₄and concentrated under reduced pressure to provide 4-bromo-1H-pyrazole-3-carbothioamide (22.0g, Quantitative yield) as a yellow solid, which was used directly in the next step without further purification.

**LCMS [ESI, M, M+2]:** 205.7, 207.8 (RT: 1.047 min, Purity: 83.88%).

### Step-2: Synthesis of ethyl 2-(4-bromo-1H-pyrazol-3-yl) thiazole-4-carboxylate

To a stirred solution of 4-bromo-1H-pyrazole-3-carbothioamide (22.0 g, 106.76 mmol) in EtOH (220 mL) at room temperature, ethyl 3-bromo-2-oxopropanoate (27.06 g, 138.79 mmol) was added. The reaction mixture was heated at 70 °C for 2 h. The progress of the reaction was monitored by TLC and LCMS analysis. After completion of the reaction, the reaction mixture was concentrated under reduced pressure. The crude material was poured into water (900 mL). The precipitate formed was isolated by filtration and dried under reduced pressure to provide ethyl 2-(4-bromo-1H-pyrazol-3-yl) thiazole-4-carboxylate (30.0 g, 93.0 %) as a light yellow solid.

**LCMS [ESI, M+1, M+2]:** 301.7, 303.6 (RT: 1.422 min, Purity: 98.11 %),

### Step-3: Synthesis of 2-(4-bromo-1H-pyrazol-3-yl) thiazole-4-carboxylic acid

To a stirred solution of ethyl 2-(4-bromo-1H-pyrazol-3-yl)thiazole-4-carboxylate (30.0 g, 99.28mmol) in EtOH (300 mL, 10 V) at room temperature, 2N NaOH solution (150 mL, 10V) was added. The resulting reaction mixture was stirred at 70 °C for 1 h. The progress of the reaction was monitored by TLC and LCMS analysis. After completion of the reaction, the reaction mixture was cooled to room temperature and concentrated under reduced pressure. The crude material was dissolved in water (100mL) and add EtOAc (60mL) added. The aqueous layer was isolated and acidified with citric acid solution (pH=~4). The solid precipitate that was formed was isolated by filtration and dried under reduced pressure to provide 2-(4-bromo-1H-pyrazol-3-yl) thiazole-4-carboxylic acid (20.0g, 73.49% yield) as an off-white solid.

**LCMS [ESI, M+1, M+2]:** 273.6, 275.6 (RT: 1.165min, Purity: 100%).

**¹H NMR (400 MHz, d*₆*-DMSO):** δ 13.86 (s, 1H), 8.44 (s, 1H), 8.13 (s, 1H).

### Step-4: Synthesis of tert-butyl (S)-4-(2-(4-bromo-1H-pyrazol-3-yl)thiazole-4-carbonyl)-2-methylpiperazine-1-carboxylate

To a stirred solution of 2-(4-bromo-1H-pyrazol-3-yl) thiazole-4-carboxylic acid (5.0 g, 18.2 mmol) in DMF (50.0mL) at room temperature, HATU (10.40 g, 27.3 mmol) was added. The reaction mixture was stirred for 5 min at room temperature, then tert-butyl (S)-2-methylpiperazine-1-carboxylate (4.0 g, 20.0 mmol) and DIPEA (9.3 mL, 54.7 mmol) were added at room temperature and the reaction mixture was stirred at room temperature for 16 h. The progress of the reaction was monitored by TLC and LCMS analysis. After completion of the reaction, the reaction mixture was poured into ice cold water (350 mL). The precipitate that formed was isolated by filtration and dried under reduced pressure to provide tert-butyl (S)-4-(2-(4-bromo-1H-pyrazol-3-yl) thiazole-4-carbonyl)-2-methylpiperazine-1-carboxylate (5.0g, 60.06% yield) as a cream solid that was used without further purification.

**LCMS [ESI, M-Boc, M-Boc+1]:** 355.8, 357.8 (RT: 1.582min, Purity: 97.88%).

### Step-5: Synthesis of tert-butyl (S)-4-(2-(4-bromo-1-(4-cyclopropylphenyl)-1H-pyrazol-3-yl) thiazole-4-carbonyl)-2-methylpiperazine-1-carboxylate

To a stirred solution of tert-butyl (S)-4-(2-(4-bromo-1H-pyrazol-3-yl)thiazole-4-carbonyl)-2-methylpiperazine-1-carboxylate (1.0 g, 2.19 mmol) in DMF (10mL) at room temperature, (4-cyclopropylphenyl) boronic acid (0.49 g, 3.07 mmol) was added. Pyridine (0.34g, 4.39mmol) and Cu(OAc)₂(0.79 g, 4.39 mmol) were added to the reaction mixture at room temperature. The reaction mixture was stirred at 60 °C for 16 h under an air atmosphere. The progress of the reaction was monitored by TLC analysis. After completion of the reaction, the reaction mixture was filter through Celite^{®}. The reaction mixture washed with EtOAc (100 mL x 2) and water (150 mL). The combined organic fractions were dried over Na₂SO₄ and concentrated under reduced pressure. The crude material was purified by normal phase column chromatography (45% EtOAc in hexane) to provide tert-butyl(S)-4-(2-(4-bromo-1-(4-cyclopropylphenyl)-1H-pyrazol-3-yl)thiazole-4-carbonyl)-2-methylpiperazine-1-carboxylate (0.5 g, 39.86 % yield) as a cream solid.

**LCMS [ESI, M-100, M-56]:** 473.9, 517.7 (RT: 2.436 min, Purity: 81.45%).

### Step-6: Synthesis of tert-butyl (S)-4-(2-(4-cyano-1-(4-cyclopropylphenyl)-1H-pyrazol-3-yl) thiazole-4-carbonyl)-2-methylpiperazine-1-carboxylate

To a stirred solution of tert-butyl (S)-4-(2-(4-bromo-1-(4-cyclopropylphenyl)-1H-pyrazol-3-yl) thiazole-4-carbonyl)-2-methylpiperazine-1-carboxylate (0.2 g, 0.35 mmol) in DMF (2mL) at room temperature, CuCN (0.09 g, 1.05 mmol) was added. The reaction mixture was stirred at 170 °C for 2 h in microwave. The progress of the reaction was monitored by TLC analysis. After completion of the reaction, the reaction mixture was filtered through Celite^{®}, washed with EtOAc (50 mL), and concentrated under reduced pressure. The crude material was purified by reverse phase column chromatography (60% CH₃CN in water) to provide tert-butyl (S)-4-(2-(4-cyano-1-(4-cyclopropylphenyl)-1H-pyrazol-3-yl) thiazole-4-carbonyl)-2-methylpiperazine-1-carboxylate (0.07g, 38.64% yield) as a light green solid.

**LCMS [ESI, M-100]:** 419.1 (RT: 1.469 min, Purity: 92.16 %).

Note that 2 batches of this reaction were carried out on the same scale as described here in parallel and the product yield quoted here is based on the combination of these 2 batches.

### Step-7: Synthesis of (S)-1-(4-cyclopropylphenyl)-3-(4-(3-methylpiperazine-1-carbonyl) thiazol-2-yl)-1H-pyrazole-4-carbonitrile hydrochloride:

To a stirred solution of tert-butyl (S)-4-(2-(4-cyano-1-(4-cyclopropylphenyl)-1H-pyrazol-3-yl) thiazole-4-carbonyl)-2-methylpiperazine-1-carboxylate (0.15 g, 0.28 mmol) in CH₂Cl₂ (1.5mL) at 0 °C, 4M HCl in Dioxane (0.75 mL, 5V) was added. The reaction mixture was stirred at room temperature for 2h. The progress of the reaction was monitored by TLC analysis. After completion of the reaction, the reaction mixture was concentrated under reduced pressure and which was purified by trituration using diethyl ether (2 x 10 mL) to provide (S)-1-(4-cyclopropylphenyl)-3-(4-(3-methyl piperazine-1-carbonyl) thiazol-2-yl)-1H-pyrazole-4-carbonitrile hydrochloride (compound C): 0.07g, 57.83% yield) as an off-white solid.
**LCMS [ESI, M+1]**: 419.1, (RT: 1.488 min, Purity: 99.32 %),
**HPLC Purity:** RT: 6.896 min, Purity: 95.57 %,
**Chiral HPLC:** RT: 6.71 min, Purity: 95.36 %,
**¹H NMR (400 MHz, D₂O):** δ 9.06 (s, 1H), 8.42 (s, 1H), 7.75 (d, *J* = 8.8 Hz, 2H), 7.28 (d, *J* = 8.8 Hz, 2H), 5.45 - 5.28 (m, 1H), 4.75 - 4.68 (m, 1H), 3.70 - 3.43 (m, 4H), 3.14 - 3.08 (m, 1H), 2.05 - 1.98 (m, 1H), 1.42 (d, *J* = 6.8 Hz, 3H), 1.09 - 1.04 (m, 2H), 0.79 - 0.75 (m, 2H).

### Synthesis of 3-(4-{[(S)-3-methyl-1-piperazinyl]carbonyl}-1,3-oxazol-2-yl)-1-(p-cyclopropylphenyl)-4-pyrazolecarbonitrile hydrochloride (compound D)

### Overall synthetic scheme

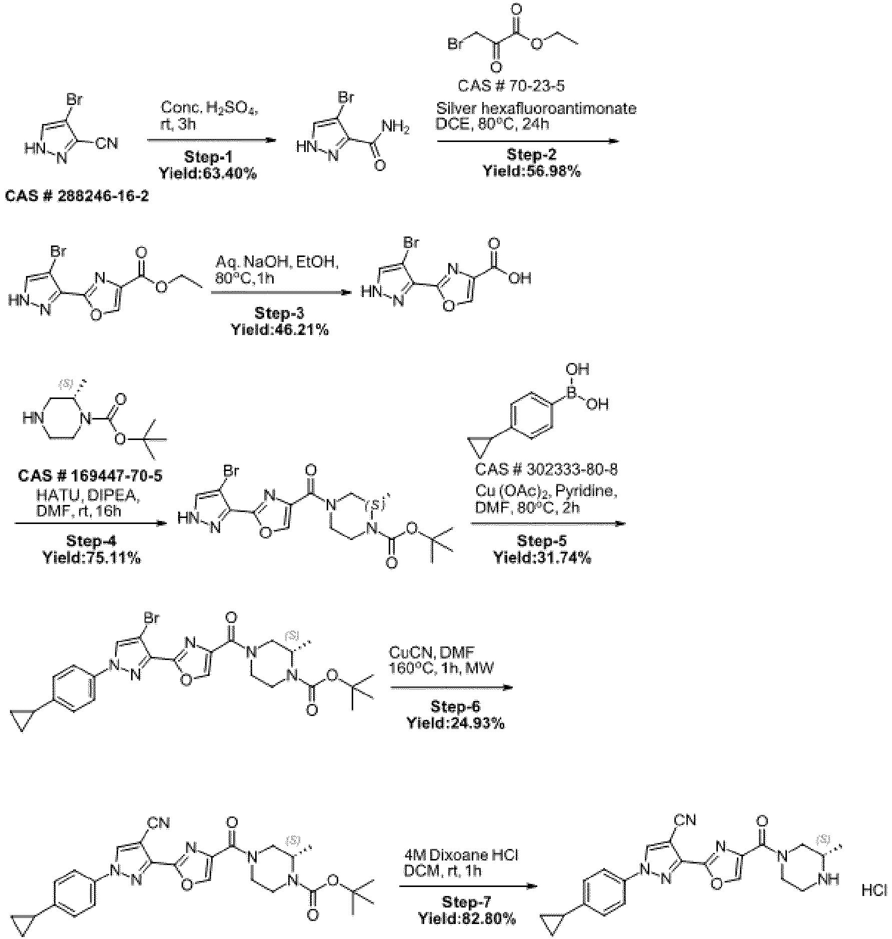

### Synthetic procedures

### Step-1: Synthesis of 4-bromo-1H-pyrazole-3-carboxamide

Conc. H₂SO₄ (60 mL, 6.0 V) was added to 4-bromo-1H-pyrazole-3-carbonitrile (10.0 g, 58.1 mmol) and the reaction mixture was stirred at room temperature for 3 h. The progress of the reaction was monitored by TLC analysis. After completion of the reaction, the reaction mixture was poured into crushed ice. A solid precipitate was formed. Then 25% aq.NH₃ (180 mL) was added until a pH of approx. 8 was obtained (180 mL). The mixture was filtered and the filtrate concentrated under reduced pressure to provide 4-bromo-1H-pyrazole-3-carboxamide (7.0g, 63.40% yield) as a white solid.

**¹H NMR (400 MHz, *d*₆-DMSO): δ** 13.55 (s, 1H), 8.05 (s, 1H), 7.52 (s, 1H), 7.27 (s, 1H).

### Step-2: Synthesis of ethyl 2-(4-bromo-1H-pyrazol-3-yl)oxazole-4-carboxylate

To a stirred solution of 4-bromo-1H-pyrazole-3-carboxamide (7.0 g, 36.8 mmol) in DCE (175 mL, 25 V) at room temperature, ethyl 3-bromo-2-oxopropanoate (28.70 g, 147.0 mmol) and silver hexafluoroantimonate (12.65 g, 36.8 mmol) were added. The reaction mixture was stirred at 80 °C for 24 h. The progress of the reaction was monitored by TLC analysis. After completion of the reaction, the reaction mixture was filter through Celite^{®} and the filtrate was diluted with water (750 mL) and extracted with CH₂Cl₂ (3 x 200 mL). The combined organic fractions were dried over Na₂SO₄ and concentrated under reduced pressure. The crude material was purified by reverse phase column chromatography (product eluted at 60 % CH₃CN in water) to provide ethyl 2-(4-bromo-1H-pyrazol-3-yl)oxazole-4-carboxylate (6.0 g, 56.98 % yield) as a yellow solid.

**LCMS [ESI, M, M+2]:** 285.7, 287.6 (RT: 1.379 min, Purity: 46.68 %).

### Step-3: Synthesis of 2-(4-bromo-1H-pyrazol-3-yl)oxazole-4-carboxylic acid

To a stirred solution of ethyl 2-(4-bromo-1H-pyrazol-3-yl)oxazole-4-carboxylate (6.0 g, 20.9 mmol) in EtOH (60.0 mL, 10 V) at room temperature, 2N NaOH solution (60.0 mL, 10 V) was added. The reaction mixture was stirred at 80 °C for 1 h. The progress of the reaction was monitored by TLC analysis. After completion of the reaction, the reaction mixture was directly distilled under vacuum. The crude material was acidified using a 1N HCl solution (pH=3-4) and extracted with EtOAc (3 x 150 mL). The combined organic fractions were dried over Na₂SO₄, and concentrated under reduced pressure to provide 2-(4-bromo-1H-pyrazol-3-yl)oxazole-4-carboxylic acid (2.5g, 46.21% yield) as an off-white solid.

**LCMS [ESI, M, M+2]:** 257.8, 259.7 (RT: 1.085min, Purity: 74.26%).

### Step-4: Synthesis of tert-butyl (S)-4-(2-(4-bromo-1H-pyrazol-3-yl)oxazole-4-carbonyl)-2-methyl piperazine-1-carboxylate

To a stirred solution of 2-(4-bromo-1H-pyrazol-3-yl)oxazole-4-carboxylic acid (2.5 g, 9.68 mmol) in DMF (25.0 mL, 10 V) at room temperature under a N₂ atmosphere, HATU (5.52 g, 14.5 mmol) was added. After 30min, tert-butyl (S)-2-methylpiperazine-1-carboxylate (2.32 g, 11.6 mmol) and DIPEA (4.99 mL, 29.0 mmol) were added at room temperature and the reaction mixture was stirred at room temperature for 16 h. The progress of the reaction was monitored by TLC analysis. After completion of the reaction, the reaction mixture was poured into ice-cold water (100 mL) and extracted with EtOAc (3 x 150 mL). The combined organic fractions were dried over Na₂SO₄ and concentrated under reduced pressure. The crude material was purified by normal phase column chromatography (product eluted at 3% MeOH in CH₂Cl₂) to provide tert-butyl (S)-4-(2-(4-bromo-1H-pyrazol-3-yl)oxazole-4-carbonyl)-2-methylpiperazine-1-carboxylate (3.2g, 75.11% yield) as a yellow sticky solid.

**LCMS [ESI, M-56]:** 385.8 (RT: 1.521 min, Purity: 87.73%).

### Step-5: Synthesis of tert-butyl (S)-4-(2-(4-bromo-1-(4-cyclopropylphenyl)-1H-pyrazol-3-yl)oxazole-4-carbonyl)-2-methylpiperazine-1-carboxylate

To a stirred solution of tert-butyl (S)-4-(2-(4-bromo-1H-pyrazol-3-yl)oxazole-4-carbonyl)-2-methylpiperazine-1-carboxylate (2.0 g, 4.54 mmol) in DMF (20.0 mL, 10.0 V) at room temperature, (4-cyclopropylphenyl)boronic acid (1.32 g, 8.18 mmol) in) was added. Then, Cu(OAc)₂ (1.64 g, 9.08 mmol) and pyridine (0.72 mL, 9.08 mmol) were added at room temperature and the reaction mixture was stirred at 80 °C for 2h. The progress of the reaction was monitored by TLC analysis. After completion of the reaction, the reaction mixture was poured into ice-cold water (100mL) and precipitates were formed. The filtrate was isolated by filtration and concentrated under reduced pressure. The crude material was purified by normal phase column chromatography (product eluted at 37 % EtOAc in Hexane) to provide tert-butyl (S)-4-(2-(4-bromo-1-(4-cyclopropylphenyl)-1H-pyrazol-3-yl)oxazole-4-carbonyl)-2-methylpiperazine-1-carboxylate(0.8 g, 31.74% yield) as a pale yellow solid.

**LCMS [ESI, M-100]:** 455.9,457.8 (RT: 2.306min, Purity: 57.50%).

### Step-6: Synthesis of tert-butyl (S)-4-(2-(4-cyano-1-(4-cyclopropylphenyl)-1H-pyrazol-3-yl)oxazole-4-carbonyl)-2-methylpiperazine-1-carboxylate

To a stirred solution of tert-butyl (S)-4-(2-(4-bromo-1-(4-cyclopropylphenyl)-1H-pyrazol-3-yl)oxazole-4-carbonyl)-2-methylpiperazine-1-carboxylate (0.4 g, 0.71 mmol) in DMF (8.0 mL, 20V) at room temperature, CuCN (0.193 g, 2.15 mmol) was added. The reaction mixture was heated at 160°C for 1h in microwave. The progress of the reaction was monitored by TLC analysis. After completion of the reaction, the reaction mixture was diluted with water (50 mL) and extracted with EtOAc (3 x 50 mL). The combined organic fractions were dried over Na₂SO₄ and concentrated under reduced pressure. The crude material was purified by normal phase column chromatography (product eluted at 43% EtOAc in hexane) to provide tert-butyl (S)-4-(2-(4-cyano-1-(4-cyclopropylphenyl)-1H-pyrazol-3-yl)oxazole-4-carbonyl)-2-methylpiperazine-1-carboxylate (0.18g, 24.93% yield) as a yellow oil. Note that 2 batches of this reaction were carried out on the same scale as described here and the product yield quoted here is based on the combination of these 2 batches.

**LCMS [ESI, M-100]:** 403.0 (RT: 2.146 min, Purity: 76.81%)

### Step-7: Synthesis of (S)-1-(4-cyclopropylphenyl)-3-(4-(3-methylpiperazine-1-carbonyl)oxazol-2-yl)-1H-pyrazole-4-carbonitrile hydrochloride (compound D)

To a stirred solution tert-butyl (S)-4-(2-(4-cyano-1-(4-cyclopropylphenyl)-1H-pyrazol-3-yl)oxazole-4-carbonyl)-2-methylpiperazine-1-carboxylate (0.18 g, 0.35 mmol) in DCM (1.8 mL) at 0°C, 4M HCl in Dioxane (1.8 mL) was added. The reaction mixture was stirred at room temperature for 1h. The progress of the reaction was monitored by TLC analysis. After completion of the reaction, the reaction mixture was concentrated under reduced pressure and triturated using diethyl ether (15 mL x 2) to provide crude material which was purified by preparative HPLC (Mobile phase: A = 0.05% HCl in water: B = MeCN:MeOH) to provide (S)-1-(4-cyclopropylphenyl)-3-(4-(3-methylpiperazine-1-carbonyl)oxazol-2-yl)-1H-pyrazole-4-carbonitrile hydrochloride compound D: 0.13 g, 82.80 % yield) as a white solid.
**LCMS [ESI, M+1]**: 403.1 (RT: 1.372 min, Purity: 100%),
**HPLC Purity:** RT: 6.468 min, Purity: 98.93%,
**Chiral HPLC Purity:** RT: 2.703 min, Purity: 99.29%,
**¹H NMR (400 MHz, CD₃OD):** δ 9.10 (s, 1H), 8.67 (s, 1H), 7.78 (d, *J* = 12.0 Hz, 2H), 7.29 (d, *J* = 8.0 Hz, 2H), 5.41 (bs, 1H), 4.63 - 4.70 (m, 1H), 3.54 - 3.50 (m, 3H), 3.32 - 3.27 (m, 1H), 3.07 (bs, 1H), 2.04 - 2.00 (m, 1H), 1.44 - 1.30 (m, 3H), 1.10 - 1.05 (m, 2H), 0.80 - 0.77 (m, 2H).

### Synthesis of 2-[4-(4-{[(S)-3-methyl-1-piperazinyl]carbonyl}-1,3-oxazol-2-yl)-1-pyrazolyl]-5-cyclopropylbenzonitrile hydrochloride (compound E)

### Overall synthetic scheme

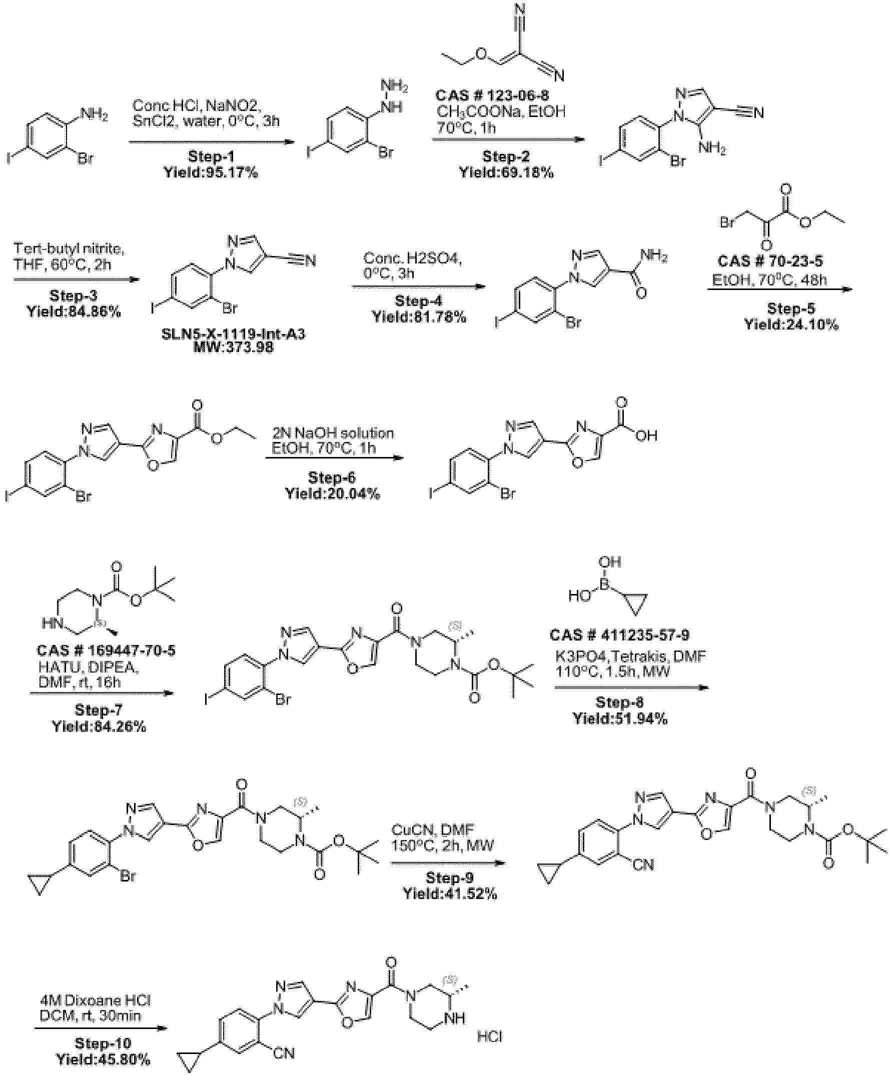

### Synthetic procedures

### Step-1: Synthesis of (2-bromo-4-iodophenyl)hydrazine

To a stirred solution of 2-bromo-4-iodoaniline (5.0 g, 16.7 mmol) in Conc. HCl (30mL) at 0 °C, a solution of sodium nitrite (1.39 g, 20.1 mmol) in water (6 mL) was added. After 1h, Tin(II) chloride (8.23 g, 43.4 mmol; solution in 17.5 mL Conc. HCl) was added at 0°C. The reaction mixture was stirred at 0 °C for 2h. The progress of the reaction was monitored by TLC analysis. After completion of the reaction, the reaction mixture was diluted with ice-cold water (100 mL). A solid precipitate was formed which was isolated by filtration and dried under reduced pressure to provide (2-bromo-4-iodophenyl)hydrazine (5.0 g, 95.17 %) as an off-white solid.
**LCMS [ESI, M+23 (Na Adduct)]:** 336.6 (RT: 1.455min, Purity: 87.73%),
**¹H NMR (400 MHz, *d*₆-DMSO): δ** 10.43 (bs, 3H), 8.09 (bs, 1H), 8.06 (s, 1H), 7.88 (d, *J* = 4.0 Hz, 1H), 7.70 (dd, *J* = 8.0, 4.0 Hz, 1H), 6.90 (d, *J* = 12.0 Hz, 1H).

### Step-2: Synthesis of 5-amino-1-(2-bromo-4-iodophenyl)-1H-pyrazole-4-carbonitrile

To a stirred solution of (2-bromo-4-iodophenyl)hydrazine (5.0 g, 15.9 mmol) in EtOH (50 mL, 10V) at 0°C, sodium acetate (2.62 g, 31.9 mmol) was added. After 15 min, 2-(ethoxymethylene)malononitrile (1.95 g, 15.9 mmol) was added at 0°C and the reaction mixture was stirred at 70°C for 1h. The progress of the reaction was monitored by TLC analysis. After completion of the reaction, the reaction mixture was concentrated under reduced pressure and poured into ice-cold water (500mL) and a solid precipitate was formed. The solid material was filtered and concentrated under reduced pressure to provide 5-amino-1-(2-bromo-4-iodophenyl)-1H-pyrazole-4-carbonitrile (4.3 g, 69.18 %) as a light brown solid.

**LCMS [ESI, M, M+2]:** 389, 390.9 (RT: 1.898min, Purity: 90.47%),

### Step-3: Synthesis of 1-(2-bromo-4-iodophenyl)-1H-pyrazole-4-carbonitrile

To a stirred solution of 5-amino-1-(2-bromo-4-iodophenyl)-1H-pyrazole-4-carbonitrile (4.3 g, 11.0 mmol) in THF (45 mL, 10V) at 0 °C, tert-butyl nitrite (2.27 g, 22.1 mmol) was added. The reaction mixture was stirred at 60 °C for 2h. The progress of the reaction was monitored by TLC analysis. After completion of the reaction, the reaction mixture was poured into ice-cold water (200 mL) and a solid precipitate was formed. The solid material was filtered and concentrated under reduced pressure to provide 1-(2-bromo-4-iodophenyl)-1H-pyrazole-4-carbonitrile (3.5 g, 84.86 % yield) as a yellow solid.

**¹H NMR (400 MHz, *d*₆-DMSO): δ** 9.01 (s, 1H), 8.38 (s, 1H), 8.28 (d, *J* = 1.6 Hz, 1H), 7.96 (dd, *J* =8.0, 4.0 Hz, 1H), 7.41 (d, *J* = 8.0 Hz, 1H).

### Step-4: Synthesis of 1-(2-bromo-4-iodophenyl)-1H-pyrazole-4-carboxamide

Conc. H₂SO₄ (21 mL) was added to 1-(2-bromo-4-iodophenyl)-1H-pyrazole-4-carbonitrile (3.5 g, 9.30 mmol) at 0 °C and the solution stirred at room temperature for 3h. The progress of the reaction was monitored by TLC analysis. After completion of the reaction, the reaction mixture was poured into crushed ice and basified using aq.NH₃ (pH=-8). A solid precipitate was formed, which was filtered and dried under reduced pressure to provide 1-(2-bromo-4-iodophenyl)-1H-pyrazole-4-carboxamide (3.0 g, 81.78% yield) as a brown solid.
**LCMS [ESI, M, M+2]:** 392, 394.0 (RT: 1.768min, Purity: 97.40%),
**¹H NMR (400 MHz, *d*₆-DMSO): δ** 8.49 (s, 1H), 8.24 (s, 1H), 8.11 (s, 1H), 7.91 (d, *J* = 8.0 Hz, 1H), 7.73 (s, 1H), 7.35 (d, *J* = 12.0 Hz, 1H), 7.20 (s, 1H).

### Step-5: Synthesis of ethyl 2-(1-(2-bromo-4-iodophenyl)-1H-pyrazol-4-yl)oxazole-4-carboxylate

To a stirred solution of 1-(2-bromo-4-iodophenyl)-1H-pyrazole-4-carboxamide (3.0 g, 7.6 mmol) in EtOH (30 mL, 10V) at room temperature, ethyl 3-bromo-2-oxopropanoate (2.98 g, 15.3 mmol) was added. The reaction mixture was stirred at 70°C for 48h. The progress of the reaction was monitored by TLC analysis. After completion of the reaction, the reaction mixture was concentrated under reduced pressure and the crude material purified by normal phase column chromatography (27% EtOAc in hexane) to provide ethyl 2-(1-(2-bromo-4-iodophenyl)-1H-pyrazol-4-yl)oxazole-4-carboxylate (0.9 g, 24.10% yield) as a brown oil.

**LCMS [ESI, M, M+2]:** 487.6, 489.6 (RT: 1.953min, Purity: 70.19%),

### Step-6: Synthesis of 2-(1-(2-bromo-4-iodophenyl)-1H-pyrazol-4-yl)oxazole-4-carboxylic acid

To a stirred solution of ethyl 2-(1-(2-bromo-4-iodophenyl)-1H-pyrazol-4-yl)oxazole-4-carboxylate (0.9 g, 1.84 mmol) in EtOH (9.0 mL, 10V) at room temperature, 2N aq. NaOH (9 mL, 10V) was added. The reaction mixture was stirred at 70 °C for 1h. The progress of the reaction was monitored by TLC analysis. After completion of the reaction, the reaction mixture was distilled under reduced pressure. The crude material was acidified using 1N HCl solution (pH=~2-3) and a solid precipitate was formed. The solid material was filtered and dried under reduced pressure to provide 2-(1-(2-bromo-4-iodophenyl)-1H-pyrazol-4-yl)oxazole-4-carboxylic acid (0.17g, 20.04%) as an off-white solid.

**LCMS [ESI, M, M+2]:** 459.7, 461.6 (RT: 1.593min, Purity: 84.40%),

### Step-7: Synthesis of tert-butyl (S)-4-(2-(1-(2-bromo-4-iodophenyl)-1H-pyrazol-4-yl)oxazole-4-carbonyl)-2-methylpiperazine-1-carboxylate

To a stirred solution of 2-(1-(2-bromo-4-iodophenyl)-1H-pyrazol-4-yl)oxazole-4-carboxylic acid (0.17 g, 0.37 mmol) in DMF (2.0 mL, 10V) at 0°C, HATU (0.21 g, 0.55 mmol) and DIPEA (0.19 mL, 1.10 mmol) were added. After 30 min, tert-butyl (S)-2-methylpiperazine-1-carboxylate (0.09 g, 0.44 mmol) was added at 0°C. The reaction mixture was stirred at room temperature for 16 h. The progress of the reaction was monitored by TLC analysis. After completion of the reaction, the reaction mixture was poured into ice-cold water (50mL) and a solid precipitate was formed. The solid material was filtered and concentrated under reduced pressure to provide tert-butyl (S)-4-(2-(1-(2-bromo-4-iodophenyl)-1H-pyrazol-4-yl)oxazole-4-carbonyl)-2-methylpiperazine-1-carboxylate (0.2 g, 84.26 % yield) as a light brown solid.

**LCMS [ESI, M, M+2]:** 642.2, 644.2 (RT: 2.521 min, Purity: 82.45%),

### Step-8: Synthesis of tert-butyl (S)-4-(2-(1-(2-bromo-4-cyclopropylphenyl)-1H-pyrazol-4-yl)oxazole-4-carbonyl)-2-methylpiperazine-1-carboxylate

To a stirred solution of tert-butyl (S)-4-(2-(1-(2-bromo-4-iodophenyl)-1H-pyrazol-4-yl)oxazole-4-carbonyl)-2-methylpiperazine-1-carboxylate (0.2 g, 0.31 mmol) in DMF (2.0 mL, 10V) at room temperature, cyclopropyl boronic acid (0.04 g, 0.46 mmol) and K₃PO₄ (0.19 g, 0.93 mmol) were added. The reaction mixture was purged with nitrogen gas for 15min, then tetrakis (0.035 g, 0.31 mmol) was added at room temperature and the resulting reaction mixture was heated at 110 °C for 1.5 h by microwave irradiation. The progress of the reaction was monitored by TLC analysis. After completion of the reaction, the reaction mixture was poured into ice-cold water (30mL) and extracted with EtOAc (3 × 20 mL). The combined organic fractions were dried over Na₂SO₄ and concentrated under reduced pressure. The resulting crude material was purified by normal phase column chromatography (40% EtOAc in hexane) to provide tert-butyl (S)-4-(2-(1-(2-bromo-4-cyclopropylphenyl)-1H-pyrazol-4-yl)oxazole-4-carbonyl)-2-methylpiperazine-1-carboxylate (0.09g, 51.94%) as a yellow sticky solid.

**LCMS [ESI, M-56]:** 501.8 (RT:2.101 min, Purity: 81.44%)

### Step-9: Synthesis of tert-butyl (S)-4-(2-(1-(2-cyano-4-cyclopropylphenyl)-1H-pyrazol-4-yl)oxazole-4-carbonyl)-2-methylpiperazine-1-carboxylate

To a stirred solution of tert-butyl (S)-4-(2-(1-(2-bromo-4-cyclopropylphenyl)-1H-pyrazol-4-yl)oxazole-4-carbonyl)-2-methylpiperazine-1-carboxylate (0.08 g, 0.14 mmol) in DMF (1.0 mL, 10V) at room temperature, CuCN (0.032 g, 0.35 mmol) was added. The reaction mixture was heated at 150 °C for 2 h by microwave irradiation. The progress of the reaction was monitored by TLC analysis. After completion of the reaction, the reaction mixture was poured in ice-cold water (30 mL). A solid precipitate was formed. The solid was filtered and concentrated under reduced pressure. The crude material was purified by reverse phase column chromatography (55 % MeCN in water) to provide tert-butyl (S)-4-(2-(1-(2-cyano-4-cyclopropylphenyl)-1H-pyrazol-4-yl)oxazole-4-carbonyl)-2-methylpiperazine-1-carboxylate (0.03 g, 41.52 %) as an off-white solid.

**LCMS [ESI, M-56]:** 447.0 (RT:1.919 min, Purity: 84.75%)

### Step-10: Synthesis of (S)-5-cyclopropyl-2-(4-(4-(3-methylpiperazine-1-carbonyl)oxazol-2-yl)-1H-pyrazol-1-yl)benzonitrile hydrochloride (compound E)

To a stirred solution tert-butyl (S)-4-(2-(1-(2-cyano-4-cyclopropylphenyl)-1H-pyrazol-4-yl)oxazole-4-carbonyl)-2-methylpiperazine-1-carboxylate (0.03 g, 0.059 mmol) in CH₂Cl₂ (1.0 mL, 10V) at 0°C, 4M HCl in Dioxane (0.03 mL) was added. The reaction mixture was stirred at room temperature for 30min. The progress of the reaction was monitored by TLC analysis. After completion of the reaction, the reaction mixture was concentrated under reduced pressure and the resulting crude material purified by trituration using diethyl ether: CH₂Cl₂ to provide (S)-5-cyclopropyl-2-(4-(4-(3-methylpiperazine-1-carbonyl)oxazol-2-yl)-1H-pyrazol-1-yl)benzonitrile hydrochloride (compound E):0.012 g, 45.80 % yield) as a yellow solid.
**LCMS [ESI, M+1]:** 403.1 (RT: 1.238 min, Purity:96.87%),
**HPLC Purity:** RT: 3.811 min, Purity: 95.73%,
**Chiral HPLC Purity:** RT: 2.71 min, Purity: 97.89%,
**¹H NMR (400 MHz, CD₃OD): δ** 8.85 (s, 1H), 8.48 (s, 1H), 8.33 (s, 1H), 7.71 (d, *J* = 4.0 Hz, 1H), 7.67 (d, *J* = 4.0 Hz, 1H), 7.57 (dd, *J* = 4.0, 2.0 Hz, 1H), 5.17 (bs, 2H), 4.80-4.49 (m, 1H), 3.49-3.33 (m, 2H), 3.32-3.30 (m, 1H), 2.13-2.07 (m, 1H), 1.42-1.30 (m, 3H), 1.16-1.12 (m, 2H), 0.75-0.68 (m, 2H).

### Synthesis of [(S)-3-methyl-1-piperazinyl]{2-[1-(p-cyclopropylphenyl)-4-pyrazolyl]-4-imidazolyl}methanone hydrochloride (compound F)

### Overall synthetic scheme

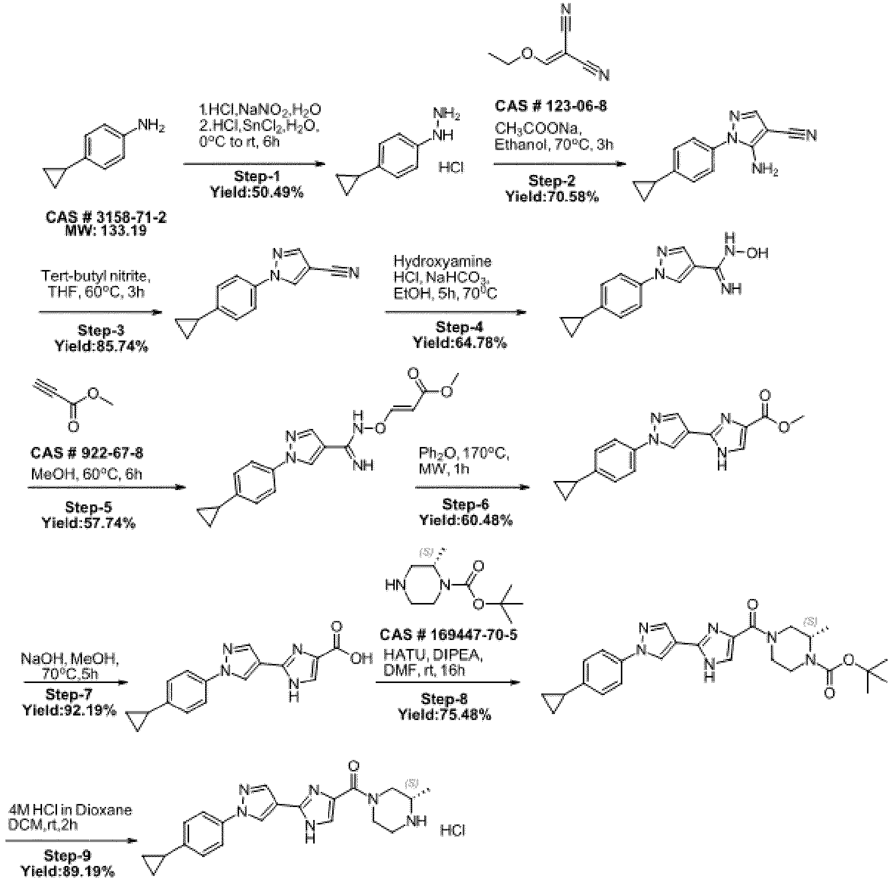

### Synthetic procedures

### Step-1: Synthesis of (4-cyclopropylphenyl) hydrazine hydrochloride

To a stirred solution of 4-cyclopropylaniline (5.0 g, 37.59 mmol) in HCl (30 mL, 6V) at 0°C, NaNO₂ (2.59 g, 37.59 mmol) in 23 mL H₂O was added and the reaction mixture stirred for 1 h at 0°C. A solution of SnCl₂ (17.76 g, 93.97 mmol) in HCl (75 mL,15V) was added drop-wise to the reaction mixture at 0 °C over 45 min. The reaction mixture was stirred at room temperature for 5 h. The progress of the reaction was monitored by TLC analysis. After completion of the reaction, the reaction mixture was filtered through a Büchner funnel. The solid material was washed with water (250 mL) and diethyl ether (30 mL). The solid was dried under reduced pressure to provide (4-cyclopropylphenyl) hydrazine hydrochloride (3.5 g, 50.49 %) as a brown solid.

**¹H NMR (400 MHz, *d*₆-DMSO):** 610.10 (s, 3H), 8.07 (bs, 1H), 7.01 (d, J = 8.6 Hz, 2H), 6.89 (d, J = 8.6 Hz, 2H), 1.85 (d, J = 8.4 Hz, 1H), 0.93 - 0.84 (m, 2H), 0.63 - 0.54 (m, 2H).

### Step-2: Synthesis of 5-amino-1-(4-cyclopropylphenyl)-1H-pyrazole-4-carbonitrile

To a stirred solution of (4-cyclopropylphenyl) hydrazine hydrochloride (3.5 g, 19.02 mmol) in ethanol (35 mL, 10V) at room temperature, sodium acetate (3.1 g, 38.04 mmol) and 2-(ethoxymethylene)malononitrile (2.3 g, 19.02 mmol) were added. The reaction mixture was stirred at 70 °C for 3 h. The progress of the reaction was monitored by TLC analysis. After completion of reaction, the reaction mixture was concentrated under reduced pressure. The crude material was diluted with ice cold water (200 mL) to form a solid precipitate. The precipitate was isolated by filtration, washed with n-pentane (20 mL) and diethyl ether (10 mL), then dried under reduced pressure to provide 5-amino-1-(4-cyclopropylphenyl)-1H-pyrazole-4-carbonitrile (3.0 g, 70.58 %) as a light red solid.

**LCMS [ESI, M+1]:** 225.2 (RT: 1.959 min, Purity: 100%)

### Step-3: Synthesis of 1-(4-cyclopropylphenyl)-1H-pyrazole-4-carbonitrile

To a stirred solution of 5-amino-1-(4-cyclopropylphenyl)-1H-pyrazole-4-carbonitrile (3.0 g, 13.39 mmol) in THF (30 mL, 10V) at room temperature, tert-butyl nitrite (2.7 g, 26.78 mmol) was added dropwise. The reaction mixture was stirred at 60 °C for 3 h. The progress of the reaction was monitored by TLC analysis. After completion of the reaction, the reaction mixture was concentrated under reduced pressure. The residual material was poured into ice cold water (150 mL) and the resulting solid material isolated by filtration using a Büchner funnel and washed with n-pentane (30 mL). The solid material was dried under reduced pressure to provide 1-(4-cyclopropylphenyl)-1H-pyrazole-4-carbonitrile (2.4g, 85.74% yield) as a brown solid.

**¹H NMR (400 MHz, *d*₆-DMSO):** δ 9.28 (s, 1H), 8.33 (s, 1H), 7.72 (d, J = 8.6 Hz, 2H), 7.25 (d, J = 8.6 Hz, 2H), 2.04 - 1.95 (m, 1H), 1.03 - 0.96 (m, 2H), 0.76 - 0.69 (m, 2H).

### Step-4: Synthesis of 1-(4-cyclopropylphenyl)-N-hydroxy-1H-pyrazole-4-carboximidamide

To a stirred solution of Hydroxylamine HCl (1.9 g, 28.70 mmol) in water (12 mL, 5V) at 0°C, NaHCO₃ (2.4 g, 28.70 mmol) was added. The reaction was stirred for 10 min at 0 °C. A solution of 1-(4-cyclopropylphenyl)-1H-pyrazole-4-carbonitrile (2.4 g, 11.48 mmol) in EtOH (12 mL, 5V) was added. The reaction mixture was stirred at 70 °C for 5 h. The progress of the reaction was monitored by TLC analysis. The reaction mixture was concentrated under reduced pressure. Ice cold water (100 mL) was added to reaction mixture. The solid precipitate was isolated by filtration with a Büchner funnel and dried under reduced pressure. The solid was washed with n-pentane (30 mL) and diethyl ether (20mL) to provide 1-(4-cyclopropylphenyl)-N-hydroxy-1H-pyrazole-4-carboximidamide (1.8 g, 64.78 % yield) as a brown solid.

**LCMS [ESI, M+1]:** 243.1 (RT:1.644 min, Purity:100 %)

### Step-5: Synthesis of methyl (E)-3-((1-(4-cyclopropylphenyl)-1H-pyrazole-4-carboximidamido) oxy) acrylate

To a stirred solution 1-(4-cyclopropylphenyl)-N-hydroxy-1H-pyrazole-4-carboximidamide (1.8 g, 7.43 mmol) in MeOH (18 mL, 10V) at room temperature, methyl propiolate (1.8 g, 22.31 mmol) was added. The reaction mixture was stirred at 60 °C for 6 h. The progress of the reaction was monitored by TLC analysis. The reaction mixture was concentrated under reduced pressure and the crude material washed with diethyl ether (20 mL) and dried under reduced pressure to provide methyl (E)-3-((1-(4-cyclopropylphenyl)-1H-pyrazole-4-carboximidamido) oxy) acrylate (1.4 g, 57.74 % yield) as a light yellow solid.

**LCMS [ESI, M+1]:** 326.9 (RT: 1.775 min, Purity:100%)

### Step-6: Synthesis of methyl 2-(1-(4-cyclopropylphenyl)-1H-pyrazol-4-yl)-1H-imidazole-4-carboxylate

A stirred solution of methyl (E)-3-((1-(4-cyclopropylphenyl)-1H-pyrazole-4-carboximidamido) oxy) acrylate (1.4 g, 4.29 mmol) in Diphenyl ether (7 mL, 5V) was heated at 170 °C for 1 h in a microwave. The progress of the reaction was monitored by TLC analysis. The starting material was not totally consumed. Silica gel was added and the reaction mixture concentrated under reduced pressure. The slurry was purified by normal phase column chromatography (100% EtOAc in Hexane) to provide methyl 2-(1-(4-cyclopropylphenyl)-1H-pyrazol-4-yl)-1H-imidazole-4-carboxylate (0.8 g, 60.48 % yield) as a white solid.

**LCMS [ESI, M+1]:** 308.8 (RT: 1.445, Purity: 98.57%).

### Step-7: Synthesis of 2-(1-(4-cyclopropylphenyl)-1H-pyrazol-4-yl)-1H-imidazole-4-carboxylic acid

To a stirred solution of methyl 2-(1-(4-cyclopropylphenyl)-1H-pyrazol-4-yl)-1H-imidazole-4-carboxylate (0.5 g, 1.62 mmol) in MeOH (5.0mL, 10V) at room temperature, a solution of NaOH (0.25 g, 6.49 mmol) in H₂O (5.0 mL, 10V) was added. The reaction mixture was stirred for 5h at 70 °C. The progress of the reaction was monitored by TLC analysis. The reaction mixture was concentrated under reduced pressure, then diluted with water (5.0mL) and acidified by the addition of a saturated solution of citric acid. The solid precipitate was isolated by filtration using a Büchner funnel and dried under reduced pressure to provide 2-(1-(4-cyclopropylphenyl)-1H-pyrazol-4-yl)-1H-imidazole-4-carboxylic acid (0.44 g, 92.19 % yield) as a white solid.

**LCMS [ESI, M+1]:** 294.8 (RT: 1.223 min, Purity: 94.36%)

### Step-8: Synthesis of tert-butyl (S)-4-(2-(1-(4-cyclopropylphenyl)-1H-pyrazol-4-yl)-1H-imidazole-4-carbonyl)-2-methylpiperazine-1-carboxylate

To a stirred solution of 2-(1-(4-cyclopropylphenyl)-1H-pyrazol-4-yl)-1H-imidazole-4-carboxylic acid (0.18 g, 0.61 mmol) in DMF (1.8mL, 10V) at room temperature, HATU (0.34 g, 0.91 mmol) was added. The reaction was stirred for 30min, then tert-butyl (S)-2-methylpiperazine-1-carboxylate (0.13 g, 0.67 mmol) and DIPEA (0.23 g, 1.83 mmol) were added to reaction mixture at room temperature. The reaction mixture was stirred at room temperature for 16 h. The progress of the reaction was monitored by TLC and LCMS analysis. After completion of the reaction, the reaction mixture was added to ice cold water (30mL). The solid precipitate was isolated by filtration, dried under reduced pressure and purified by normal phase column chromatography (100% EtOAc/Hexane) to provide tert-butyl (S)-4-(2-(1-(4-cyclopropylphenyl)-1H-pyrazol-4-yl)-1H-imidazole-4-carbonyl)-2-methylpiperazine-1-carboxylate (0.22 g, 75.48 % yield) as a white solid. **LCMS [ESI, M-55]:** 477.2 (RT: 1.66 min, Purity: 98.37%).

### Step-9: Synthesis of (S)-(2-(1-(4-cyclopropylphenyl)-1H-pyrazol-4-yl)-1H-imidazol-4-yl)(3-methylpiperazin-1-yl)methanone hydrochloride (compound F)

To a stirred solution of tert-butyl (S)-4-(2-(1-(4-cyclopropylphenyl)-1H-pyrazol-4-yl)-1H-imidazole-4-carbonyl)-2-methylpiperazine-1-carboxylate (0.22 g, 0.46 mmol) in DCM (2.2 mL, 10 V) at 0 °C, 4 M HCl in Dioxane (1.1 mL, 5V) was added. The reaction mixture was stirred at room temperature for 2h. The progress of the reaction was monitored by TLC and LCMS analysis. After completion of the reaction, the reaction mixture was concentrated under reduced pressure. The crude material was diluted with HPLC water (5 mL) and extracted using ethyl acetate (3 × 2 mL). The water layer was lyophilized to provide (S)-(2-(1-(4-cyclopropylphenyl)-1H-pyrazol-4-yl)-1H-imidazol-4-yl)(3-methylpiperazin-1-yl)methanone hydrochloride (compound F); 0.17 g, 89.19 % yield) as an off-white solid.
**LCMS [ESI, M+1]:** 377.06 (RT: 1.159min, Purity: 100%),
**HPLC Purity:** RT: 3.460min, Purity: 100%
**Chiral HPLC Purity:** RT: 3.856 min, Purity: 99.84%,
**¹H NMR (400 MHz, CD₃OD): δ** 8.89 (s, 1H), 8.33 (s, 1H), 8.15 (s, 1H), 7.71 (d, *J* = 8.4 Hz, 2H), 7.28 (d, *J* = 8.8 Hz, 2H), 4.62 - 4.59 (m, 2H), 3.56 - 3.50 (m, 4H), 2.05 - 1.99 (m, 1H), 1.43 (d, *J* = 6.4 Hz, 3H), 1.30 (s, 1H), 1.09 - 1.04 (m, 2H), 0.79 - 0.78 (m, 2H).

### Synthesis of [(S)-3-methyl-1-piperazinyl](2-{1-[p-(tert-butyl)phenyl]-4-pyrazolyl}-1,3-thiazol-4-yl)methanone hydrochloride (compound G)

### Overall synthetic scheme

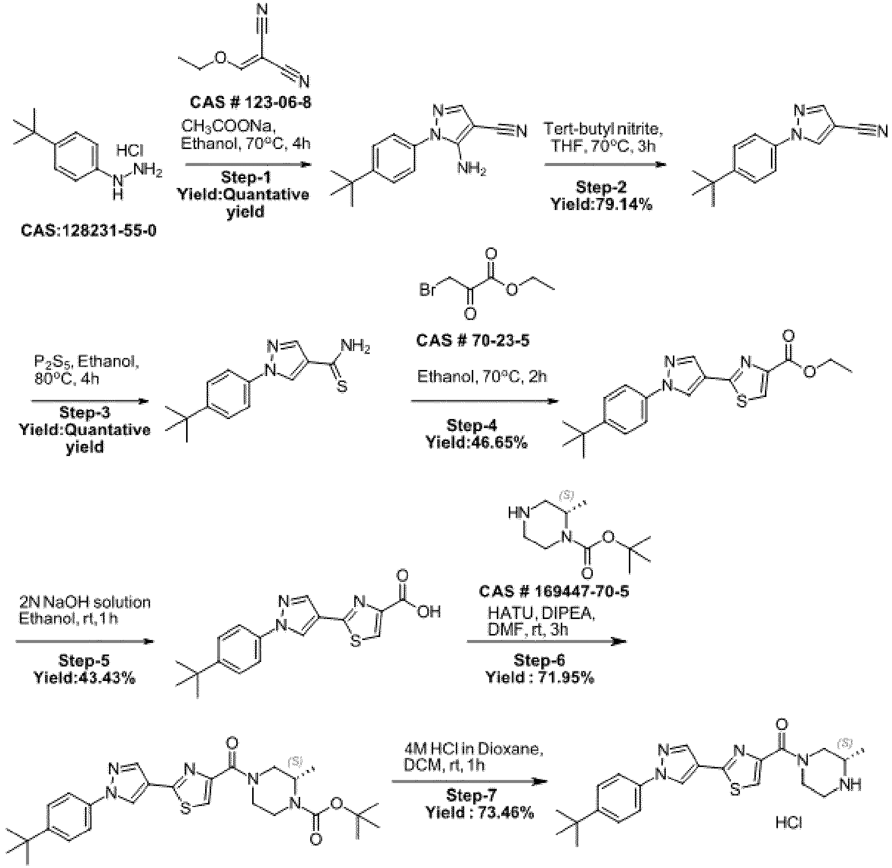

### Synthetic procedures

### Step-1: Synthesis of 5-amino-1-(4-(tert-butyl) phenyl)-1H-pyrazole-4-carbonitrile

To a stirred solution of (4-(tert-butyl) phenyl) hydrazine (2.0 g, 121mmol) in ethanol (15 mL, 15V) at room temperature, sodium acetate (2.0 g, 24.3 mmol) and 2-(ethoxy methylene) malononitrile (1.48 g, 12.1 mmol) were added. The reaction mixture was refluxed at 70 °C for 4h. The progress of the reaction was monitored by TLC and LCMS analysis. After completion of the reaction, the reaction mixture was poured into ice water. A precipitate was formed which was isolated by filtration to provide 5-amino-1-(4-(tert-butyl) phenyl)-1H-pyrazole-4-carbonitrile (3.1 g, Quantitative yield) as a brownish yellow solid.
**LCMS [ESI, M+1]:** 240.9 (RT:1.941 min, Purity:93.56%)
**¹H NMR (400 MHz, *d*₆-DMSO):** δ 7.77 (s, 1H), 7.54 (d, J = 8.6 Hz, 2H), 7.42 (d, J = 8.6 Hz, 2H), 6.65 (s, 2H), 1.32 (s, 9H).

### Step-2: Synthesis of 1-(4-(tert-butyl) phenyl)-1H-pyrazole-4-carbonitrile

To a stirred solution of 5-amino-1-(4-(tert-butyl) phenyl)-1H-pyrazole-4-carbonitrile (3.1 g, 12.9 mmol) in THF (30mL, 10V) at 0°C, tert-butyl nitrile (90 %) (3.72 g, 36.1 mmol) was added. The reaction mixture was refluxed at 70°C for 3 h. The progress of the reaction was monitored by TLC and LCMS analysis. After completion of the reaction, the reaction mixture was poured into ice water. A precipitate was formed which was isolated by filtration to provide 1-(4-(tert-butyl) phenyl)-1H-pyrazole-4-carbonitrile (2.3 g, 79.14 % yield) as a brownish yellow solid.

**LCMS [ESI, M+1]:**225.9 (RT:2.330min, Purity:97.91%)

### Step-3: Synthesis of 1-(4-(tert-butyl) phenyl)-1H-pyrazole-4-carbothioamide

To a stirred solution of 1-(4-(tert-butyl) phenyl)-1H-pyrazole-4-carbonitrile (2.3 g, 10.2 mol) in ethanol (20 mL, 10V) at room temperature, phosphorus pentasulfide (4.53 g, 20.5 mmol) was added. The reaction mixture was refluxed at 80 °C for 4 h. The progress of the reaction was monitored by TLC and LCMS analysis. After completion of the reaction, the reaction mixture was concentrated under reduced pressure. The crude material was washed with sodium bicarbonate solution until neutral pH and extracted with ethyl acetate (3 × 50 mL). The combined organic fractions were dried over anhydrous sodium sulphate and concentrated under reduced pressure to provide 1-(4-(tert-butyl)phenyl)-1H-pyrazole-4-carbothioamide (5.8 g, Quantitative yield) which was used directly in the next step of the synthesis without further purification.

**LCMS [ESI, M+1]:** 259.8 (RT: 2.087min, Purity: 56.55%)

### Step-4: Synthesis of ethyl 2-(1-(4-(tert-butyl) phenyl)-1H-pyrazol-4-yl) thiazole-4-carboxylate

To a stirred solution of 1-(4-(tert-butyl) phenyl)-1H-pyrazole-4-carbothioamide (5.7 g, 12.9 mmol) in ethanol (50 mL, 10V) at room temperature, ethyl 3-bromo-2-oxopropanoate (4.96 g, 43.9 mmol) was added. The reaction mixture was stirred at 70 °C for 2 h. The progress of the reaction was monitored by TLC and LCMS analysis. After completion of the reaction, the reaction mixture was concentrated under reduced pressure.

The crude material was purified by column chromatography (20% EtOAc in hexane) to provide ethyl 2-(1-(4-(tert-butyl) phenyl)-1H-pyrazol-4-yl) thiazole-4-carboxylate (3.5 g, 46.65 % yield) as a brown semi-solid.

**LCMS [ESI, M+1]:**317.94 (RT: 2.551 min, Purity: 72.80%)

### Step-5: Synthesis of 2-(1-(4-(tert-butyl) phenyl)-1H-pyrazol-4-yl) thiazole-4-carboxylic acid

To a stirred solution of ethyl 2-(1-(4-(tert-butyl) phenyl)-1H-pyrazol-4-yl) thiazole-4-carboxylate (3.5 g, 9.84 mmol) in ethanol (30 mL, 10V) at room temperature, 2 N NaOH solution (1.968 mL, 49.2 mmol) was added. The reaction mixture was stirred at room temperature for 1 h. The progress of the reaction was monitored by TLC and LCMS analysis. After completion of the reaction, the reaction mixture was concentrated under reduced pressure. The crude material was poured into water. Concentrated hydrochloric acid was added slowly until acidic pH was achieved. A precipitate was formed which was isolated by filtration using a Büchner funnel. The crude material was purified by trituration using diethyl ether to provide 2-(1-(4-(tert-butyl) phenyl)-1H-pyrazol-4-yl) thiazole-4-carboxylic acid (1.4 g, 43.43% yield) as an off-white solid.
**LCMS [ESI, M+1]:**327.8 (RT: 2.125 min, Purity: 99.25%)
**HPLC:** RT: 8.62 min, Purity:98.42%
**¹H NMR (400 MHz, *d*₆-DMSO):** δ 9.04 (s, 1H), 8.24 (s, 1H), 7.93 - 7.79 (m, 3H), 7.53 (d, J = 8.7 Hz, 2H), 1.89 (s, 1H), 1.29 (s, 9H).

### Step-6: Synthesis of tert-butyl (S)-4-(2-(1-(4-(tert-butyl)phenyl)-1H-pyrazol-4-yl)thiazole-4-carbonyl)-2-methylpiperazine-1-carboxylate

To a stirred solution of 2-(1-(4-(tert-butyl)phenyl)-1H-pyrazol-4-yl)thiazole-4-carboxylic acid (0.25 g, 0.66 mmol) in DMF (2.5 mL) at room temperature, HATU (0.43 g, 1.13 mmol) and DIPEA (0.36 g, 2.13 mmol) were added. After 15 min, tert-butyl (S)-2-methylpiperazine-1-carboxylate (0.16 g, 0.80 mmol) was added and the reaction mixture was stirred at room temperature for 3 h. The progress of the reaction was monitored by TLC and LCMS analysis. After completion of the reaction, the reaction mixture was poured into cold water (15 mL) and extracted with EtOAc (2 × 25 mL). The combined organic fractions were washed with cold water 3-4 times, dried over Na₂SO₄ and concentrated under reduced pressure. The crude material was purified with column chromatography (40% EtOAc in hexane) to provide tert-butyl (S)-4-(2-(1-(4-(tert-butyl)phenyl)-1H-pyrazol-4-yl)thiazole-4-carbonyl)-2-methylpiperazine-1-carboxylate (0.28 g, 71.95 % yield) as an off white sticky solid.

**LCMS [ESI, M+1]:** 510.34 (RT: 2.730 min, Purity: 98.18%).

### Step-7: Synthesis of (S)-(2-(1-(4-(tert-butyl)phenyl)-1H-pyrazol-4-yl)thiazol-4-yl)(3-methylpiperazin-1-yl)methanone hydrochloride (compound G)

To a stirred solution of tert-butyl (S)-4-(2-(1-(4-(tert-butyl)phenyl)-1H-pyrazol-4-yl)thiazole-4-carbonyl)-2-methylpiperazine-1-carboxylate (0.28 g, 0.54 mmol) in CH₂Cl₂ (2.8 mL, 10V) at 0 °C, 4M HCl in Dioxane (1.4 mL, 5V) was added. The reaction mixture was stirred at room temperature for 1 h. The progress of the reaction was monitored by TLC and LCMS analysis. After completion of the reaction, the reaction mixture was concentrated under reduced pressure and the crude material purified by trituration using diethyl ether:CH₂Cl₂ (9:1) to provide (S)-(2-(1-(4-(tert-butyl)phenyl)-1H-pyrazol-4-yl)thiazol-4-yl)(3-methylpiperazin-1-yl)methanone hydrochloride (compound G) 0.18 g, 73.46 % yield) as a white solid.
**LCMS [ESI, M+1]:** 410.11 (RT: 1.575 min, Purity: 98.99%),
**HPLC Purity:** RT: 6.009 min, Purity: 98.99%,
**Chiral HPLC:** RT: 5.65min, Purity: 100%
**¹H NMR (400 MHz, CD₃OD):** δ 8.87 (s, 1H), 8.22 (s, 2H), 8.12 (s, 1H), 7.78 - 7.76 (m, 2H), 7.60 - 7.58 (m, 2H), 4.76 (bs, 2H), 3.54-3.51 (m, 5H), 1.39 (s, 12H).

### Synthesis of [(S)-3-methyl-1-piperazinyl]{2-[1-(p-cyclopropylphenyl)-4-pyrazolyl]-1,3-thiazol-4-yl}methanone hydrochloride (compound H)

### Overall synthetic scheme

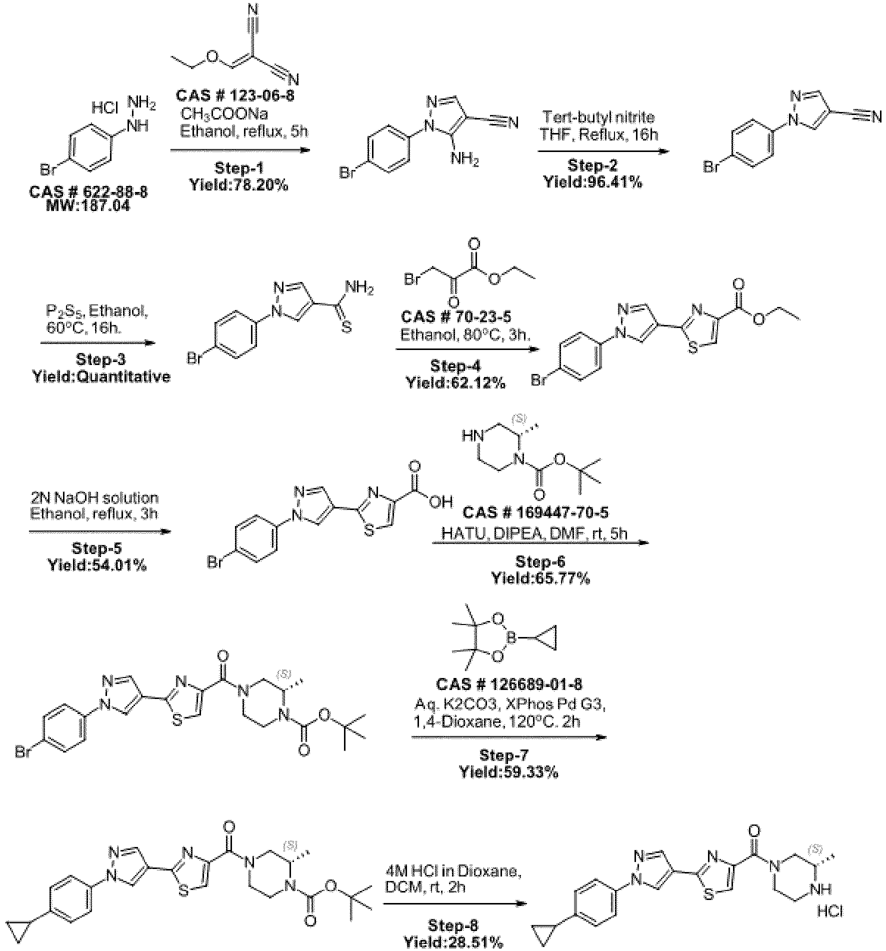

### Synthetic procedures

### Step-1: Synthesis of 5-amino-1-(4-bromophenyl)-1H-pyrazole-4-carbonitrile

To a stirred solution of (4-bromophenyl)hydrazine hydrochloride (20.0 g, 106.95 mmol) in ethanol (200 mL, 10V) at room temperature, sodium acetate (15.25 g, 213.90 mmol) and 2-(ethoxymethylene)malononitrile (15.16 g, 106.95 mmol) were added. The reaction mixture was heated at reflux for 5h. The progress of the reaction was monitored by TLC and LCMS analysis. After completion of the reaction, the reaction mixture was concentrated under reduced pressure. The crude material was washed with cold water (200mL) and extracted with ethyl acetate (2 × 200 mL). The combined organic fractions were dried over anhydrous sodium sulphate and concentrated under reduced pressure to provide **5-amino-1-(4-bromophenyl)-1H-pyrazole-4-carbonitrile (22 g, 78.20 % yield**) as a yellow solid.

**LCMS [ESI, M+1]:** 264.73 (RT: 1.62min, Purity: 95.34%)

### Step-2: Synthesis of 1-(4-bromophenyl)-1H-pyrazole-4-carbonitrile

To a stirred solution of 5-amino-1-(4-bromophenyl)-1H-pyrazole-4-carbonitrile (22 g, 83.65 mmol) in THF (220 mL, 10V) at room temperature, tert-butyl nitrite (90 %) (19.87 mL, 167.30 mmol) was added. The reaction mixture was heated at reflux for 16h. The progress of the reaction was monitored by TLC and LCMS analysis. After completion of the reaction, the reaction mixture was poured into water (200 mL), extracted using ethyl acetate (2 × 150 mL) and dried over sodium sulphate. The crude material was purified by trituration using Dichloromethane (10 mL) and Diethyl Ether (90 mL) to provide 1-(4-bromophenyl)-1H-pyrazole-4-carbonitrile (20 g, 96.41 % yield) as a dark yellow solid.

**¹H NMR (400 MHz, *d*₆-DMSO):** δ 9.37 (s, 1H), 8.39 (s, 1H), 7.81 (q, J = 25.1, 8.9 Hz, 4H).

### Step-3: Synthesis of 1-(4-bromophenyl)-1H-pyrazole-4-carbothioamide

To a stirred solution of 1-(4-bromophenyl)-1H-pyrazole-4-carbonitrile (20 g, 80.8 mmol) in ethanol (200 mL, 10V) at 0 °C, Phosphorus pentasulfide (35.8 g, 161.2 mmol) was added. The reaction mixture was heated at reflux (60 °C) for 16h. The progress of the reaction was monitored by TLC and LCMS analysis. After completion of the reaction, the reaction mixture was concentrated under reduced pressure. The crude material was washed with sodium bicarbonate solution until neutral pH (50 mL) and extracted with ethyl acetate (3 × 200mL). The combined organic fractions were dried over anhydrous sodium sulphate and concentrated under reduced pressure. The crude material was purified by trituration using diethyl ether (100mL × 2) to provide 1-(4-bromophenyl)-1H-pyrazole-4-carbothioamide (30g, Quantitative yield) as a light brown solid.

**LCMS [ESI, M, M+2]:** 281.6, 283.6 (RT:1.828 min, Purity: 91.35%)

### Step-4: Synthesis of ethyl 2-(1-(4-bromophenyl)-1H-pyrazol-4-yl)thiazole-4-carboxylate

To a stirred solution of 1-(4-bromophenyl)-1H-pyrazole-4-carbothioamide (30.0 g, 106.38 mmol) in ethanol (300 mL, 10V) at room temperature, ethyl 3-bromo-2-oxopropanoate (41.49 g, 212.76 mmol) was added. The reaction mixture was stirred at 80°C for 3h. The progress of the reaction was monitored by TLC and LCMS analysis. After completion of the reaction, the reaction, the reaction mixture was concentrated under reduced pressure and the crude material triturated using dichloromethane (10mL) and Diethyl ether (50 mL × 2) to provide ethyl 2-(1-(4-bromophenyl)-1H-pyrazol-4-yl)thiazole-4-carboxylate (25 g, 62.12 % Yield) as an orange solid.

**LCMS [ESI, M, M+2]:** 377.8, 379.7 (RT: 2.301min, Purity: 86.61%)

### Step-5: Synthesis of 2-(1-(4-bromophenyl)-1H-pyrazol-4-yl)thiazole-4-carboxylic acid

To a stirred solution of ethyl 2-(1-(4-bromophenyl)-1H-pyrazol-4-yl)thiazole-4-carboxylate (10.5 g, 0.027 mmol) in ethanol (105 mL, 10V) at 0 °C, 2N NaOH solution (105 mL, 10V) was added. The reaction mixture was stirred at 80 °C for 3 h. The progress of the reaction was monitored by TLC and LCMS analysis. After completion of the reaction, the reaction mixture was concentrated under reduced pressure. The crude product was poured into water (100mL) and extracted with ethyl acetate (2 × 100mL). The resulting aqueous layer was acidified using conc. HCl up to pH ~2-3. A solid precipitate was formed, which was isolated by filtration using a Büchner funnel and purified by trituration using n-hexane (100 mL × 3) to provide 2-(1-(4-bromophenyl)-1H-pyrazol-4-yl)thiazole-4-carboxylic acid (6 g, 61.72 % yield) as a light yellow solid.

**LCMS [ESI, M, M+2]:** 349.7, 351.6 (RT: 1.78 min, Purity: 95.73%)

### Step-6: Synthesis of tert-butyl (S)-4-(2-(1-(4-bromophenyl)-1H-pyrazol-4-yl)thiazole-4-carbonyl)-2-methylpiperazine-1-carboxylate

To a stirred solution of 2-(1-(4-bromophenyl)-1H-pyrazol-4-yl)thiazole-4-carboxylic acid (0.8g, 2.24mmol) in DMF (8 mL, 10V) at room temperature, HATU (1.3 g, 1.71 mmol) and DIPEA (1.16mL, 6.84 mmol) were added. After stirred for 30 minutes, tert-butyl (S)-2-methylpiperazine-1-carboxylate (0.45 g, 2.24 mmol) was added to the reaction mixture at room temperature. The reaction mixture was stirred at room temperature for 5 h. The progress of the reaction was monitored by TLC and LCMS analysis. After completion of the reaction, crushed ice was added to reaction mixture. A precipitate was formed which was isolated by filtration using a Büchner funnel and dried under reduced pressure to provide tert-butyl (S)-4-(2-(1-(4-bromophenyl)-1H-pyrazol-4-yl)thiazole-4-carbonyl)-2-methylpiperazine-1-carboxylate (0.8g, 65.77% yield) as an off-white solid.

**LCMS [ESI, M, M+2]:** 532.0, 534.0 (RT: 2.527 min, Purity: 100%),

### Step-7: Synthesis tert-butyl (S)-4-(2-(1-(4-cyclopropylphenyl)-1H-pyrazol-4-yl)thiazole-4-carbonyl)-2-methylpiperazine-1-carboxylate

To a stirred solution of tert-butyl (S)-4-(2-(1-(4-bromophenyl)-1H-pyrazol-4-yl)thiazole-4-carbonyl)-2-methylpiperazine-1-carboxylate (0.8 g, 1.5 mmol) in 1,4-dioxane (6.4 mL, 8V) at room temperature, K₂CO₃ (0.61, 4.50mmol) in water (1.6 mL, 2V) and 2-cyclopropyl-4,4,5,5-tetramethyl-1,3,2-dioxaborolane (0.3 g, 1.80 mmol) were added. The reaction mixture was purged using N₂ gas for 15 min and Xphos PdG₃ (0.12 g, 0.15 mmol) was added to the reaction mixture at room temperature. The reaction mixture was stirred at 120 °C for 2 h using microwave irradiation. The progress of the reaction was monitored by TLC and LCMS analysis. After completion of the reaction, the reaction mixture was poured into cold water (15 mL) and extracted with Ethyl Acetate (2 × 30 mL). The combined organic fractions were dried over anhydrous Sodium Sulphate and concentrated under reduced pressure. The crude material was purified by column chromatography using neutral alumina (80% EtOAc in hexane) to provide tert-butyl (S)-4-(2-(1-(4-cyclopropylphenyl)-1H-pyrazol-4-yl)thiazole-4-carbonyl)-2-methylpiperazine-1-carboxylate (0.44g, 59.33% yield) as a white solid.

**LCMS [ESI, M+1]:** 494.0 (RT: 2.516min, Purity: 60.43%).

### Step-8: Synthesis of (S)-(2-(1-(4-cyclopropylphenyl)-1H-pyrazol-4-yl)thiazol-4-yl)(3-methyl piperazin-1-yl)methanone Hydrochloride (compound H)

To a stirred solution of tert-butyl (S)-4-(2-(1-(4-cyclopropylphenyl)-1H-pyrazol-4-yl)thiazole-4-carbonyl)-2-methylpiperazine-1-carboxylate (0.44 g, 0.89 mmol) in CH₂Cl₂ (4.4 mL, 10V) at 0 ° C, 4M HCl in Dioxane (2.2 mL, 5V) was added. The reaction mixture was stirred at room temperature for 2 h. The progress of the reaction was monitored by TLC and LCMS analysis. After completion of the reaction, the reaction mixture was concentrated under reduced pressure. The crude material was purified by preparative HPLC using Acetonitrile/0.05 M HCl in water as mobile phase, in order to provide (S)-(2-(1-(4-cyclopropylphenyl)-1H-pyrazol-4-yl)thiazol-4-yl)(3-methylpiperazin-1-yl)methanone Hydrochloride (compound H); 0.1g, 28.51% yield) as a white solid.
**LCMS [ESI, M+1]:** 394.0 (RT: 1.474min, Purity: 100%),
**HPLC:** RT: 5.18 min, Purity: 99.25%
**Chiral HPLC:** RT: 9.42 min, Purity: 98.93%
**¹H NMR (400 MHz, CD₃OD):** δ 8.83 (s, 1H), 8.20 (s, 1H), 8.11 (s, 1H), 7.71 (d, J = 8.5 Hz, 2H), 7.25 (d, J = 8.5 Hz, 2H), 4.82 - 4.72 (m, 3H), 3.66 - 3.39 (m, 4H), 2.07 - 1.96 (m, 1H), 1.42 (s, 3H), 1.06 - 1.02 (m, 2H), 0.77 - 0.73 (m, 2H).

### Synthesis of 3-(4-{[(S)-3-methyl-1-piperazinyl]carbonyl}-1,3-oxazol-2-yl)-1-(2-cyano-4-cyclopropylphenyl)-4-pyrazolecarbonitrile hydrochloride (compound N)

### Overall synthetic scheme

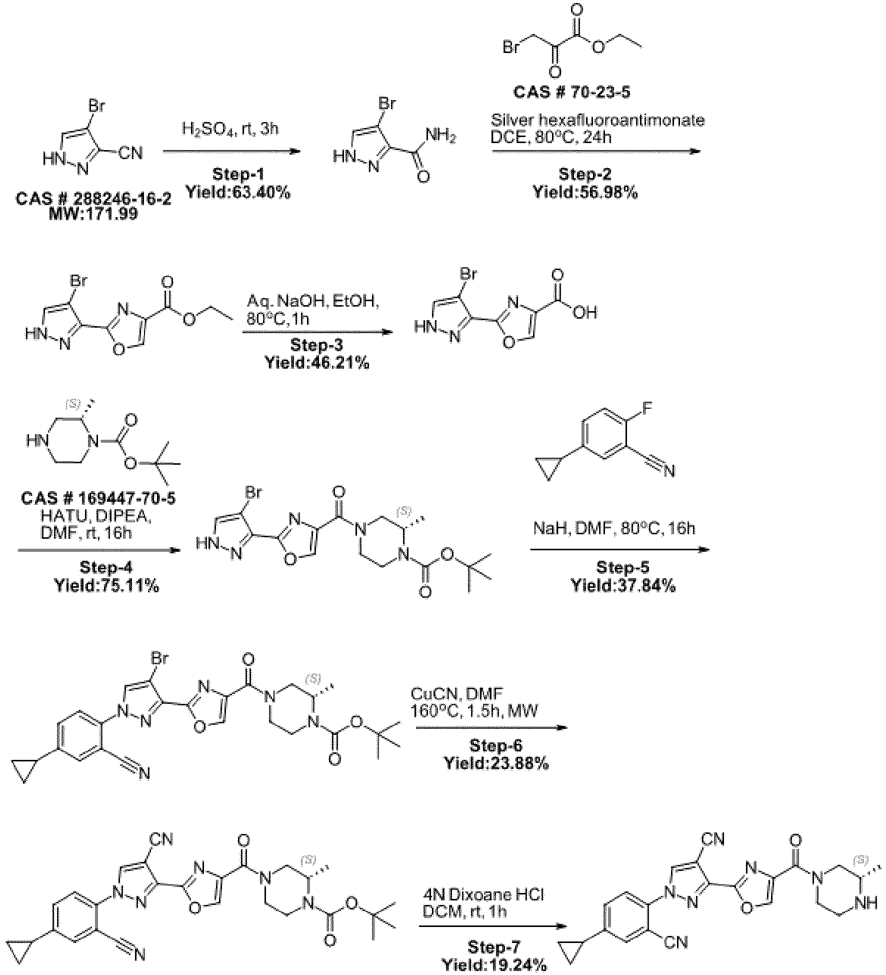

### Synthetic procedures

### Step-1: Synthesis of 4-bromo-1H-pyrazole-3-carboxamide

A stirred solution of 4-bromo-1H-pyrazole-3-carbonitrile (10.0 g, 58.1 mmol) in Conc. H₂SO₄ (60 mL) was stirred at room temperature for 3h. The progress of the reaction was monitored by TLC analysis. After completion of the reaction, the reaction mixture was poured into crushed ice to form a solid precipitate, followed by the addition of 25% aq.NH₃ until a pH of approximately 8 was reached (180mL). The solid was filtered and dried under reduced pressure to provide 4-bromo-1H-pyrazole-3-carboxamide (7.0g, 63.40% yield) as a white solid.

**¹H NMR (400 MHz, *d*₆-DMSO):** δ 13.55 (s, 1H), 8.05 (s, 1H), 7.52 (s, 1H), 7.27 (s, 1H).

### Step-2: Synthesis of ethyl 2-(4-bromo-1H-pyrazol-3-yl)oxazole-4-carboxylate

To a stirred solution of 4-bromo-1H-pyrazole-3-carboxamide (7.0 g, 36.8 mmol) in DCE (175 mL, 25V) at room temperature, ethyl 3-bromo-2-oxopropanoate (28.70 g, 147.0 mmol) and silver hexafluoroantimonate (12.65 g, 36.8 mmol) were added. The reaction mixture was stirred at 80°C for 24h. The progress of the reaction was monitored by TLC analysis. After completion of the reaction, the reaction mixture was filter through Celite^{®} and the filtrate was diluted with water (750 mL) and extracted with CH₂Cl₂ (3 × 200mL). The combined organic fractions were dried over Na₂SO₄ and concentrated under reduced pressure. The crude material was purified by reverse phase column chromatography (60 % CH₃CN in water) to provide ethyl 2-(4-bromo-1H-pyrazol-3-yl)oxazole-4-carboxylate (6.0 g, 56.98 % yield) as a yellow solid.

**LCMS [ESI, M, M+2]:** 285.7, 287.6 (RT: 1.379min, Purity: 46.68%),

### Step-3: Synthesis of 2-(4-bromo-1H-pyrazol-3-yl)oxazole-4-carboxylic acid

To a stirred solution of ethyl 2-(4-bromo-1H-pyrazol-3-yl) oxazole-4-carboxylate (6.0 g, 20.9 mmol) in EtOH (60 mL, 10V) at room temperature, 2 N NaOH solution (60 mL, 10V) was added. The reaction mixture was stirred at 80 °C for 1 h. The progress of the reaction was monitored by TLC analysis. After completion of the reaction, the reaction mixture was directly distilled under reduced pressure to obtained crude material, then acidified using 1N HCl solution (pH=~3-4) and extracted with EtOAc (3 × 150 mL). The combined organic fractions were dried over Na₂SO₄ and concentrated under reduced pressure to provide 2-(4-bromo-1H-pyrazol-3-yl) oxazole-4-carboxylic acid (2.5 g, 46.21 %) as an off white solid.

**LCMS [ESI, M, M+2]:** 257.8, 259.7 (RT: 1.085min, Purity: 74.26%),

### Step-4: Synthesis of tert-butyl (S)-4-(2-(4-bromo-1H-pyrazol-3-yl)oxazole-4-carbonyl)-2-methylpiperazine-1-carboxylate

To a stirred solution of 2-(4-bromo-1H-pyrazol-3-yl) oxazole-4-carboxylic acid (2.5 g, 9.68 mmol) in DMF (25 mL, 10V) at room temperature under nitrogen, HATU (5.52 g, 14.5 mmol) was added. After 30 min, tert-butyl (S)-2-methylpiperazine-1-carboxylate (2.32 g, 11.6 mmol) and DIPEA (4.99 mL, 29.0 mmol) were added at room temperature and the reaction mixture was stirred at room temperature for 16h. The progress of the reaction was monitored by TLC analysis. After completion of the reaction, the reaction mixture was poured into ice-cold water (100 mL) and extracted with EtOAc (3 × 150 mL). The combined organic fractions were dried over Na₂SO₄ and concentrated under reduced pressure. The crude material was purified by normal phase column chromatography (3% MeOH in CH₂Cl₂) to provide tert-butyl (S)-4-(2-(4-bromo-1H-pyrazol-3-yl)oxazole-4-carbonyl)-2-methylpiperazine-1-carboxylate (3.2 g, 75.11 % yield) as a yellow sticky solid.

**LCMS [ESI, M-56]:** 385.8 (RT: 1.521 min, Purity: 87.73%),

### Step-6: Synthesis of tert-butyl (S)-4-(2-(4-bromo-1-(2-cyano-4-cyclopropylphenyl)-1H-pyrazol-3-yl) oxazole-4-carbonyl)-2-methylpiperazine-1-carboxylate

To a stirred solution of tert-butyl (S)-4-(2-(4-bromo-1H-pyrazol-3-yl)oxazole-4-carbonyl)-2-methylpiperazine-1-carboxylate (0.4 g, 0.90 mmol) in DMF (8.0mL, 20V) at 0°C, NaH(60% in mineral oil) (0.054 g, 2.25 mmol) was added. After 30 min, 5-cyclopropyl-2-fluorobenzonitrile (0.58 g, 3.63 mmol) was added at 0 °C, and the reaction mixture was stirred at 80 °C for 16h. The progress of the reaction was monitored by TLC analysis. After completion of the reaction, the reaction mixture was poured into ice-cold water (100 mL) and extracted with EtOAc (3 × 100 mL). The combined organic fractions were dried over Na₂SO₄ and concentrated under reduced pressure. The crude material was purified by normal phase column chromatography (70% EtOAc in Hexane) to provide tert-butyl (S)-4-(2-(4-bromo-1-(2-cyano-4-cyclopropylphenyl)-1H-pyrazol-3-yl)oxazole-4-carbonyl)-2-methylpiperazine-1-carboxylate (0.6 g, 37.84% yield) as an off white sticky solid. Note that three batches of this reaction were carried out in parallel (on the same scale as described here) and the product yield quoted here is based on the combination of all these three batches.

**LCMS [ESI, M+2]:** 482.8 (RT: 2.090min, Purity: 73.99%),

### Step-6: Synthesis of tert-butyl (S)-4-(2-(4-cyano-1-(2-cyano-4-cyclopropylphenyl)-1H-pyrazol-3-yl)oxazole-4-carbonyl)-2-methylpiperazine-1-carboxylate

To a stirred solution of tert-butyl (S)-4-(2-(4-bromo-1-(2-cyano-4-cyclopropylphenyl)-1H-pyrazol-3-yl)oxazole-4-carbonyl)-2-methylpiperazine-1-carboxylate (0.3g, 0.51mmol) in DMF (6 mL, 20V) at room temperature, CuCN (0.138 g, 1.54 mmol) was added. The reaction mixture was heated at 160 °C for 1.5 h in microwave. The progress of the reaction was monitored by TLC analysis. After completion of the reaction, the reaction mixture was diluted with water (50 mL) and extracted with EtOAc (3 × 50 mL). The combined organic fractions were dried over Na₂SO₄ and concentrated under reduced pressure. The crude material was purified by normal phase column chromatography (45% EtOAc in hexane) to provide tert-butyl (S)-4-(2-(4-cyano-1-(2-cyano-4-cyclopropylphenyl)-1H-pyrazol-3-yl)oxazole-4-carbonyl)-2-methylpiperazine-1-carboxylate (0.13 g, 23.88 % yield) as an off white solid. Two batches of this reaction were carried out in parallel (on the same scale as described here), and the product yield quoted here is based on the combination of all these two batches.

**LCMS [ESI, M-100]:** 428.1 (RT: 1.916 min, Purity: 87.42%)

### Step-7: Synthesis of (S)-1-(2-cyano-4-cyclopropylphenyl)-3-(4-(3-methylpiperazine-1-carbonyl) oxazol-2-yl)-1H-pyrazole-4-carbonitrile hydrochloride (compound N):

To a stirred solution tert-butyl (S)-4-(2-(4-cyano-1-(2-cyano-4-cyclopropylphenyl)-1H-pyrazol-3-yl) oxazole-4-carbonyl)-2-methylpiperazine-1-carboxylate (0.13 g, 0.24 mmol) in CH₂Cl₂ (2 mL, 15V) at 0 °C, 4 M HCl in Dioxane (1.0 mL, 7.5V) was added. The reaction mixture was stirred at room temperature for 1 h. The progress of the reaction was monitored by TLC analysis. After completion of the reaction, the reaction mixture was concentrated under reduced pressure and triturated using diethyl ether to provide crude material. The crude material was purified by preparative HPLC using (MP-A:0.05% HCl in water, MP-B: CH₃CN) to provide (S)-1-(2-cyano-4-cyclopropylphenyl)-3-(4-(3-methylpiperazine-1-carbonyl) oxazol-2-yl)-1H-pyrazole-4-carbonitrile hydrochloride (0.022g, 19.24% yield) as a white solid.
**LCMS [ESI, M+1]:** 428.1 (RT: 1.294 min, Purity: 100%),
**HPLC Purity:** RT: 4.25 min, Purity: 100%,
**Chiral HPLC Purity:** RT: 2.486 min, Purity: 100%,
**¹H NMR (400 MHz, CD₃OD):** δ 9.07 (s, 1H), 8.68 (s, 1H), 7.76-7.72 (m, 2H), 7.61 (dd, *J* = 4.0,8.0 Hz, 1H), 5.65-5.35 (m, 1H), 4.88 (bs,1H), 3.54 (bs, 1H), 3.45-3.50 (m, 2H), 3.32-3.25 (m, 1H), 3.15 (bs, 1H), 2.16-2.09 (m, 1H), 1.45 (bs, 3H), 1.20-1.15 (m, 2H), 0.90-0.86 (m, 2H).

### Synthesis of 2-[4-(4-{[(S)-3-methyl-1-piperazinyl]carbonyl}-1H-1,2,3-triazol-1-yl)-1-pyrazolyl]-5-cyclopropylbenzonitrile hydrochloride (compound O)

### Overall synthetic scheme

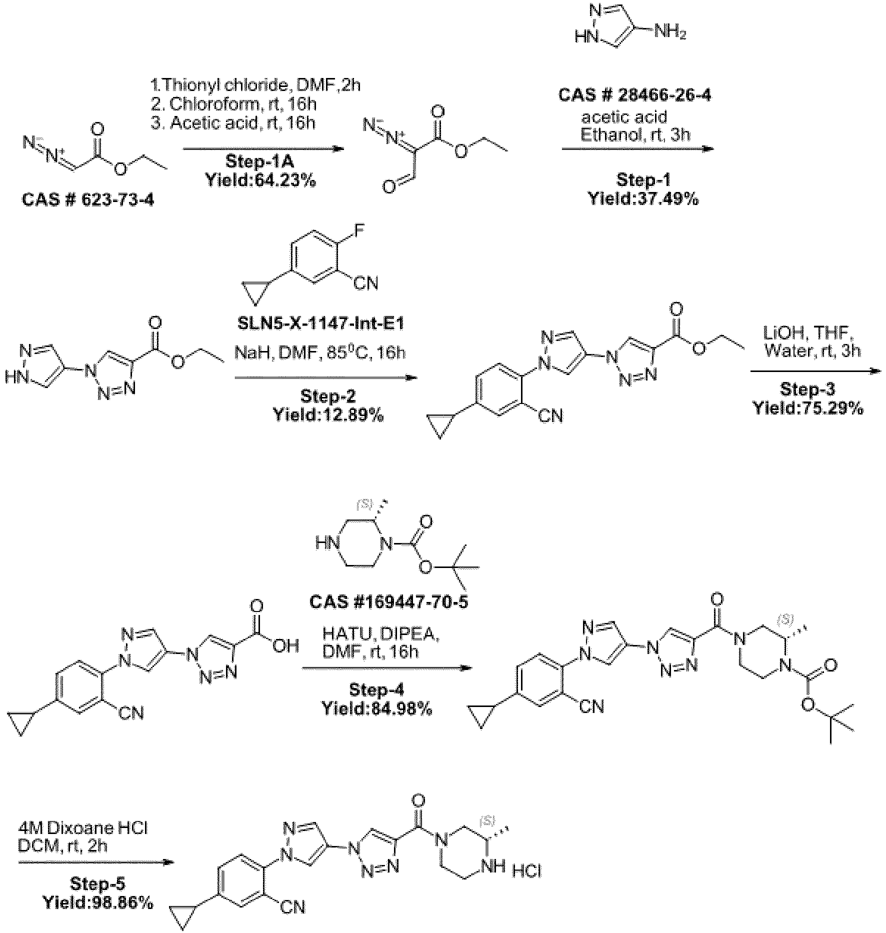

### Synthetic procedures

### Step-1A: Synthesis of ethyl 2-diazo-3-oxopropanoate

Thionyl chloride (11.0 mL, 0.44 V) was added dropwise with stirring to DMF (11.5 mL, 0.46 V) at room temperature under nitrogen. The reaction mixture was stirred at 40°C for 2h. After 2h the reaction mixture was concentrated under reduced pressure under nitrogen to obtain a white solid (note that the solid is highly moister sensitive. The solid was dissolved in chloroform (66.75 mL, 2.67V) and ethyl 2-diazoacetate (25g, 219.10mmol) was added dropwise at 0⁰C.The solution was stirred at room temperature for 16h. The solvent was removed under reduced pressure and azeotroped with diethyl ether (3 × 50 mL). Acetic acid (66.75 mL, 2.67V) was added at room temperature to the residue and the reaction mixture was stirred at room temperature for 16 h. The progress of the reaction was monitored by TLC analysis. After completion of the reaction, the reaction mixture was poured into ice-cold water (200 mL) and extracted with Diethyl ether (3 × 100 mL). The combined organic fractions were washed with water, 1N HCl and 1N NaOH, then dried over Na₂SO₄ and concentrated under reduced pressure at 25 °C to provide ethyl 2-diazo-3-oxopropanoate (20.0 g, 64.23% yield) as yellow liquid (confirmed by TLC analysis using the 2,4-DNP stain and matched with authentic sample). The material was used directly in the next step without purification and analysis. Note: product has low boiling point; under high vacuum product evaporation was observed.

### Step-1: Synthesis of ethyl 1-(1H-pyrazol-4-yl)-1H-1,2,3-triazole-4-carboxylate

To a stirred solution of ethyl 2-diazo-3-oxopropanoate (8.64 g, 60.89 mmol) in EtOH (11.5 mL, 2.5V) at room temperature, 1H-pyrazol-4-amine (4.60 g, 55.36 mmol) and acetic acid (4.6 mL, 1.0V) were added. The reaction mixture was stirred at room temperature for 3 h. The progress of the reaction was monitored by TLC and LCMS analysis. After completion of the reaction, the reaction mixture was concentrated under reduced pressure. Ice cold water (100mL) was added to the obtained material to form a solid precipitate. The osolid material was filtered and dried under reduce pressure to provide ethyl 1-(1H-pyrazol-4-yl)-1H-1,2,3-triazole-4-carboxylate (4.3 g, 37.49 % yield) as a black-brown solid (confirmed by TLC analysis using ninhydrine stain and matched with authentic sample). The material was used directly in the next step without purification and analysis.

### Step-2: Synthesis of ethyl 1-(1-(2-cyano-4-cyclopropylphenyl)-1H-pyrazol-4-yl)-1H-1,2,3-triazole-4-carboxylate

To a stirred solution of ethyl 1-(1H-pyrazol-4-yl)-1H-1,2,3-triazole-4-carboxylate (0.2 g, 0.96 mmol) in DMF (2.0 mL, 10V) at 0°C, NaH (60% in mineral oil) (0.05 g, 2.41 mmol) was added. After 30 min, 5-cyclopropyl-2-fluorobenzonitrile (0.21 g, 1.30 mmol) was added at 0 °C, and the reaction mixture was stirred at 85 °C for 16h. The progress of the reaction was monitored by TLC and LCMS analysis. After completion of the reaction, the reaction mixture was poured into cold water (50 mL) and extracted with Ethyl acetate (3 × 50 mL). The combined organic fractions were dried over Na₂SO₄ and concentrated under reduced pressure to obtain crude material, which was purified by normal phase column chromatography (65% Ethyl acetate in hexane) to provide ethyl 1-(1-(2-cyano-4-cyclopropylphenyl)-1H-pyrazol-4-yl)-1H-1,2,3-triazole-4-carboxylate (0.13 g, 12.89% yield) as an off-white yellow sticky solid.

Note that two batches of this reaction were carried out in parallel (on the same scale as described here) and the product yield quoted here is based on the combination of these two batches.

**LCMS [ESI, M+1]:** 348.9 (RT: 1.70min, Purity: 80.04%)

### Step-3: Synthesis of 1-(1-(2-cyano-4-cyclopropylphenyl)-1H-pyrazol-4-yl)-1H-1,2,3-triazole-4-carboxylic acid

To a stirred solution of ethyl 1-(1-(2-cyano-4-cyclopropylphenyl)-1H-pyrazol-4-yl)-1H-1,2,3-triazole-4-carboxylate (0.13 g, 0.37mmol) in THF: water (8: 2) (1.3 mL, 10V) at room temperature, LiOH (0.04 g, 1.11 mmol) was added. The reaction mixture was stirred at room temperature for 3h. The progress of the reaction was monitored by TLC analysis. After completion of the reaction, reaction mixture was concentrated under reduced pressure. The crude material was acidified using citric acid solution (pH=~3-4) to form a solid precipitate, which was filtered and dried under reduce pressure to provide 1-(1-(2-cyano-4-cyclopropylphenyl)-1H-pyrazol-4-yl)-1H-1,2,3-triazole-4-carboxylic acid (0.09 g, 75.29 % yield) as a yellow solid.

**LCMS [ESI, M+1]:** 320.7 (RT: 1.44min, Purity: 97.51%)

### Step-4: Synthesis of tert-butyl (S)-4-(1-(1-(2-cyano-4-cyclopropylphenyl)-1H-pyrazol-4-yl)-1H-1,2,3-tr iazole-4-carbonyl)-2-methylpiperazine-1-carboxylate

To a stirred solution of 1-(1-(2-cyano-4-cyclopropylphenyl)-1H-pyrazol-4-yl)-1H-1,2,3-triazole-4-carboxylic acid (0.09 g, 0.28 mmol) in DMF (0.9 mL, 10V) at room temperature, HATU (0.16 g, 0.42 mmol) was added. After 30 min, tert-butyl (S)-2-methylpiperazine-1-carboxylate (0.06 g, 0.33 mmol) and DIPEA (0.14 mL, 0.84 mmol) were added at room temperature and the reaction mixture was stirred at room temperature for 16h. The progress of the reaction was monitored by TLC and LCMS analysis. After completion of the reaction, the reaction mixture was poured into cold water (30 mL) and extracted with Ethyl acetate (3 × 30 mL). The combined organic fractions were washed with ice- cold water (50mL), dried over Na₂SO₄ and concentrated under reduced pressure. The crude material was purified by normal phase column chromatography (52% Ethyl acetate in Hexane) to provide tert-butyl (S)-4-(1-(1-(2-cyano-4-cyclopropylphenyl)-1H-pyrazol-4-yl)-1H-1,2,3-triazole-4-carbonyl)-2-methylpiperazine-1-carboxylate (0.12g, 84.98% yield) as a yellow sticky solid.

**LCMS [ESI, M-100]:** 402.8 (RT: 1.89min, Purity: 100%)

### Step-5: Synthesis of (S)-5-cyclopropyl-2-(4-(4-(3-methylpiperazine-1-carbonyl)-1H-1,2,3-triazol-1-yl)-1H-pyrazol-1-yl) benzonitrile hydrochloride (compound O)

To a stirred solution of tert-butyl (S)-4-(1-(1-(2-cyano-4-cyclopropylphenyl)-1H-pyrazol-4-yl)-1H-1,2,3-triazole-4-carbonyl)-2-methylpiperazine-1-carboxylate (0.12 g, 0.23 mmol) in CH₂Cl₂ (1.2 mL, 10V) at 0 °C, 4 M HCl in dioxane (0.6 mL, 5V) was added. The reaction mixture was stirred at room temperature for 2 h. The progress of the reaction was monitored by TLC analysis. After completion of the reaction, the reaction mixture was concentrated under reduced pressure. The crude material was purified by trituration using di-ethyl ether (2 × 5 mL) to provide (S)-5-cyclopropyl-2-(4-(4-(3-methylpiperazine-1-carbonyl)-1H-1,2,3-triazol-1-yl)-1H-pyrazol-1-yl) benzonitrile hydrochloride (compound O): 0.095g, 98.86% yield) as a white solid.
**LCMS [ESI, M+1]:** 403.1 (RT:1.29min, Purity: 97.80%),
**HPLC Purity:** RT:4.90min, Purity:99.46%
**Chiral HPLC Purity:** RT:4.96min, Purity:99.66%
**¹H NMR (400 MHz, CD₃OD):** δ 8.94 (s, 1H), 8.88 (s, 1H), 8.34 (s, 1H), 7.72 - 7.68 (m, 2H), 7.57 (dd, J = 8.8, 2.4 Hz, 1H), 5.31 (bs, 1H), 4.71 (bs, 1H), 3.77 (bs, 1H), 3.56 - 3.53 (m, 3H), 3.20 (bs, 1H), 2.12 - 2.07 (m, 1H), 1.43 - 1.42 (m, 3H), 1.17 - 1.13 (m, 2H), 0.88 - 0.86 (m, 2H).

### Synthesis of [(S)-3-methyl-1-piperazinyl]{1-[1-(p-cumenyl)-4-pyrazolyl]-1H-1,2,3-triazol-4-yl}methanone hydrochloride (compound P)

### Overall synthetic scheme

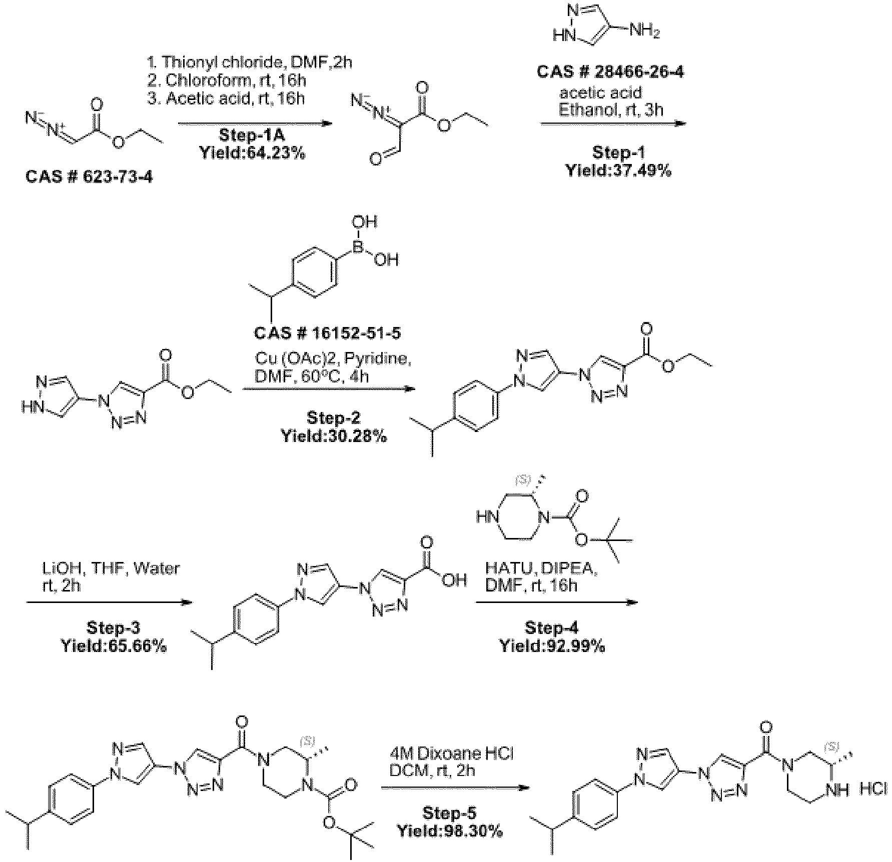

### Synthetic procedures

### Step-1A and Step-1: see synthesis of compound O

### Step-2: Synthesis of ethyl 1-(1-(4-isopropylphenyl)-1H-pyrazol-4-yl)-1H-1,2,3-triazole-4-carboxylate

To a stirred solution of ethyl 1-(1H-pyrazol-4-yl)-1H-1,2,3-triazole-4-carboxylate (0.63 g, 3.04 mmol) and (4-isopropylphenyl) boronic acid (0.50 g, 3.04 mmol) in DMF (5 mL, 10V) at room temperature, Cu(OAc)₂ (1.1 g, 6.09 mmol) and pyridine (0.49 mL, 6.09 mmol) were added. The reaction mixture was stirred at 60 °C for 4h. The progress of the reaction was monitored by TLC analysis. After completion of the reaction, the reaction mixture was diluted with water (50 mL) and Ethyl acetate (100 mL) and filtered through Celite^{®} under reduced pressure. The filtrate was extracted with Ethyl acetate (3 × 50 mL). The combined organic fractions were dried over Sodium Sulphate and concentrated under reduced pressure. The crude material was purified by normal phase column chromatography (63% Ethyl acetate in Hexane) to provide ethyl 1-(1-(4-isopropylphenyl)-1H-pyrazol-4-yl)-1H-1,2,3-triazole-4-carboxylate (0.3g, 30.28% yield) as a white solid.

**LCMS [ESI, M+1]:** 325.95 (RT: 1.96min, Purity: 100%).

### Step-3: Synthesis of 1-(1-(4-isopropylphenyl)-1H-pyrazol-4-yl)-1H-1,2,3-triazole-4-carboxylic acid

To a stirred solution of ethyl 1-(1-(4-isopropylphenyl)-1H-pyrazol-4-yl)-1H-1,2,3-triazole-4-carboxylate (0.3 g, 0.92 mmol) in THF: Water (8:1) (3 mL, 10V) at room temperature, LiOH (0.11 g, 2.76 mmol) was added. The reaction mixture was stirred at room temperature for 2 h. The progress of the reaction was monitored by TLC analysis. After completion of the reaction, the reaction mixture was concentrated under reduced pressure at 35 °C. The residue was diluted with water (10mL) and the solution acidified using citric acid solution (pH=-4-5). A solid precipitate was formed which was filtered using a Büchner funnel and dried under reduced pressure to provide 1-(1-(4-isopropylphenyl)-1H-pyrazol-4-yl)-1H-1,2,3-triazole-4-carboxylic acid (0.18g, 65.66% yield) as an off-white solid.

**LCMS [ESI, M+1]:** 297.84 (RT:1.63min, Purity: 100%),

### Step-4: Synthesis of tert-butyl (S)-4-(1-(1-(4-isopropylphenyl)-1H-pyrazol-4-yl)-1H-1,2,3-triazole-4-carbonyl)-2-methylpiperazine-1-carboxylate

To a stirred solution of 1-(1-(4-isopropylphenyl)-1H-pyrazol-4-yl)-1H-1,2,3-triazole-4-carboxylic acid (0.18 g, 0.60 mmol) in DMF (1.8 mL, 10V) at room temperature, HATU (0.34 g, 0.90 mmol) was added. The reaction was stirred for 30 minutes at room temperature, then tert-butyl (S)-2-methylpiperazine-1-carboxylate (0.14g, 0.72mmol) and DIPEA (0.30 mL, 1.81 mmol) were added. The reaction mixture was stirred at room temperature for 16 h. The progress of the reaction was monitored by TLC and LCMS analysis. After completion of the reaction, the reaction mixture was diluted with cold water (50 mL) and extracted with Ethyl Acetate (3 × 50 mL). The combined organic fractions were washed with cold water (100 mL), dried over Sodium Sulphate and concentrated under reduced pressure. The crude material was purified by flash column chromatography (43% Ethyl acetate in Hexane) to provide tert-butyl (S)-4-(1-(1-(4-isopropylphenyl)-1H-pyrazol-4-yl)-1H-1,2,3-triazole-4-carbonyl)-2-methylpiperazine-1-carboxylate (0.27 g, 92.99 % yield) as a pale yellow solid.

**LCMS [ESI, M-100]:** 380.1 (RT: 2.17min, Purity: 99.25%).

### Step-5: Synthesis of (S)-(1-(1-(4-isopropylphenyl)-1H-pyrazol-4-yl)-1H-1,2,3-triazol-4-yl) (3-methylpiperazin-1-yl) methanone hydrochloride (compound P)

To a stirred of tert-butyl (S)-4-(1-(1-(4-isopropylphenyl)-1H-pyrazol-4-yl)-1H-1,2,3-triazole-4-carbonyl)-2-methylpiperazine-1-carboxylate (0.27 g, 0.56 mmol) in CH₂Cl₂ (2.7 mL, 10V) at 0 °C, 4M HCl in Dioxane (1.35 mL, 5V) was added. The reaction mixture was stirred at room temperature for 2h. The progress of the reaction was monitored by TLC and LCMS analysis. After completion of the reaction, the reaction mixture was concentrated under reduced pressure. The crude material was purified by trituration with diethyl ether to provide (S)-(1-(1-(4-isopropylphenyl)-1H-pyrazol-4-yl)-1H-1,2,3-triazol-4-yl) (3-methylpiperazin-1-yl) methanone hydrochloride Compound P); 0.21 g, 98.30 % yield) as a white solid.
**LCMS [ESI, M+1]:** 380.01 (RT: 1.40 min, Purity: 100%),
**HPLC Purity:** RT: 5.38min, Purity: 99.22%,
**Chiral HPLC:** RT: 3.52 min, Purity: 99.54%.
**¹H NMR (400 MHz, CD₃OD)** δ 8.91 (s, 2H), 8.23 (s, 1H), 7.76 (d, J = 8.8 Hz, 2H), 7.43 (d, J = 8.8 Hz, 2H), 5.31 (bs, 1H), 4.71 (bs,1H), 3.56 (bs, 1H), 3.52 - 3.49 (m, 3H), 3.04 (bs, 1H), 3.03 - 2.97 (m, 1H), 1.42 (d, J = 5.6 Hz, 3H), 1.32 (s, 3H), 1.30 (s, 3H).

**Table 1: Compounds according to the present invention with chemical name, LCMS data and experimental procedure details**

| **Comp ound/ Form ula** | **Structure** | **Chemical Name** | **MW** | **Observed MW in LCMS** | **Synthesis details** |
|---|---|---|---|---|---|
| A | 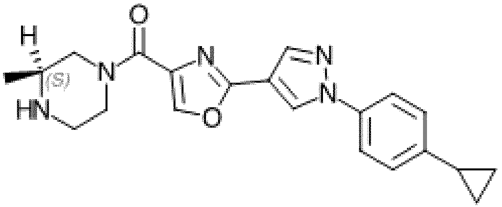 | [(S)-3-methyl-1-piperazinyl]{2-[1-(p-cyclopropylphenyl)-4-pyrazolyl]-1,3-oxazol-4-yl}methanone | 377.4 | 378.01 [ESI, M+1] RT: 1.27 min, purity: 100% | Prepared by GP1A using ethyl 2-bromooxazole-4-carboxylate (0.5g, 2.27mmol) is step-1 and 1-bromo-4-cyclopropylbenzene in step-2A. Isolated as the HCl salt |
| B | 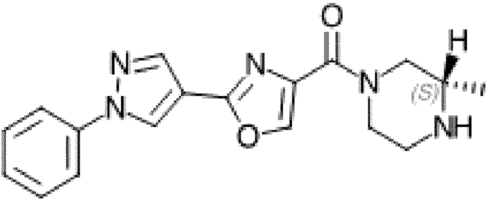 | [(S)-3-methyl-1-piperazinyl][2-(1-phenyl-4-pyrazolyl)-1,3-oxazol-4-yl]methanone | 337.4 | 338.0 [ESI, M+1] RT: 1.173 min, purity: 100% | See full experimental (isolated as HCl salt) |
| C | 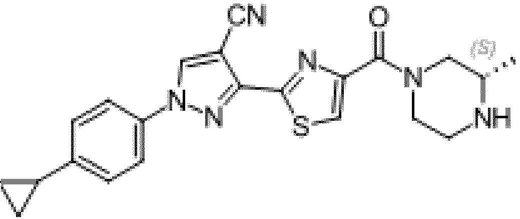 | 3-(4-{[(S)-3-methyl-1-piperazinyl]carbonyl}-1,3-thiazol-2-yl)-1-(p-cyclopropylphenyl)-4-pyrazolecarbonitrile | 418.5 | 419.1 [ESI, M+1] RT: 1.488 min, purity: 99.32%. | See full experimental (isolated as HCl salt) |
| D | 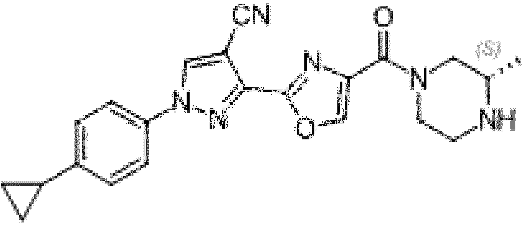 | 3-(4-{[(S)-3-methyl-1-piperazinyl]carbonyl}-1,3-oxazol-2-yl)-1-(p-cyclopropylphenyl)-4-pyrazolecarbonitrile | 402.5 | 403.1 [ESI, M+1] RT: 1.372 min, purity: 100% | See full experimental (isolated as HCl salt) |
| E | 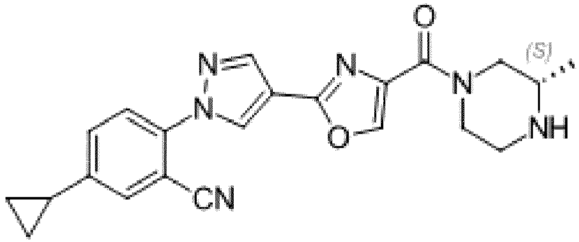 | 2-[4-(4-{[(S)-3-methyl-1-piperazinyl]carbonyl}-1,3-oxazol-2-yl)-1-pyrazolyl]-5-cyclopropylbenzonitrile | 402.5 | 403.1 [ESI, M+1] RT: 1.238 min, purity:96.87% | See full experimental (isolated as HCl salt) |
| F | 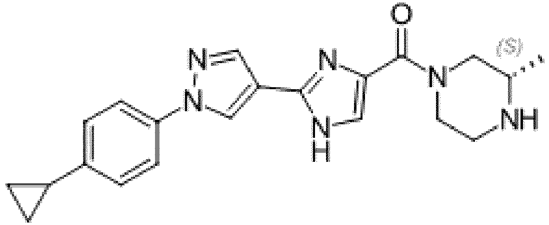 | [(S)-3-methyl-1-piperazinyl]{2-[1-(p-cyclopropylphenyl)-4-pyrazolyl]-4-imidazolyl } methanone | 376.5 | 377.06 [ESI, M+1] RT: 1.159min, purity: 100% | See full experimental (isolated as HCl salt) |
| G | 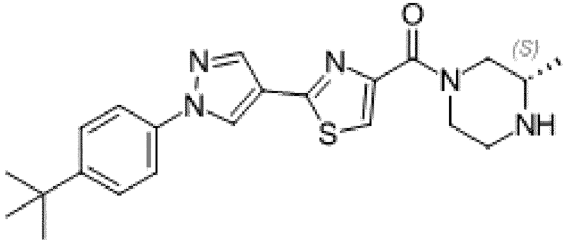 | [(S)-3-methyl-1-piperazinyl](2-{ 1-[p-(tert-butyl)phenyl]-4-pyrazolyl}-1,3-thiazol-4-yl)methanone | 409.6 | 410.11 [ESI, M+1] RT: 1.575 min, purity: 98.99% | See full experimental (isolated as HCl salt) |
| H | 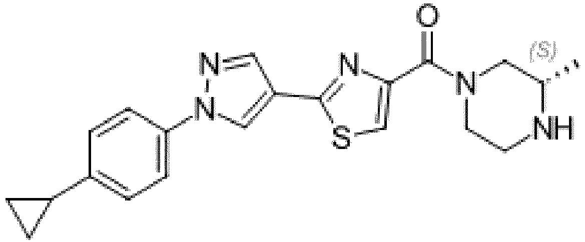 | [(S)-3-methyl-1-piperazinyl]{2-[1-(p-cyclopropylphenyl)-4-pyrazolyl]-1,3-thiazol-4-yl}methanone | 393.5 | 394.0 [ESI, M+1] RT: 1.474min, purity: 100% | See full experimental (isolated as HCl salt) |
| I | 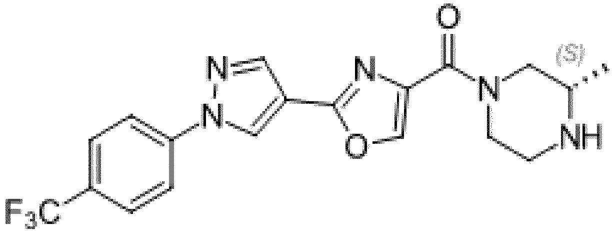 | [(S)-3-methyl-1-piperazinyl](2-{ 1-[p-(trifluoromethyl)phenyl]-4-pyrazolyl}-1,3-oxazol--yl)methanone | 405.4 | 406.01 [ESI. M+1] RT: 1.377 min, purity: 99.59% | Prepared by general procedure 1B using ethyl 2-bromooxazole-4-carboxylate (60.0g, 271.24mmol) in step-1 and 1-bromo-4-(trifluoromethyl)benzen e in step-4. Isolated as the HCl salt. |
| J | 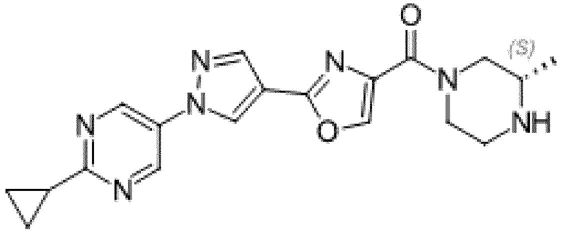 | [(S)-3-methyl-1-piperazinyl]{2-[1-(2-cyclopropyl-5-pyrimidinyl)-4-pyrazolyl]-1,3-oxazol-4-yl}methanone | 379.4 | 380.1 [ESI, M+1] RT: 1.134 min; purity: 98.28% | Prepared by general procedure 1B using ethyl 2-bromooxazole-4-carboxylate (60.0g, 271.24mmol) in step-1 and 5-bromo-2-cyclopropylpyrimidine in step-4. Isolated as the HCl salt. |
| K | 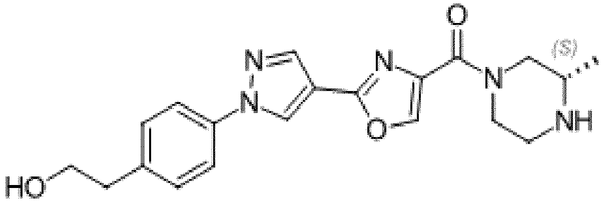 | [(S)-3-methyl-1-piperazinyl](2-{ 1-[p-(2-hydroxyethyl)phenyl] -4-pyrazolyl} -1,3-oxazol-4-yl)methanone | 381.4 | 382.0 [ESI, M+1] RT: 1.023 min, purity: 98.89% | Prepared by general procedure 1B using ethyl 2-bromooxazole-4-carboxylate (60.0g, 271.24mmol) in step-1 and 2-(4-bromophenyl)ethan-1 -ol in step-4. Isolated as the HCl salt. |
| L | 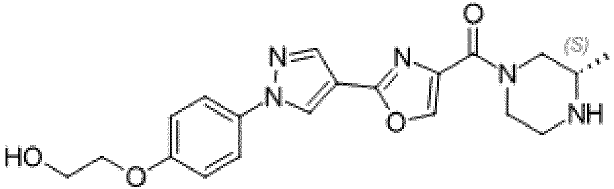 | [(S)-3-methyl-1-piperazinyl](2-{1-[p-(2-hydroxyethoxy)phenyl]-4-pyrazolyl}-1,3-oxazol--yl)methanone | 397.4 | 398.06 [ESI, M+1) RT: 1.029 min, purity: 100% | Prepared by general procedure 1B using ethyl 2-bromooxazole-4-carboxylate (60.0g, 271.24mmol) in step-1 and 2-(4-bromophenoxy)ethan-1-ol in step-4. Isolated as the HCl salt. |
| M | 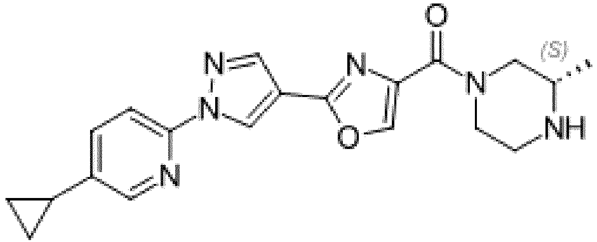 | [(S)-3-methyl-1-piperazinyl] {2-[1-(5-cyclopropyl-2-pyridyl)-4-pyrazolyl]-1,3-oxazol-4-yl}methanone | 378.4 | 379.1 [ESI, M+1] RT: 1.232 min, purity: 100% | Prepared by general procedure 1A using ethyl 2-bromooxazole-4-carboxylate (10.0g, 45.45mmol) in Step-1 and 5-cyclopropyl-2-fluoropyridine in Step-2. Isolated as the HCl salt. |
| N | 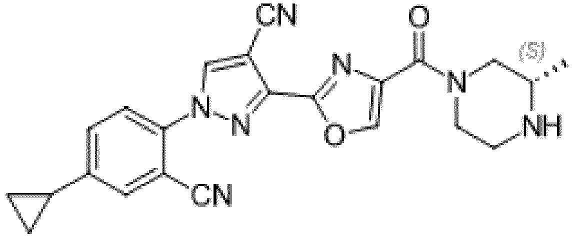 | 3-(4-{[(S)-3-methyl-1-piperazinyl]carbonyl}-1,3-oxazol-2-yl)-1-(2-cyano-4-cyclopropylphenyl)-4-pyrazolecarbonitrile | 427.5 | 428.1 [ESI, M+1] RT: 1.294 min, purity: 100% | See full experimental (isolated as HCl salt) |
| O | 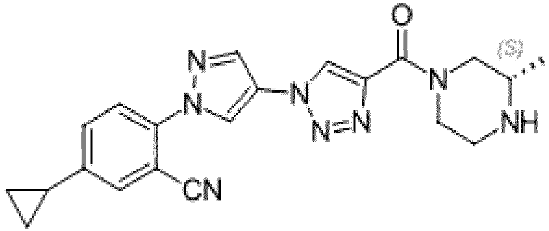 | 2-[4-(4-{[(S)-3-methyl-1-piperazinyl]carbonyl}-1H-1,2,3-triazol-1-yl)-1-pyrazolyl]-5-cyclopropylbenzonitrile | 402.5 | 403.1 RT:1.29min, purity: 97.80% | See full experimental (isolated as HCl salt) |
| P | 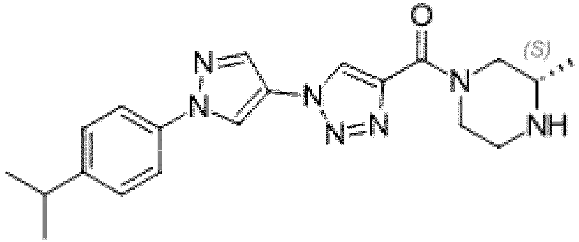 | [(S)-3-methyl-1-piperazinyl]{1-[1-(p-cumenyl)-4-pyrazolyl]-1H-1,2,3-triazol-4-yl}methanone | 379.5 | 380.01 RT: 1.40 min, purity: 100% | See full experimental (isolated as HCl salt) |

### Biological assays and data

As stated above, the compounds of the present invention induce and/or stimulate autophagy and are useful in treating autophagy-related diseases, in particular of the CNS. The biological activity of the compounds of the present invention can generally be determined by any appropriate test to determine the ability to induce and/or stimulate autophagy.

### Assessment of stimulation of autophagy

Compounds of this invention were assessed for their ability to stimulate autophagy using one or more of the assays as described below.

### Assay to measure lysosome and autolysosome formation

Lysosomes play a fundamental role in the autophagic pathway by fusing with autophagosomes and creating 'autolysosomes' in order to digest their contents. Stimulation of lysosome and autolysosome formation by a compound is indicative of a stimulation of autophagy. The ability of compounds to stimulate lysosome and autolysosome formation, and thus autophagy, in five cells was assessed via fluorescent microscopy using various fluorescent stains for labelling and tracking acidic organelles (including lysosomes and autolysosomes) such as: LysoView^{™} 650 (70059 and 70059-T, Biotium), LysoViewTM633 (70058 and 70058-T, Biotium) and LysoTracker^{™} Deep Red (L12492, ThermoFisher Scientific). The cellular phenotype was quantitatively assessed for the induction of acidic vesicle formation and compared to a non-treated control, thus providing a measure of the ability of the compound under investigation to stimulate autophagy.

Representative procedure using LysoView^{™}633 dye or LysoTracker^{™} Deep Red dye: Human osteosarcoma U2OS cells (40,000 cells/well) were seeded in a 24 well glass bottom plate (Sensoplate, Greiner Bio-One) and were incubated overnight in a humidified atmosphere at 37°C and 5% CO2. Cells were grown in DMEM (Gibco) supplemented with 10% Fetal Bovine Serum (FBS) and 100 units/ml penicillin and 100 µg/ml streptomycin (Invitrogen). After the attachment period, cells were treated with different compounds of interest (at various concentrations in DMSO) or DMSO (non-treated control) in cell culture medium and were incubated for 24 hours. Compound-containing medium was removed and cells were incubated with pre-warmed cell culture medium containing 1× LysoView^{™} 633 (70058 and 70058-T, Biotium) or 50 nM LysoTracker^{™} Deep Red (L12492, ThermoFisher Scientific) for 45 minutes at 37°C. Finally, cell nuclei were stained for 10 minutes using Hoechst 33342 (1 µg/mL) and then the medium was replaced with fresh medium. The 24 well plate was then fitted into a heated stage on the microscope and cells maintained at 37°C. Images were captured using appropriate filter set for Cy5 and DAPI detection with the EVOS M7000 Microscope (ThermoFisher Scientific). The cellular phenotype was visually assessed for the induction of acidic vesicle formation relative to the non-treated control; multiple images were acquired and analyzed using ImageJ or Cell Profiler.

### Tandem reporter assay (assay to assess autophagic flux)

Selected compounds of this invention were also assessed for their ability to stimulate autophagy using a U2OS cell line stabling expressing RFP-eGFP-hLC3b (a tandem reporter cell line).

During the process of autophagy, the cargo to be degraded is first enveloped by organelles called autophagosomes. These autophagosomes then fuse with lysosomes, causing them to become acidified and their digestive enzymes to become activated. The term "autophagic flux" is used to represent the dynamic process of autophagy: autophagic flux refers to the whole process of autophagy, including autophagosome formation, maturation, fusion with lysosomes, subsequent breakdown and the release of macromolecules back into the cytosol (Zhang XJ, Chen S, Huang KX, Le WD. Why should autophagic flux be assessed? Acta Pharmacol Sin. 2013 May; 34(5): 595-9. doi: 10.1038/aps.2012.184. Epub 2013 Mar 11). Compounds that stimulate autophagic flux stimulate the dynamic process of autophagy (the whole process of autophagy).

LC3b is a protein found in the membrane of autophagosomes which has been used to generate genetic reporters of autophagy in cells. In these systems a tandem fusion of LC3b is engineered with two fluorescent proteins of different wavelengths: one which is acid sensitive (for example eGFP, which fluoresces green) and the other which is acid insensitive (for example RFP, which fluoresces red). Cells expressing RFP-eGFP-hLC3b can be examined using fluorescent microscopy: the autophagosomes present will fluoresce in both channels (either yellow in an overlay of both channels or puncta that are present in both the individual red and green channels) but the autolysosomes present will fluoresce in the RFP (red) channel only. Using a fluorescent microscope, the number of autophagosomes and autolysosomes can be counted, allowing for the monitoring of both the induction of autophagy (total puncta count) and the rate of autophagic flux (ratio of red-only to red-and-green puncta). The influence of small molecules on autophagy induction and flux can be assessed using this system by comparison to a non-treated control.

Representative Procedure: Human osteosarcoma U2OS cells stably expressing RFP-eGFP-hLC3b were seeded (10,000 cells/well) into a 96-well glass-bottom plate (Cell Carrier Ultra, Perkin Elmer) in DMEM Glutamax media (Gibco) supplemented with 10% Fetal Bovine Serum (FBS) and antibiotics (100 u/ml penicillin, 100 µg/ml streptomycin, Invitrogen), then incubated overnight in a humified atmosphere at 37 °C and 5% CO₂. The cells were then treated with the compounds of interest, dissolved in DMSO, in a duplicated 5- or 10-point dose-response. 8 wells were treated with an equivalent volume of DMSO, 4 with 0.3 µM torin-1 as a positive control (MCE) and 4 wells with 0.4 µM torin-1 plus 0.2 µM bafilomycin (MCE) as a control for flux inhibition. The cells were returned to the incubator and the treatment continued for 24 hours. After treatment, the media was aspirated off and the cells fixed with a solution of 4% formaldehyde + 1% glutaraldehyde (Sigma) in dPBS (plus magnesium and calcium, Gibco). Fixation was allowed to proceed for 15 minutes at room temperature then the fixative solution discarded and replaced with PBS (Gibco) plus Hoechst 33342 (1 µg/ml, Sigma). After 30 minutes the plates were imaged on an Opera Phenix confocal microscope (Perkin-Elmer) using a 40x water objective, collecting using DAPI, mCherry and GFP channels. Cells were detected based upon the staining of their nuclei with DAPI using the Harmony image analysis software (Perkin-Elmer) and the number of autophagosomes (GFP and RFP puncta) and autolysosomes (RFP only puncta) per cell were counted using the spot picking function within the software.

Selected compounds of this invention showed an increase in the number of RFP-only puncta (autolysosomes) relative to a DMSO-treated control (non-treated control) at different concentrations. This is evidence for significant stimulation of autophagic flux (and thus the whole process of autophagy) by the compounds of this invention, and particularly by these compounds under the conditions as tested. Table 2 shows this data for selected compounds. Compounds having an activity designated as "+" provided between a 1% and 30% increase in activity (based on the number of RFP-only puncta [autolysosomes] as a percentage of the DMSO treated control [non-treated control]). Compounds having an activity designated as "++" provided between a 30% and 50% increase in activity (based on the number of RFP-only puncta [autolysosomes] as a percentage of the DMSO treated control [non-treated control]). Compounds having an activity designated as "+++" provided between a 50% and 100% increase in activity (based on the number of RFP-only puncta [autolysosomes]) as a percentage of the DMSO treated control [non-treated control]). Compounds having an activity designated as "++++" provided above a 100% increase in activity (based on the number of RFP-only puncta [autolysosomes]) as a percentage of the DMSO treated control [non-treated control]).

**Table 2: Assessment of autophagy stimulatory activity of compounds based on the change in the number of RFP-only puncta (autolysosomes) relative to a DMSO-treated control (non-treated control) in a U2OS cell line stabling expressing RFP-eGFP-hLC3b (a tandem reporter cell line)**

| **Compound** | **Compound concentration (mM)** | **Activity** |
|---|---|---|
| A | 1 | ++++ |
| B | 1.25 | + |
| C | 1 | +++ |
| D | 1 | ++++ |
| E | 0.9 | ++ |
| F | 10 | ++++ |
| G | 1 | ++++ |
| H | 0.9 | ++++ |
| I | 1 | ++++ |
| J | 7.5 | +++ |
| K | 0.9 | + |
| L | 10 | ++ |
| M | 1 | ++ |
| N | 5 | + |
| O | 1 | +++ |
| P | 5 | + |

## Claims

1. A compound according to the general Formula (I) 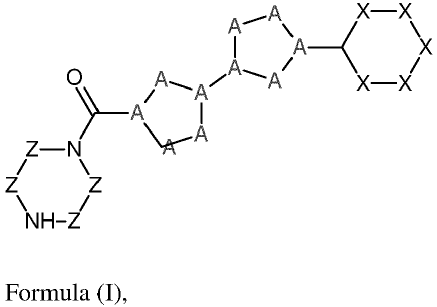 wherein
A is independently selected from chemically possible combinations of C, CH, CH₂, N, NH, O, and S, optionally substituted with cyano,
X is independently selected from chemically possible combinations of C, CH, CH₂, N and NH, optionally substituted with straight or branched C₂ to C₆ alkyl, straight or branched C₁ to C₄ hydroxyalkyl, such as, for example, -CH₂-CH₂-OH, straight or branched C₁ to C₄ hydroxyalkoxy, such as, for example, -O-CH₂-CH₂-OH), halo, trifluoromethyl, cyclopropyl, and cyano,
Z is independently selected from chemically possible combinations of CH, CH₂, N and NH, optionally substituted with straight or branched C₂ to C₆ alkyl,
and a physiologically acceptable salt, a solvate, a hydrate, an enantiomer or a polymorph thereof.

2. A compound according to claim 1 according to the following formula II, 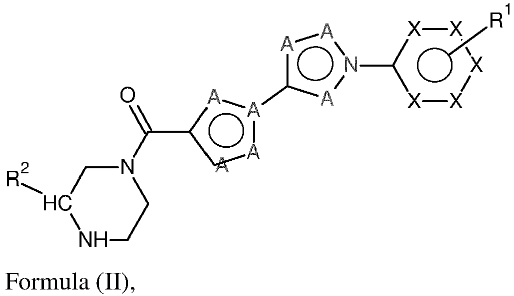 wherein
A is as above,
X is independently selected from chemically possible combinations of C, CH, CH₂, N and NH, and is optionally substituted with R¹,
R¹ is independently selected from straight or branched C₂ to C₆ alkyl, straight or branched C₁ to C₄ hydroxyalkyl (for example, -CH₂-CH₂-OH), straight or branched C₁ to C₄ hydroxyalkoxy (for example, -O-CH₂-CH₂-OH), halo, trifluoromethyl, cyclopropyl, or cyano,
R² is independently selected from H, or straight or branched C₂ to C₆ alkyl,
and
a physiologically acceptable salt, a solvate, a hydrate, an enantiomer or a polymorph thereof.

3. A compound according to claim 1 or 2 according to the following formula III, 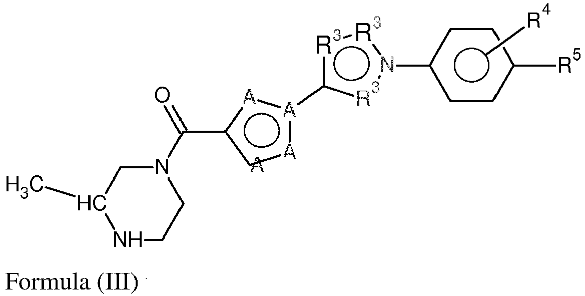 wherein
A is as above,
R⁵ is independently selected from H, straight or branched C₂ to C₆ alkyl, straight or branched C₁ to C₄ hydroxyalkyl, such as, for example, -CH₂-CH₂-OH, straight or branched C₁ to C₄ hydroxyalkoxy, such as, for example, -O-CH₂-CH₂-OH, halo, trifluoromethyl, cyclopropyl, or cyano,
R³ is independently selected from chemically possible combinations of C, CH, N and NH, optionally substituted with cyano
R⁴ is independently selected from H or cyano
and
a physiologically acceptable salt, a solvate, a hydrate, an enantiomer or a polymorph thereof.

4. A compound selected from the following group 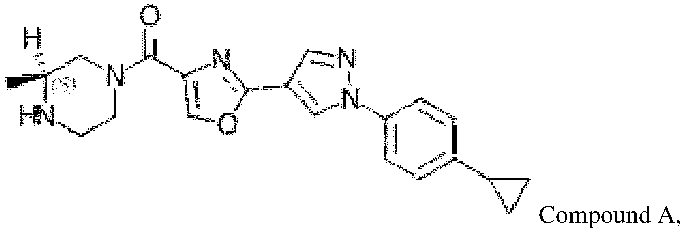 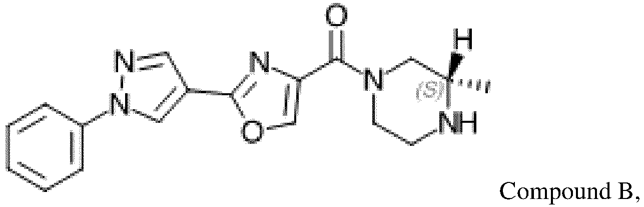 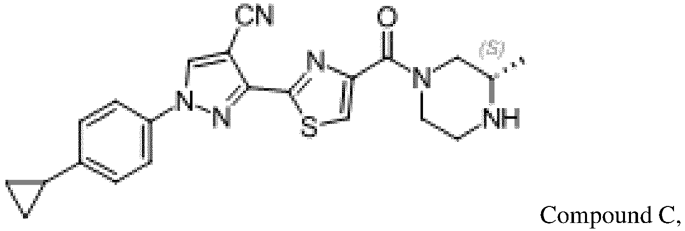 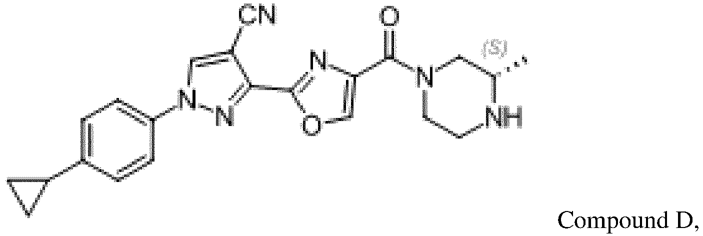 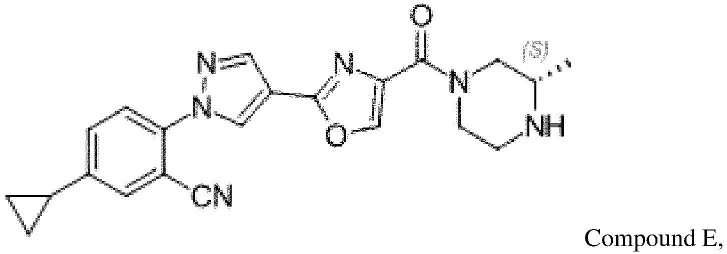 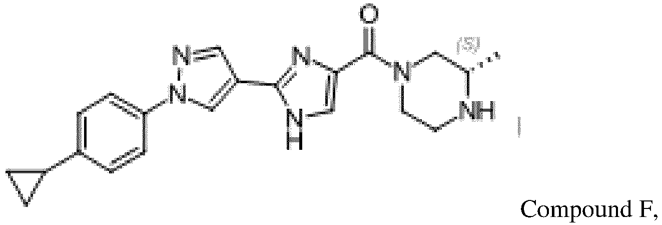 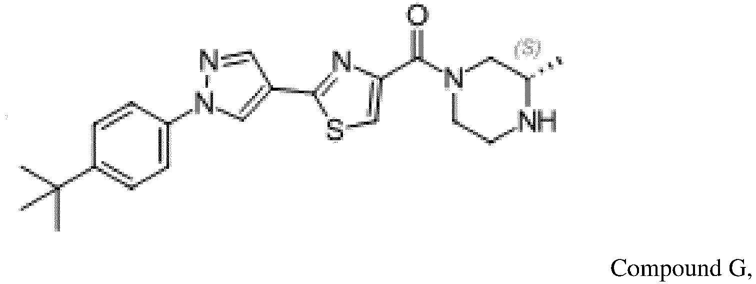 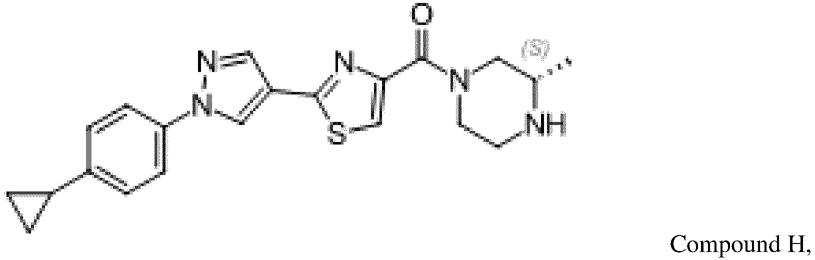 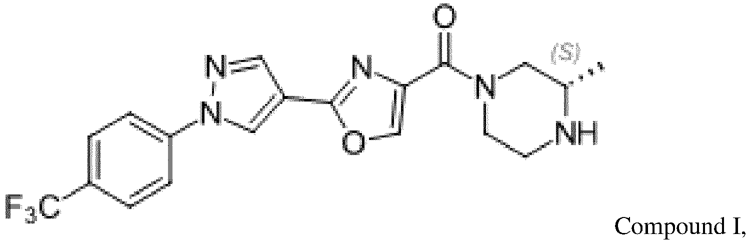 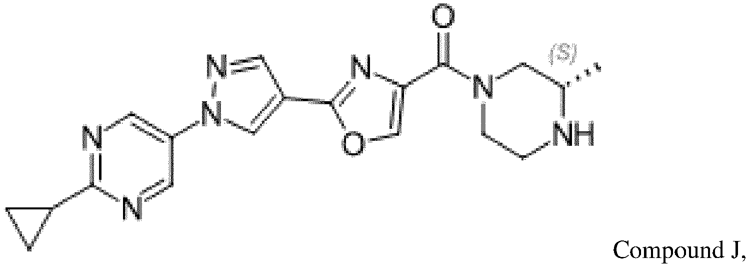 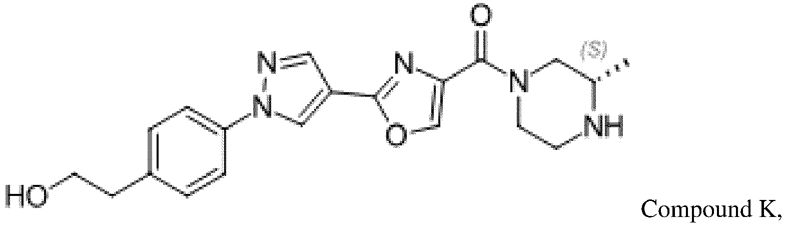 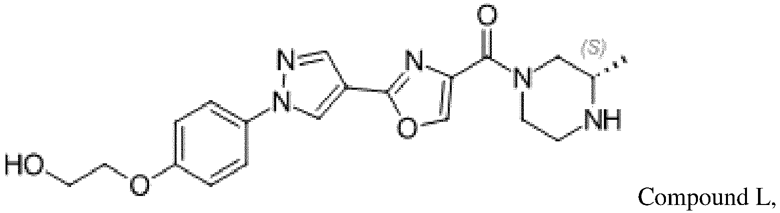 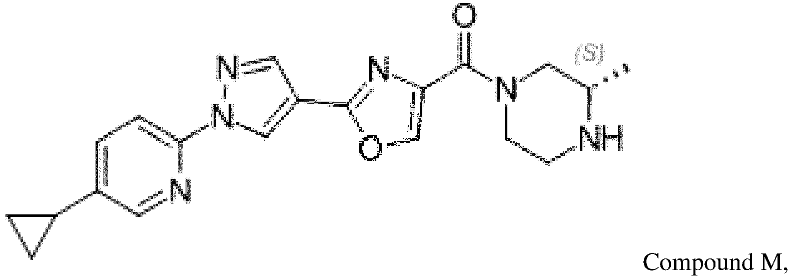 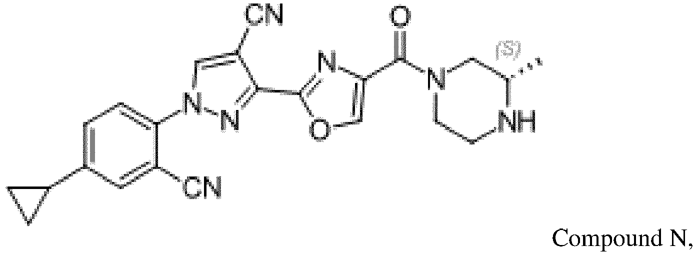 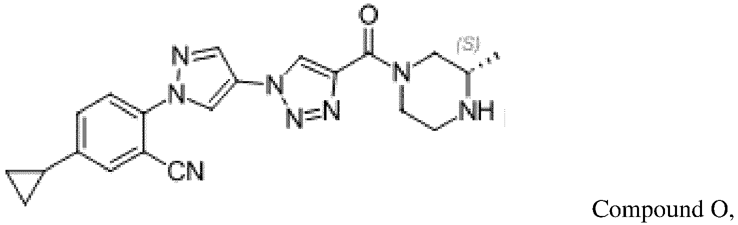 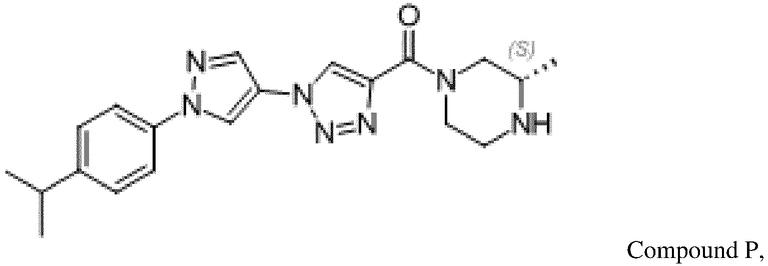 and
a physiologically acceptable salt, a solvate, a hydrate, an enantiomer or a polymorph thereof.

5. A pharmaceutical composition, comprising a pharmaceutically effective amount of the compound according to any one of claims 1 to 4, and a pharmaceutically acceptable carrier.

6. The compound according to any one of claims 1 to 4 or the pharmaceutical composition according to claim 5 for use in the prevention and/or treatment of diseases in a mammalian subject, such as a human.

7. The compound according to any one of claims 1 to 5 or the pharmaceutical composition according to claim 6 for use as an autophagy inducer, wherein preferably said use is cosmetic and/or in vitro.

8. The compound according to any one of claims 1 to 4 or the pharmaceutical composition according to claim 5 for use in the prevention and/or treatment of an autophagy-related disease or condition in a mammalian subject, such as a human.

9. The compound for use according to claim 8, wherein said autophagy-related disease or condition is selected from the group consisting of diseases and conditions of the CNS, neuronal diseases involving misfolded and/or non-folded proteins, neurodegenerative diseases, Huntington's disease, Alzheimer's disease, Parkinson's disease, epilepsy, brain or other neuronal cancer, Wilson Disease, viral infection and related neuronal diseases, Niemann-Pick type C (NPC) disease, autoimmune diseases, multiple sclerosis, stroke, Wegener's granulomatosis, neuropathic pain, post-operative phantom limb pain or postherpetic neuralgia, ALS, and spinal cord injury.

10. The compound for use according to claim 8 or 9, wherein said prevention and/or treatment comprises a combination of at least two compounds and/or a combination with at least one additional pharmaceutically active substance for said autophagy-related disease or condition.

11. The compound for use according to any one of claims 8 to 10, wherein said prevention and/or treatment further comprises detecting and/or monitoring in said subject a response of at least one autophagy -biomarker, preferably selected from the group of BECN1, and the ATG8/LC3 family, including LC3A, LC3B, LC3C), LC3-II, ULK1, p62, NBR1, ATG5 and ATG7.

12. The compound according to any one of claims 1 to 4 or the pharmaceutical composition according to claim 5 for use according to any one of claims 8 to 11, wherein said compound after administration thereof exhibits an increased ratio of concentration levels in the CNS and brain to levels outside the CNS and brain and/or when compared to a control.

13. A method for preventing and/or treating an autophagy-related disease or condition in a mammalian subject, such as a human, comprising administering to said mammal an effective amount of at least one compound according to any one of claims 1 to 4 or of a pharmaceutical composition according to claim 5, the method preferably further comprising detecting and/or monitoring in said subject a response of at least one autophagy-biomarker, preferably selected from the group of BECN1, and the ATG8/LC3 family, including LC3A, LC3B, LC3C), LC3-II, ULK1, p62, NBR1, ATG5 and ATG7.

14. The method according to claim 13, wherein said autophagy-related disease or condition is selected from the group consisting of diseases and conditions of the CNS, neuronal diseases involving misfolded and/or non-folded proteins, neurodegenerative diseases, Huntington's disease, Alzheimer's disease, Parkinson's disease, epilepsy, brain or other neuronal cancer, Wilson Disease, viral infection and related neuronal diseases, Niemann-Pick type C (NPC) disease, autoimmune diseases, multiple sclerosis, stroke, Wegener's granulomatosis, neuropathic pain, post-operative phantom limb pain or postherpetic neuralgia, ALS, and spinal cord injury.
